# EUROPEAN PATENT SPECIFICATION

(11) **EP 2 057 116 B1**
(45) Date of publication and mention of the grant of the patent: **13.02.2013**
(21) Application number: 07824995.0
(22) Date of filing: 27.10.2007
(51) Int. Cl.: C07C 311/21, A61K 31/18, A61P 29/00, C07D 209/08, C07D 211/26, C07D 211/58, C07D 213/40, C07D 213/75, C07D 231/40, C07D 233/54, C07D 235/30, C07D 241/08, C07D 261/14, C07D 277/46, C07D 277/62, C07D 277/82, C07D 285/135, C07D 295/12, C07D 317/46, C07K 5/06

(54) **NEW PHENYLSULFAMOYL BENZAMIDE DERIVATIVES AS BRADYKININ ANTAGONISTS**
NEUE PHENULSULFAMOYLBENZAMIDDERIVATE ALS BRADYKININANTAGONNISTEN
NOUVEAUX DÉRIVÉS DE PHÉNYLSULFAMOYL-BENZAMIDE UTILISÉS COME ANTAGONISTES DE LA BRADYQUININE

(30) Priority: 27.10.2006 HU 0600809
(43) Date of publication of application: 13.05.2009
(73) Proprietor: Richter Gedeon Nyrt., 1103 Budapest (HU)
(72) Inventor: BEKE, Gyula, 2092 Budakeszi (HU); BOZÓ, Éva, 1102 Budapest (HU); FARKAS, Sándor, 1103 Budapest (HU); HORNOK, Katalin, 1141 Budapest (HU); KESERÜ, György, 2089 Telki (HU); SCHMIDT, Éva, 1021 Budapest (HU); SZENTIRMAY, Éva, 2030 Érd (HU); VÁGÓ, István, 1121 Budapest (HU); VASTAG, Mónika, 1025 Budapest (HU)
(74) Representative: HOFFMANN EITLE
(86) International application number: PCT/HU2007/000103
(87) International publication number: WO 2008/050167

(56) References cited:
- WO-A-2005/004810
- WO-A-2007/072092
- US-A- 5 714 497
- US-B1- 6 451 816
- J.-L. SOULIER, ET AL.: "Design and synthesis of specific probes for human 5-HT4 receptor dimerisation studies", JOURNAL OF MEDICINAL CHEMISTRY, vol. 48, no. 20, 1 October 2005 (2005-10-01), pages 6220-6228, XP055016060, American Chemical Society, Washington, DC, US ISSN: 0022-2623, DOI: 10.1021/jm050234z

## Description

### FIELD OF THE INVENTION

The present invention relates to new phenylsulfamoyl benzamide derivatives of formula (I) and optical antipodes or racemates and/or salts and/or hydrates and/or solvates thereof which are useful in the treatment or prevention of painful and inflammatory processes. The present invention also relates to the processes for producing compounds of formula (I) and to pharmacological compositions containing the same.

### BACKGROUND OF THE INVENTION

Kinins are endogenous peptides formed in plasma and peripheral tissues in response to tissue injury or infection following catalytic cleavage of kininogens by kallikrein enzymes. Kinins play an important role in the pathophysiological processes accompanying pain and inflammation. Their biological actions are mediated by two G-protein coupled membrane receptors, denoted B1 and B2. Both B1 and B2 receptors have been cloned *[*Biochem. Biophys. Res. Commun., 184 (1992) 260-268 and J.Biol.Chem., 269 (1994) 21583-21586] and the mechanisms regulating their expression, self-maintenance and signalling function is under intensive investigations *[*Mol. Pharmacol., 56 (1999) 325-333 and J. Cell. Physiol. 193 (2002) 275-286].

The first set of kinins, bradykinin (BK) and kallidin (LysBK) preferentially act through stimulation of constitutively expressed and rapidly desensitising B2 receptors, which are widely distributed in many tissues. On the other hand, their active carboxypeptidase metabolites, the second set of kinins, desArg⁹BK (DABK) and LysdesArg⁹BK (LysDABK) activate inducible and non-desensitising B1 receptors, which are rarely expressed under non-pathological conditions. Generally B1 receptors rapidly appear after injuries of various natures (tissue trauma, infections, *etc*.). Thus the B 1 receptor up-regulation appears to be part of a generalized response that includes the local co-expression (eventually up-regulation) of enzymes, receptors, autacoids, cytokines and chemokines that notoriously play key roles in the early and late responses of tissues to various types of injury.

In animal models it has been demonstrated that there is a switch in dominance of function from B2 to B1 in chronic inflammatory states. While the B2 receptor is implicated in the acute phase of the inflammatory and pain response, the B1 receptor is involved in the chronic phase of this response. The involvement of kinin receptors in inflammation and pain transduction has been supported by the results of studies on mice lacking bradykinin B 1 receptors. B 1 receptor deficient mice are different from wild-type mice in sensory functions, exhibiting increased analgesic thresholds to noxious chemical and heat stimuli, and drastic reduction in the accumulation of polymorphonuclear leukocytes at sites of inflammation [PNAS, 97 (2000) 8140-8145 and Neuropharmacology 41 (2001) 1006-1012]. Furthermore the most original finding in B1 receptor deficient mice was the direct evidence for a role of central kinin receptors in nociception suggesting that the hypoalgesia seen in B1-receptor knockout mice is partly due to reduced central sensitisation in the spinal cord. However, apart from the above changes B1 knockout mice were apparently normal without any apparent pathological changes.

Apart from the evidence of basal expression of B1 receptors on the periphery recently more and more evidence shows that B 1 receptors are constitutively expressed 'centrally' in some neuronal elements, including the spinal cord and some higher structures as well. The function of these receptors is unclear but they have been implicated in pain transmission and hyperalgesia. Therefore, B1 receptor antagonists are believed to be useful in alleviating pain not only via peripheral sites but also to have possibly broader spectrum of analgesic effects if they block central B1 receptors as well *[*NeuroReport 11 (2000) 4003-4005; NeuroReport, 12 (2001) 2311-2313; Neuroscience 107 (2001) 665-673 and Neuroscience Letters 294 (2000) 175-178].

On the basis of scientific data bradykinin receptors are involved in mediation of pain and hyperalgesia in several ways. B1 receptor antagonists may have diverse modes of action. They have (1) indirect ('peripheral') effects on the nociceptors via inhibition of release of other algogenic mediators. N.B. B1 receptors appear upon inflammatory induction on cells adjacent to sensory neurones (macrophages, fibroblasts or endothelial cells) are involved in releasing mediators (prostaglandins, cytokines and nitric oxide) that sensitize or activate the nociceptors. (2) direct ('peripheral') effects on nociceptors expressing B1 receptors (constitutively) or upon induction and (3) 'central' effects on pain processing in the superficial dorsal horn of spinal cord.

Therefore, an orally active non-peptide bradykinin B1 receptor antagonist could be a potential therapeutic agent in the treatment of chronic inflammatory pain.

Several patents and patent applications describe bradykinin B1 receptor antagonists which have different chemical structures. Such documents are for instance the following international patent applications: WO200075107, WO02076964, WO04054584, WO02099388, WO05004810.

### SUMMARY OF THE INVENTION

We have found a class of benzamide derivatives which have high affinity for bradykinin B1 receptors and selectivity over bradykinin B2 receptors. The selectivity is particularly important as the undesired side effects of the compounds are much less pronounced.

The present invention relates to new phenylsulfamoyl benzamide derivatives of formula (I) 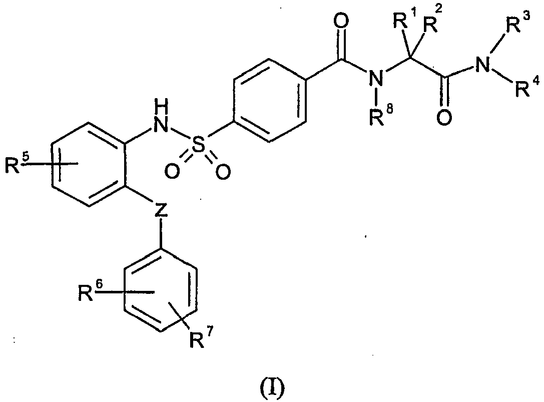 wherein
- R¹: is hydrogen atom or C₁-C₄ alkyl group;
- R²: is selected from (1) hydrogen atom; (2) C₁-C₆ straight or branched alkyl group; (3) -(CH₂)ₙ-NH₂; (4) -(CH₂)ₙ-OH; (5) -(CH₂)ₙ-CO-NH₂; (6) -(CH₂)ₙ-COOR^{c}; (7) benzyl optionally substituted with one or more hydroxy group or halogen atom; or
- R¹, R²: and the carbon atom to which they are both attached together form a 3-7 membered cycloalkyl ring;
- R³: is (1) hydrogen atom, (2) C₁-C₈ straight or branched alkyl group, optionally substituted with one or more substituents independently selected from amino, hydroxy, 1H-imidazol-4-yl, NR^{a}R^{b}, -COOR^{c}, -NH-C(=NH)-NH₂, -CO-NH₂, group;
- R⁴: is (1) hydrogen atom, (2) -(CH₂)ₙ-NR^{a}R^{b} group; (3) -(CH₂)ₘ-X-P group;
- R⁵, R⁶ and R⁷: are independently of each other hydrogen atom, halogen atom, cyano, nitro, amino, or amino substituted with one or more C₁-C₄ alkyl group; trifluoromethyl; C₁-C₄ alkyl, C₁-C₄ alkoxy, -C(=O)-NH₂, C₁-C₄ alkoxycarbonyl, trifluoromethoxy or hydroxy group;
- R⁸: is hydrogen atom or C₁-C₄ alkyl group;
- Z: is selected from (1) single bond; (2) oxygen atom; (3) CH₂ group; (4) CO group; (5) NR^{c} group; (6) S atom; (7) SO₂ group;
- n: is an integer from 1 to 6;
- m: is an integer from 0 to 6;
- X: is selected from (1) single bond; (2) oxygen atom; (3) -CO-NR^{c} group; (4) CO or SO₂ group;
- P: is selected from (1) phenyl group, optionally substituted with one or more halogen atom, hydroxy, -(CH₂)ₘ-CN, -O-CO-NR^{c}R^{c}, NH-CO-R^{c}, C₁-C₄ alkyl, C₁-C₄ alkoxy, cyano, amino, [1,4']bipiperidinyl-1'-yl or -(CH₂)ₙ-NH-C(=NH)-NH₂ group; (2) a saturated, partially unsaturated or aromatic 4-7 membered ring containing 1-3 heteroatom selected from O, S, SO₂ and N; wherein said ring is optionally substituted with one or more halogen atom, oxo, hydroxy, cyano, amino, trifluoromethyl, -NH-CO-R^{c}, -C(=NH)-NH₂, C₁-C₄ alkyl, pyridin-4-yl, piperidin-1-yl or pyridine-2-yl group; (3) a saturated, partially unsaturated or aromatic 8-10 membered bicyclic ring system containing 1-3 heteroatom selected from O, S, SO₂ and N; wherein said ring system is optionally substituted with one or more halogen atom, oxo, hydroxy, cyano, amino, -NH-CO-R^{c}, trifluoromethyl or C₁-C₄ alkyl group; (4) C₅-C₈ cycloalkyl group, optionally substituted with -(CH₂)ₘ-NR⁸R^{b} group;
- R^{a} and R^{b}: are (1) hydrogen atom; (2) straight or branched C₁-C₆ alkyl group; (3) R⁸, R^{b} and the nitrogen atom to which they are both attached together form a saturated, partially unsaturated or aromatic 4-7 membered ring containing 0-3 heteroatom (in addition to the nitrogen atom to which R^{a} and R^{b} attached) selected from O, S, SO₂ and N; wherein said ring is optionally substituted with one or more halogen atom, oxo, cyano, hydroxy or C₁-C₄ alkyl group;
- R^{c}: is hydrogen atom or C₁-C₄ alkyl group;
and optical antipodes or racemates and/or salts and/or hydrates and/or solvates thereof.

The invention also relates to the pharmaceutical compositions containing the compounds of formula (I) or optical antipodes or racemates or salts or hydrates or solvates thereof as active ingredient.

Furthermore objects of the present invention are the synthesis of compounds of formula (I), and the chemical and pharmaceutical manufacture of medicaments containing these compounds, as well as the compounds for use in methods of treatment, which means administering to a mammal to be treated - including human - effective amount/amounts of compounds of formula (I) of the present invention as such or as medicament.

### DETAILED DESCRIPTION OF THE INVENTION

The present invention relates to new bradykinin B1 receptor antagonist phenylsulfamoyl benzamide derivatives of formula (I) 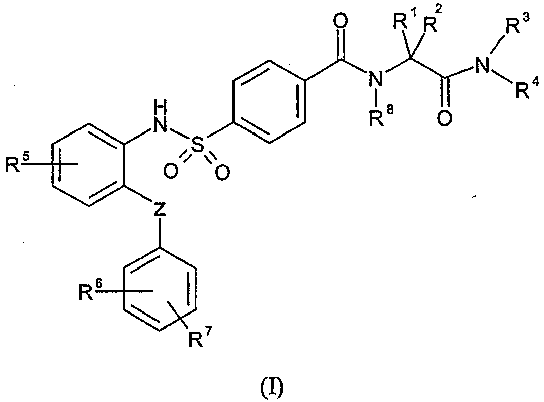 wherein
- R¹: is hydrogen atom or Cₗ₁-C₄ alkyl group;
- R²: is selected from (1) hydrogen atom; (2) C₁-C₆ straight or branched alkyl group; (3) -(CH₂)ₙ-NH₂; (4) -(CH₂)ₙ-OH; (5) -(CH₂)ₙ-CO-NH₂; (6) -(CH₂)ₙ-COOR^{c}; (7) benzyl optionally substituted with one or more hydroxy group or halogen atom; or
- R¹, R²: and the carbon atom to which they are both attached together form a 3-7 membered cycloalkyl ring;
- R³: is (1) hydrogen atom, (2) C₁-C₈ straight or branched alkyl group, optionally substituted with one or more substituents independently selected from amino, hydroxy, 1*H*-imidazol-4-yl, -NR^{a}R^{b}, -COOR^{c}, -NH-C(=NH)-NH₂, -CO-NH₂, group;
- R⁴: is (1) hydrogen atom, (2) -(CH₂)ₙ-NR^{a}R^{b} group; (3) -(CH₂)ₘ-X-P group;
- R⁵, R⁶ and R⁷: are independently of each other hydrogen atom, halogen atom, cyano, nitro, amino, or amino substituted with one or more C₁-C₄ alkyl group; trifluoromethyl; C₁-C₄ alkyl, C₁-C₄ alkoxy, -C(=O)-NH₂, C₁-C₄ alkoxycarbonyl, trifluoromethoxy or hydroxy group;
- R⁸: is hydrogen atom or C₁-C₄ alkyl group;
- Z: is selected from (1) single bond; (2) oxygen atom; (3) CH₂ group; (4) CO group; (5) NR^{c} group; (6) S atom; (7)SO₂ group;
- n: is an integer from 1 to 6;
- m: is an integer from 0 to 6;
- X: is selected from (1) single bond; (2) oxygen atom; (3) -CO-NR^{c} group; (4) CO or SO₂ group;
- P: is selected from (1) phenyl group, optionally substituted with one or more halogen atom, hydroxy, -(CH₂)ₘ-CN, -O-CO-NR^{c}R^{c}, -NH-CO-R^{c}, C₁-C₄ alkyl, C₁-C₄ alkoxy, cyano, amino, [1,4']bipiperidinyl-1'-yl or -(CH₂)ₙ-NH-C(=NH)-NH₂ group; (2) a saturated, partially unsaturated or aromatic 4-7 membered ring containing 1-3 heteroatom selected from O, S, SO₂ and N; wherein said ring is optionally substituted with one or more halogen atom, oxo, hydroxy, cyano, amino, trifluoromethyl, NH-CO-R^{c}, -C(=NH)-NH₂, C₁-C₄ alkyl, pyridin-4-yl, piperidin-1-yl or pyridine-2-yl group; (3) a saturated, partially unsaturated or aromatic 8-10 membered bicyclic ring system containing 1-3 heteroatom selected from O, S, SO₂ and N; wherein said ring system is optionally substituted with one or more halogen atom, oxo, hydroxy, cyano, amino, -NH-CO-R^{c}, trifluoromethyl or C₁-C₄ alkyl group; (4) C₅-C₈ cycloalkyl group, optionally substituted with -(CH₂)ₘ-NR⁸R^{b} group;
- R^{a} and R^{b}: are (1) hydrogen atom; (2) straight or branched C₁-C₆ alkyl group; (3) R^{a}, R^{b} and the nitrogen atom to which they are both attached together form a saturated, partially unsaturated or aromatic 4-7 membered ring containing 0-3 heteroatom (in addition to the nitrogen atom to which R^{a} and R^{b} attached) selected from O, S, SO₂ and N; wherein said ring is optionally substituted with one or more halogen atom, oxo, cyano, hydroxy or C₁-C₄ alkyl group;
- R^{c}: is hydrogen atom or C₁-C₄ alkyl group;
and optical antipodes or racemates and/or salts and/or hydrates and/or solvates thereof.

The invention also relates to the pharmaceutical compositions containing the compounds of formula (I) or optical antipodes or racemates or salts or hydrates or solvates thereof as active ingredient.

Furthermore objects of the present invention are the synthesis of compounds of formula (I), and the chemical and pharmaceutical manufacture of medicaments containing these compounds, as well as the compounds for use in methods of treatment, which means administering to a mammal to be treated - including human - effective amount/amounts of compounds of formula (I) of the present invention as such or as medicament.

The term "halogen" substituent denotes fluorine, chlorine, bromine or iodine atoms. The term C₁-C₄ alkyl group used in the present description denotes methyl, ethyl, normal- and isopropyl and different butyl groups. These C₁-C₄ alkyl groups can be in the C₁-C₄ alkoxy groups.

The saturated, partially unsaturated or aromatic 4-7 membered heterocyclic ring in the meaning of R^{a} and R^{b} can be *e.g*. piperidine, pyrrolidine, piperazine, homopiperazine, imidazole, triazole, tetrazole, morpholine, thiomorpholine and the like.

The saturated, partially unsaturated or aromatic 4-7 membered heterocyclic ring in the meaning of P can be *e.g*. piperidine, pyrrolidine, pyridine, piperazine, homopiperazine, imidazole, triazole, tetrazole, morpholine, thiomorpholine and the like.

The saturated, partially unsaturated or aromatic 8-10 membered bicyclic ring system in the meaning of P can be *e.g*. indole, benzimidazole, benzo[1,3]dioxol, benzothiazole and the like.

The invention relates also to the salts of compounds of formula (I) formed with acids or bases.

Both organic and inorganic acids can be used for the formation of acid addition salts. Suitable inorganic acids can be *e.g*. hydrochloric acid, sulfuric acid and phosphoric acid. Representatives of monovalent organic acids can be *e.g*. formic acid, acetic acid, trifluoroacetic acid, propionic acid, and different butyric acids, valeric acids and capric acids. Representatives of bivalent organic acids can be *e.g*. oxalic acid, malonic acid, maleic acid, fumaric acid and succinic acid. Other organic acids can also be used, such as hydroxy acids *e.g*. citric acid, tartaric acid, or aromatic carboxylic acids *e.g*. benzoic acid or salicylic acid, as well as aliphatic and aromatic sulfonic acids *e.g*. methanesulfonic acid and p-toluenesulfonic acid. Especially valuable group of the acid addition salts is in which the acid component itself does not have therapeutical effect in the applied dose or it does not have unfavorable influence on the effect of the active ingredient. These acid addition salts are pharmaceutically acceptable acid addition salts. The reason why acid addition salts, which do not belong to the pharmaceutically acceptable acid addition salts belong to the present invention is, that in given case they can be advantageous in the purification and isolation of the desired compounds.

Among the salts formed with bases especially important are the salts formed with alkali metals, *e.g*. sodium, potassium, alkaline-earth metals, *e.g*. calcium and magnesium, as well as with ammonia or organic amines. The latter bases can have further substituents, *e.g*. hydroxy or amino groups, which can influence *e.g*. the solubility and the handling of the product. The salts formed with bases are pharmaceutically acceptable base addition salts.

According to the invention the compounds of formula (I) can be synthesized by one of the following methods:

### Method a)

An amine derivative of formula (II) 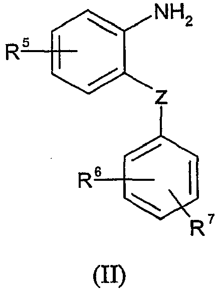 - wherein the meaning of R⁵, R⁶ and R⁷ is as described above for the formula (I) - is reacted with the sulfonyl chloride of formula (III) 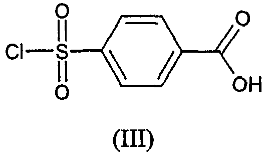 then the so obtained phenylsulfamoyl benzoic acid derivative of formula (IV) 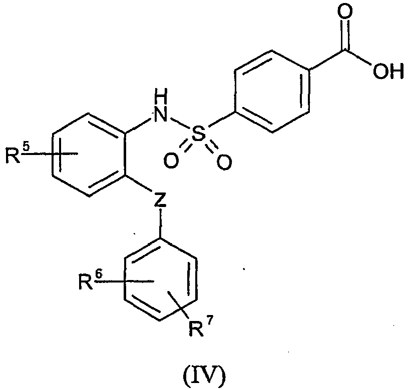 - wherein the meaning of R⁵, R⁶ and R⁷ is as defined above - is reacted with an amine derivative of formula (V) 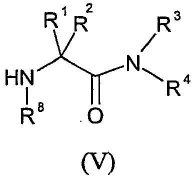 - wherein the meaning of R¹, R², R³, R⁴ and R⁸ is as defined above - and the obtained phenylsulfamoyl benzamide derivative of formula (I) in given case can be transformed into an other compound of formula (I) by introducing new substituents and/or modifying or removing the existing ones, and/or salt formation and/or liberating the compound from salts.

### Method b)

The phenylsulfamoyl benzoic acid derivative of formula (IV) - obtained according to the method described in method a) - is reacted with an amino acid derivative of formula (VI) 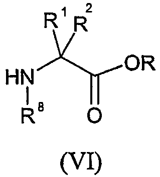 - wherein the meaning of R¹, R² and R⁸ is as defined above, and R is C₁-C₄ alkyl group - and the so obtained compound of formula (VII) 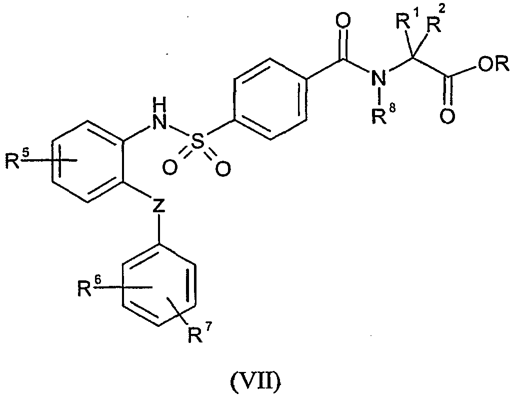 - wherein the meaning of R¹, R², R⁵, R⁶, R⁷, R⁸ and R is as defined above - is hydrolyzed to furnish a carboxylic acid derivative of formula (VIII) 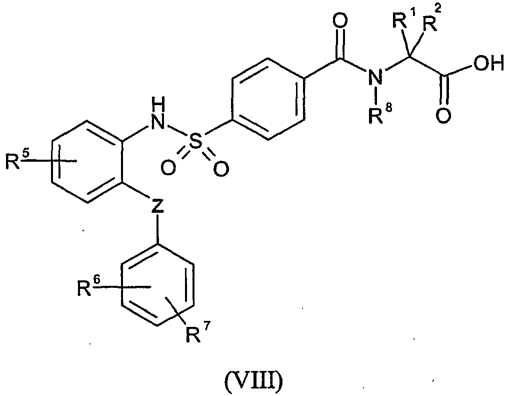 - wherein the meaning of R¹, R², R⁵, R⁶, R⁷ and R⁸ is as defined above - finally the latter is reacted with an amine derivative of formula (IX) 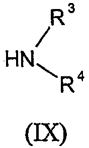 - wherein the meaning of R³ and R⁴ is as defined above - and the obtained phenylsulfamoyl benzamide derivative of formula (I) in given case can be transformed into an other compound of formula (I) by introducing new substituents and/or modifying or removing the existing ones, and/or salt formation and/or liberating the compound from salts.

The sulfonylation reaction is preferably carried out in a proper solvent, preferably in the presence of a base. The reactions are followed by thin layer chromatography. The necessary reaction time is 6-20 h. The work-up of the reaction mixture can be carried out by different methods.
a) The reaction mixture is concentrated and the product is isolated by crystallization or extraction. If the crude product is not pure enough, then column chromatography can be used for the purification of it. The column chromatography is carried out either on normal phase using Kieselgel 60 as adsorbent and different solvent systems, e.g. n-hexane/ethyl acetate, chloroform/methanol, dichloromethane/ethyl acetate or chloroform/acetone as eluents, or on reversed phase using YMC-Pack ODS-AQ type packings (produced by YMC) and acetonitrile/water/trifluoroacetic acid or acetonitrile/water/acetic acid as eluent.
b) The reaction mixture is poured into ice-water and the product is isolated by filtration or extraction. The crude product is crystallized or purified by column chromatography as described above. The structures of the products are determined by IR, NMR and mass spectrometry.

The amide bond formation is preferably carried out by preparing an active derivative from a carboxylic acid of formula (IV) or (VIII) which is reacted with an amine of formula (V) or (IX), respectively, preferably in the presence of a base.

The transformation of a carboxylic acid into an active derivative can be carried out *in situ* during the amide bond formation in a proper solvent (*e.g*. dimethylformamide, acetonitrile, chlorinated hydrocarbons or hydrocarbons or the mixture thereof). The active derivatives can be acid chlorides (*e.g*. prepared from carboxylic acid with thionyl chloride), mixed anhydrides (*e.g*. prepared from carboxylic acid with isobutyl chloroformate in the presence of a base, *e.g*. triethylamine), active esters (*e.g*. prepared from carboxylic acid with hydroxybenztriazol (HOBt) and dicyclohexyl-carbodiimide (DCC) or O-benzotriazol-1-yl-N,N,N',N'-tetramethyluronium hexafluorophosphate (HBTU) in the presence of a base *e.g*. triethylamine). The active derivatives can be prepared at a temperature in the range of 0 °C to room temperature. A proper amine of formula (V) or (IX) is added as a base or as a salt formed with inorganic acid to the so obtained solution or suspension in the presence of a base, *e.g*. triethylamine, needed for the liberation of the amine. The condensation reactions are followed by thin layer chromatography. The necessary reaction time is 6-20 h. The work-up of the reaction mixture can be carried out by different methods.
a) The reaction mixture is concentrated, and the residue is crystallized or extracted with a proper organic solvent and in given case purified by column chromatography. The column chromatography is carried out on normal phase using Kieselgel 60 as adsorbent and different solvent systems, *e.g*. toluene/methanol, chloroform/methanol or toluene/acetone, as eluents or on reversed phase using YMC-Pack ODS-AQ type packings (produced by YMC) and acetonitrile/water/trifluoroacetic acid or acetonitrile/water/acetic acid as eluent.
b) The reaction mixture is directly purified by column chromatography as described above to yield the pure product.

The structures of the products are determined by IR, NMR and mass spectrometry.

The obtained benzamide derivatives of formula (I) - independently from the method of preparation - in given case can be transformed into another compound of formula (I) by introducing further substituents and/or modifying and/or removing the existing ones, and/or formation of salts with acids and/or liberating the benzamide derivative of formula (I) from the obtained acid addition salts by treatment with a base and/or the free sulfonamide derivative of formula (I) can be transformed into a salt by treatment with a base.

The compounds of formula (I) containing free hydroxy group can be transformed into acyloxy or sulfoxy derivatives with different acylating or sulfonylating agents. The reactions can be carried out for example in chlorinated hydrocarbons using acid chloride or acid anhydride as acylating agent in the presence of a base (*e.g*. triethylamine or sodium carbonate). The sulfonamide derivatives of formula (I) containing a nitro group can be transformed into amines by reduction and the amines can be further reacted to give acid amides as described for the acylation of hydroxy groups or carbamate derivatives can be synthesized. Ester groups can be hydrolyzed and the obtained free carboxylic acids can be transformed into amides by reacting with proper amine derivatives. N-(*tert*-Butoxycarbonyl) group can be cleaved by organic or inorganic acids (*e.g*. trifluoroacetic acid or hydrogen chloride). Free amino groups can be transformed into guanidino groups. Cyano groups can be transformed into amide, N-hydroxy-amidine or different N-containing heterocyclic groups.

Most of the amino acids of formula (VI) and amines of formula (IX) are either commercially available or can be synthesized by different known methods. The syntheses of some new amines of formula (IX) are described in the Examples. Following these procedures the other amines of formula (IX) can also be prepared. The amines of formula (V) are new.

The compounds of the present invention as well as their pharmaceutically acceptable salts or hydrates or solvates can be used as such or suitably in the form of pharmaceutical compositions. These compositions (drugs) can be in solid, liquid or semiliquid form and pharmaceutical adjuvant and auxiliary materials can be added, which are commonly used in practice, such as carriers, excipients, diluents, stabilizers, wetting or emulsifying agents, pH- and osmotic pressure-influencing, flavoring or aromatizing, as well as formulation-promoting or formulation-providing additives.

The dosage required to exert the therapeutical effect can vary within wide limits and will be fitted to the individual requirements in each of the particular case, depending on the stage of the disease, the condition and the bodyweight of the patient to be treated, as well as the sensitivity of the patient against the active ingredient, route of administration and number of daily treatments. The actual dose of the active ingredient to be used can safely be determined by the attending physician skilled in the art in the knowledge of the patient to be treated.

The pharmaceutical compositions containing the active ingredient according to the present invention usually contain 0.01 to 100 mg of active ingredient in a single dosage unit. It is, of course possible that the amount of the active ingredient in some compositions exceeds the upper or lower limits defined above.

The solid forms of the pharmaceutical compositions can be *e.g*. tablets, dragées, capsules, pills or lyophilized powder ampoules useful for the preparation of injections. Liquid compositions are the injectable and infusable compositions, fluid medicines, packing fluids and drops. Semiliquid compositions can be ointments, balsams, creams, shaking mixtures and suppositories.

For the sake of a simple administration it is suitable if the pharmaceutical compositions comprise dosage units containing the amount of the active ingredient to be administered once, or a few multiples or a half, third or fourth part thereof. Such dosage units are *e.g*. tablets, which can be powdered with grooves promoting the halving or quartering of the tablet in order to exactly administer the required amount of the active ingredient.

Tablets can be coated with an acid-soluble layer in order to assure the release of the active ingredient content after leaving the stomach. Such tablets are enteric-coated. A similar effect can be achieved also by encapsulating the active ingredient.

The pharmaceutical compositions for oral administration can contain *e.g*. lactose or starch as excipients, sodium carboxymethylcellulose, methylcellulose, polyvinyl pyrrolidine or starch paste as binders or granulating agents. Potato starch or microcrystalline cellulose is added as disintegration agents, but ultraamylopectin or formaldehyde casein can also be used. Talcum, colloidic silicic acid, stearin, calcium or magnesium stearate can be used as antiadhesive and lubricants.

The tablets can be manufactured *e.g*. by wet granulation, followed by pressing. The mixed active ingredients and excipients, as well as in given case part of the disintegrants are granulated with an aqueous, alcoholic or aqueous alcoholic solution of the binders in an appropriate equipment, then the granulate is dried. The other disintegrants, lubricants and antiadhesive agents are added to the dried granulate, and the mixture is pressed to a tablet. In given case the tablets are made with halving groove to ease the administration.

The tablets can be made directly from the mixture of the active ingredient and the proper auxiliaries by pressing. In given case, the tablets can be coated by using additives commonly used in the pharmaceutical practice, *e.g*. stabilizers, flavoring, coloring agents, such as sugar, cellulose derivatives (methyl- or ethylcellulose, sodium carboxymethylcellulose, etc), polyvinyl pyrrolidone, calcium phosphate, calcium carbonate, food coloring agents, food laces, aroma agents, iron oxide pigments, etc. In the case of capsules the mixture of the active ingredient and the auxiliaries is filled into capsules.

Liquid oral compositions, *e.g*. suspensions, syrups, elixirs can be made by using water, glycols, oils, alcohols, coloring and flavoring agents.

For rectal administration the composition is formulated in suppositories or clysters. The suppository can contain beside the active ingredient a carrier, so called adeps pro suppository. Carriers can be vegetable oils, such as hydrogenated vegetable oils, triglycerides of C₁₂-C₁₈ fatty acids (preferably the carriers under the trade name Witepsol). The active ingredient is homogeneously mixed with the melted adeps pro suppository and the suppositories are moulded.

For parenteral administration the composition is formulated as injection solution. For manufacturing the injection solution the active ingredients are dissolved in distilled water and/or in different organic solvents, such as glycolethers, in given case in the presence of solubilizers, *e.g*. polioxyethylensorbitane-monolaurate, -monooleate, or monostearate (Tween 20, Tween 60, Tween 80). The injection solution can also contain different auxiliaries, such as conserving agents, *e.g*. ethylendiamine tetraacetate, as well as pH adjusting agents and buffers and in given case local anaesthetic, *e.g*. lidocain. The injection solution containing the active ingredient of the invention is filtered before it is filled into ampoules, and it is sterilized after filling.

If the active ingredient is hygroscopic, then it can be stabilized by liophylization.

### Utilities

The compounds of the present invention are bradykinin receptor antagonists, in particular selective bradykinin B1 receptor antagonists, consequently are useful in the treatment or prevention of painful and inflammatory processes. The compounds would be effective in the treatment of pain including, *e.g*., chronic pain, particularly inflammatory pain, hyperalgesia, bone and joint pain (osteoarthritis), repetitive motion pain, myofascial pain (muscular injury, fibromyalgia), visceral pain (ulcerative colitis, pancreatitis, cystitis, uveitis), perioperative pain (general surgery, gynecological), postoperative pain (postsurgical pain syndrome), posttraumatic pain (*e.g*. sprains or fracture), neuropathic pain (postherpetic neuralgia, nerve injury, phantom limb pain, mononeuropthy, polyneuropathy) dental pain, and cancer pain. Furthermore for the treatment of pain associated with angina, menstruation, diabetic vasculopathy, post capillary resistance or diabetic symptoms associated with insulitis (*e.g*. hyperglycemia, diuresis, proteinurea and increased nitrite and kallikrein urinary excretion), diabethic hyperalgeisa. Moreover the compounds may be used for the treatment angioedema, atherosclerosis, septic shock *e.g*. as anti-hypovolemic and/or anti-hypotensive agents, and sepsis. They may be used as smooth muscle relaxants for the treatment of spasm of the gastrointestinal tract or uterus. Further, the compounds of this invention can additionally be used to treat inflammatory skin disorders, such as psoriasis and eczema, and skin injuries including burning and sunburning (UV-erythema and pain). The compounds may be used to treat inflammatory pain of varied origins (*e.g*. rheumatoid arthritis, rheumatic disease, tenosynovitis, liver disease, irritable bowel syndrome, inflammatory bowel disease, Crohn's disease, nephritis, allergic rhinitis, vasomotor rhinitis, uveitis, gingivitis), allergies. Such compounds may be used therapeutically to treat inflammatory airways disease *e.g*. chronic obstructive pulmonary disease, adult respiratory distress syndrome, bronchitis, pneumonia, asthma. They may be used to control, restrict or reverse airways hyperreactivity in asthma, to treat intrinsic and extrinsic asthma including allergic asthma (atopic or non-atopic), occupational asthma, viral or bacterial exacerbated asthma, other non-allergic asthmas, "wheezy-infant syndrome", as well as exercise-induced bronchoconstriction. They may be effective against pneumoconiosis, including aluminosis, antracosis, asbestosis, chalicosis, ptilosis, siderosis, silicosis, tabacosis and byssinosis. Additionally, they may be effective in some neurological disorders, *e.g*. against multiple sclerosis, Alzheimer's disease, epilepsy, cerebral edema, headache including cluster headache, migraine including prophylactic and acute use, as well as closed head trauma.

### Biological evaluation

### Functional assay:

### Assessment of antagonist potency at B1 and B2 receptors in vitro by measurement of cytosolic calcium ion concentration with a plate reader fluorimeter in cells expressing recombinant human B1 or B2 receptors

### Cell culture

Chinese hamster ovary (CHO) cells stably expressing recombinant human B1 (CHO-B1, Euroscreen) or B2 (CHO-B2, Perkin-Elmer) receptors were cultured in Dulbecco's Modified Eagle's Medium (DMEM) containing 10% Fetal Calf Serum (FCS), 100 U/ml penicillin, 0.1 mg/ml streptomycin, 0.25 µg/ml amphotericin B, 1% Minimum Essential Medium Eagle (MEM), non essential amino acid solution, 600 µg/ml G418, 1% pyruvate (for the B2 cell line). Cells were kept at 37°C in a humidified incubator in an atmosphere of 5% CO₂/95% air and were passaged 1:4 three times a week. Cells were plated at 1.5-2.5×10⁴ cell/well on standard 96-well microplates, measurements of cytosolic calcium ion concentration ([Ca²⁺]ᵢ) were carried out 1-2 days after cell plating.

### Fluorimetric measurement of cytosolic calcium concentration

Measurements of [Ca²⁺]ᵢ were carried out on CHO-B1 and CHO-B2 cells stably expressing human B1 and B2 receptors, respectively. Cells were grown in standard 96-well microplates and before the measurement were loaded with a fluorescent Ca²⁺-sensitive dye, fluo-4/AM (2 µM): after removing the culture medium the dye was added to the cells (dissolved in assay buffer: 145 mM NaCl, 5 mM KCl, 2 mM MgCl₂, 2 mM CaCl₂, 10 mM HEPES, 20 mM D-glucose, 2 mM probenecid, 100 µl/well) and cells were incubated at 37°C in a humidified incubator in an atmosphere of 5% CO₂/95% air for 40-120 min. To stop dye loading cells were washed twice with assay buffer. After washing, various concentrations of the test compounds (diluted in extracellular medium from a DMSO stock solution, final DMSO concentration was <0.1%) or buffer were added to each well depending on the experimental setup. After incubation at 37°C for 20-25 min. baseline and agonist-evoked changes of [Ca²⁺]ᵢ were measured column by column with a plate reader fluorimeter (Fluoroskan Ascent, Labsystems). Excitation and detection of emission was carried out from the bottom of the plate. Filters used for Fluo-4: excitation filter - 485 nm, emission filter - 538 nm. The whole measurement process was performed at 37°C and was controlled by custom software. Inhibitory potency of the test compounds was assessed by measuring the reduction in the agonist-evoked [Ca²⁺]ᵢ-elevation in the presence of different concentrations of the compounds. The agonists were LysDABK for CHO-B1, and bradykinin for CHO-B2 cells. Agonists were applied at an EC₈₀ concentration, the EC₈₀-values were derived from daily determined dose-response curves. Fluorescence data were expressed as ΔF/F (fluorescence change normalized to baseline). All treatments on a single plate were measured in multiple wells. Data from all wells with the same treatment were averaged and the average values were used for analysis. Inhibitory potency of a compound at a single concentration point was expressed as percent inhibition of the control agonist response. Sigmoidal concentration-inhibition curves were fitted to the data (derived from at least three independent experiments) and IC₅₀-values were determined as the concentration that produces half of the maximal inhibition caused by the compound.

The examined reference compounds measured in functional and binding tests are the following:
1) 4- {2-[(2,2-diphenyl-ethyl)-amino]-5-{4-[4-[(4-methyl-1-piperazinyl)-carbonyl]-1-piperidinyl]-sulfonyl}-benzoyl}-morpholine (NVP-SAA164, Br. J. Pharmacol.144 (2005) 889-899); Kᵢ 8 nM; IC₅₀: 33 nM;
2) (R)-N-[2,3-dihydro-2-oxo-5-(2-phenyl-ethyl)-1-propyl-1H-1,4-benzodiazepin-3-yl]-N'-{4-[4-(4-pyridinyl)-l-piperazinyl]-phenyl}-urea (J. Med. Chem. 46 (2003) 1803-1806); Kᵢ 0.59 nM; IC₅₀ 1.9 nM;
3) N-[4-(1,4'-bipiperidin)-1'-ylphenyl]-N'-[(3R)-2,3-dihydro-5-(4-methyl-phenyl)-2-oxo-1-propyl-1H-1,4-benzodiazepin-3-yl]-urea (J. Med. Chem. 46 (2003) 1803-1806); Kᵢ 13.4 nM; IC₅₀ 64.5 nM

The Kᵢ and IC₅₀ data measured by us for the reference compounds are in good agreement with the data given in the literature.

In Table 1 the most effective compounds of this invention measured in functional assay are listed.

**Table 1**

| **Number of example** | **B1 func.** | **Number of example** | **B1 func.** |
|---|---|---|---|
| **1** | ++++ | **25** | ++++ |
| **2** | ++++ | **27** | ++++ |
| **3** | ++++ | **28** | ++++ |
| **4** | ++++ | **29** | ++++ |
| **5** | +++ | **30** | ++++ |
| **6** | ++++ | **31** | ++++ |
| **7** | ++++ | **37** | ++++ |
| **8** | ++++ | **39** | ++++ |
| **10** | ++++ | **48** | ++++ |
| **11** | +++ | **49** | ++++ |
| **12** | ++++ | **50** | ++++ |
| **13** | ++++ | **54** | ++++ |
| **14** | ++++ | **56** | ++++ |
| **15** | ++++ | **60** | ++++ |
| **16** | ++++ | **61** | ++++ |
| **18** | ++++ | **62** | ++++ |
| **22** | ++++ | **63** | ++++ |
| **23** | ++++ | **66** | ++++ |

| | | | |
|---|---|---|---|
| + IC₅₀ > 0.5 µM +++ IC₅₀ is between 20 and 100 nM ++ IC₅₀ is between 0.1 and 0.5 µM ++++ IC₅₀ < 20 nM | | | |

### Receptor binding assays

### 1. Human recombinant bradykinin B1 receptor binding

Binding assays were carried out on human recombinant bradykinin1 receptors (expressed in CHO cells) according to the Euroscreen Technical Data Sheet (Cat.No.:ES-091). 20µg protein/tube was incubated with [3,4-prolyl-3,4-³H(N)]-[Des-Arg¹⁰] Kallidin as radioligand. Non specific binding was determined in the presence of 10 µM Lys-des-Arg⁹-Bradykinin. The final incubation volume was 250 µl. Samples were incubated for 15 min. at 25 °C then were rapidly vacuum filtered through GF/B filters presoaked for at least 1 h in 0.5 % PEI. Radioactivity was determined by liquid scintillation spectroscopy.

In Table 2 the most effective compounds of this invention measured in binding assay are listed.

**Table 2**

| **Number of example** | **B1 binding** | **Number of example** | **B1 binding** |
|---|---|---|---|
| **1** | ++++ | **29** | ++++ |
| **2** | ++++ | **30** | ++++ |
| **3** | ++++ | **31** | ++++ |
| **4** | ++++ | **35** | ++++ |
| **5** | +++ | **37** | ++++ |
| **6** | ++++ | **39** | ++++ |
| **7** | ++++ | **43** | ++++ |
| **8** | ++++ | **48** | ++++ |
| **10** | ++++ | **49** | ++++ |
| **11** | +++ | **50** | ++++ |
| **12** | ++++ | **54** | ++++ |
| **13** | ++++ | **56** | ++++ |
| **14** | ++++ | **59** | ++++ |
| **15** | ++++ | **60** | ++++ |
| **16** | ++++ | **61** | ++++ |
| **18** | ++++ | **62** | ++++ |
| **22** | ++++ | **63** | ++++ |
| **23** | ++++ | **66** | ++++ |
| **25** | ++++ | **73** | ++++ |
| **27** | ++++ | **103** | ++++ |
| **28** | ++++ | | |

| | | | |
|---|---|---|---|
| + Kᵢ > 0.5 µM +++ Kᵢ is between 20 and 100 nM ++ Kᵢ is between 0.1 and 0.5 µM ++++ Kᵢ < 20 nM | | | |

### 2. Human recombinant bradykinin B2 receptor binding

Binding assays were carried out on human recombinant bradykinin2 receptors (expressed in CHO cells) according to the Receptor Biology Technical Data Sheet (Cat.No.:RBHB2M) with minor modifications. 8.4 µg protein/tube was incubated with [2,3,-prolyl-3,4-³H(N)]-Bradykinin as radioligand. Non specific binding was determined in the presence of 5 µM bradykinin. The final incubation volume was 200 µl. Samples were incubated for 90 min. at +4 °C then were rapidly vacuum filtered through GF/B filters presoaked for at least 1 h in 0.5 % PEI. Radioactivity was determined by liquid scintillation spectroscopy.

The compounds exhibited high affinity and selectivity (>50 fold) for the human B1 receptor over the human B2 receptor according to both functional and binding assays.

The synthesis of compounds and pharmaceutical compositions according to the invention is illustrated by the following not limiting Examples.

### Reference Example 1

### 4-[(2-Amino-acetylamin)-methyl]-piperidine-1-carboxylic acid tert-butyl ester

### a) 4-[2-Benzyloxycarbonylamino-acetylamino)-methyl]-piperidine-1-carboxylic acid tert-butyl ester

A solution of Z-glycine (Aldrich) (1.95 g, 9.32 mmol), HOBt [1-hydroxybenzotriazol] (1.26 g, 9.32 mmol) and EDC hydrochloride [*N*-(3-dimethylaminopropyl)-*N*'-ethylcarbodiimide] (1.79 g, 9.32 mmol) in dry dimethylformamide (20 mL) was stirred at 0 °C for ten minutes before 4-aminomethyl-piperidine-1-carboxylic acid *tert*-butyl ester (Fluka) (2.0 g, 9.32 mmol) was added and the reaction mixture was stirred at room temperature overnight. The mixture was concentrated *in vacuo,* the residue was treated with saturated sodium hydrogencarbonate solution (50 mL), extracted with chloroform (3x50 mL), the combined organic layers were dried over sodium sulfate, filtered and concentrated. The residue was purified by column chromatography using Kieselgel 60 (0.040-0.063 mm) (Merck) as adsorbent, and chloroform:methanol = 25:1 as eluent to yield 3.57 g (94.5 %) of the title compound as white amorphous solid. MS (EI) 428.2 (M+Na⁺).

### b)4-[(2-Amino-acetylamino)-methyl]-piperidine-1-carboxylic acid tert-butyl ester

A stirred suspension of 4-[(2-benzyloxycarbonylamino-acetylamino)-methyl]-piperidine-1-carboxylic acid *tert*-butyl ester (3.57 g, 8.80 mmol) and 10 % Pd/C (0.36 g) in methanol : acetic acid : water 5:1:1 was hydrogenated at room temperature for 3 h. The catalyst was filtered off and the filtrate was concentrated *in vacuo.* The remaining crude material was purified by column chromatography using Kieselgel 60 (0.015-0.040 mm) (Merck) as adsorbent, and chloroform:methanol:ammonium hydroxide = 25:1:0.1 as eluent to yield 1.62 g (64.0 %) of the title compound as white amorphous solid. MS (EI) 294.3 (M+Na⁺).

### Reference Example 2

### 4-[2-(2-Amino-acetylamino)-ethyl]-piperidine-1-carboxylic acid tert-butyl ester

### a) 4-[2-(2-Benzyloxycarbonylamino-acetylamino)-ethyl]-piperidine-1-carboxylic acid tert-butyl ester

The title compound was prepared from 4-(2-amino-ethyl)-piperidine-1-carboxylic acid *tert*-butyl ester *[*Bioorg. Med. Chem. Lett.; 13 (2003) 2167-2172.] and Z-glycine according to the method described in Reference Example 1/a. MS (EI) 442.2 (M+Na⁺).

### b) 4-(2-(2-Amino-acetylamino)-ethyl)-piperidine-1-carboxylic acid tert-butyl ester

The title compound was prepared from 4-[2-(2-benzyloxycarbonylamino-acetylamino)-ethyl]-piperidine-1-carboxylic acid *tert*-butyl ester according to the method described in Reference Example 1/b. MS (EI) 286.2 (MH⁺).

### Reference Example 3

### (3-[1,4']Bipiperidinyl-1'-yl)-propylamine trihydrochloride

### a) (3-[1,4']Bipiperidinyl-1'-yl-propyl)-carbamic acid tert-butyl ester

A mixture of 4-piperidinopiperidine (Aldrich) (2.0 g, 11.88 mmol), (3-bromopropyl)-carbamic acid *ter*-t-butyl ester [Eur. J. Med. Chem. Chim. Ther. 37 (2002) 573-584] (3.96 g, 16.63 mmol), dimethylformamide (130 mL) and potassium carbonate (1.64 g, 11.88 mmol) was stirred at room temperature overnight, then concentrated *in vacou.* The residue was dissolved in water (150 mL), extracted with dichloromethane (3x150 mL), the combined organic layers were washed with brine (150 mL), dried over sodium sulfate, filtered and concentrated. The crude product was submitted to column chromatography using Kieselgel 60 (0.040-0.063 mm) (Merck) as adsorbent, and chloroform:methanol:ammonium hydroxide = 10:1:0.1 as eluent to yield 2.27 g (59 %) of the title compound as an oil.

### b) 3-[1,4']Bipiperidinyl-1'-yl-propylamine trihydrochloride

A mixture of (3-[1,4']bipiperidinyl-1'-yl-propyl)-carbamic acid *tert*-butyl ester (2.15 g, 6.6 mmol), dry dioxane (40 mL) and 6.5 N hydrogen chloride in dioxane (22 mL) was stirred at room temperature overnight, then diluted with diethyl ether and stirred at 0 °C for 1 h. The precipitated crystals were filtered off, washed with diethyl ether and dried to yield 2.03 g (92 %) of the title compound as a beige solid. Mp: 305-307 °C.

### Reference Example 4

### Trans-4-(2-pyrrolidin-1-yl-ethyl)-cyclohexylamine dihydrochloride

### a) Trans-2-{1-[4-(N-tert-butoxynarbonyl)-amino]-cyclohexyl}-ethanol

A solution of trans-2-{1-[4-(*N*-*tert*-butoxycarbonyl)-amino]-cyclohexyl}-acetic acid methyl ester [J. Med. Chem. 43 (2000) 1878-1885] (28.5 g, 105.2 mmol) in dry tetrahydrofuran (500 mL) was cooled to -2 °C, lithium aluminum hydride (5.4 g, 142 mmol) was added portionwise and the mixture was stirred at -2 °C for 60 minutes. The reaction mixture was cooled to -10 °C and quenched with ethyl acetate (15 mL), then brine (43 ml) was slowly added to the mixture at 0 °C. The precipitated salts were filtered, and washed with ethyl acetate. The filtrate was concentrated *in vacuo.* The residue was recrystallized from diisopropyl ether (100 ml) to yield 23.7 g (93 %) of the title compound as a white powder. Mp: 100-102 °C.

### b) Methanesulfonic acid trans-2-(4-tert-butoxycarbonylamino-cyclohexyl)-ethyl ester

To a stirred solution of trans-2-{1-[4-(N-*tert*-butoxycarbonyl)-amino]-cyclohexyl}-ethanol (15 g, 62 mmol), and triethylamine (10.5 mL, 75 mmol) in dry dichloromethane (150 mL) methanesulfonyl chloride (5.7 mL, 73.4 mmol) in dichloromethane (25 mL) was added dropwise at 0 °C. After stirring 30 minutes at 0 °C, the solution was extracted three times with water. The organic solution was dried over sodium sulfate and concentrated *in vacuo* to yield 13.0 g (65 %) of the title compound. Mp:116°C.

### c) Trans-[4-(2-pyrrolidin-1-yl-ethyl)-cyclohexyl]-carbamic acid tert-butyl ester

A mixture of methanesulfonic acid trans-2-(4-*tert*-butoxycarbonylaminocyclohexyl)-ethyl ester (3.2 g, 10 mmol), potassium carbonate (1.4 g, 10 mmol) and pyrrolidine (1.25 mL, 15 mmol) in acetonitrile (40 mL) was stirred at 60 °C for 2 hours. The mixture was cooled to room temperature and poured into water (200 mL). The precipitated white crystals were filtered off and washed with water to yield 1.9 g (64 %) of the title compound. Mp: 110 °C

### d) Trans-4-(2-pyrrolidin-1-yl-ethyl)-cyclohexylamine dihydrochloride

The title compound was prepared from trans-[4-(2-pyrrolidin-1-yl-ethyl)-cyclohexyl]-carbamic acid *tert*-butyl ester according to the method described in Reference Example 3/b. Mp: 331-336 °C

### Reference Example 5

### 4-(4-Amino-butyl)-piperidine-1-carboxylic acid tert-butyl ester

### a) 4-(3-Methanesulfonyloxy-propyl)-piperidine-1-carboxylic acid tert-butyl ester

To a solution of 4-(3-hydroxypropyl)-piperidine-1-carboxylic acid *tert*-butyl ester (2.12 g, 8.7 mmol) in dichloromethane (10 mL) triethylamine (1.33 mL, 9.57 mmol) and methanesulfonyl chloride (0.74 mL, 9.57 mmol) were added at 0 °C and the reaction mixture was stirred at this temperature for 1 h. After quenching the reaction by the addition of methanol (1 mL), the mixture was washed with water, saturated sodium hydrogencarbonate solution and water, dried over sodium sulfate, filtered and concentrated to yield 2.73 g (97%) of the title compound.

### b) 4-(3-Cyano-propyl)-piperidine-1-carboxylic acid tert-butyl ester

To a solution of 4-(3-methanesulfonyloxy-propyl)-piperidine-1-carboxylic acid *tert*-butyl ester (1.35 g, 4.2 mmol) in dimethylformamide (30 mL) potassium cyanide (0.33 g, 5.1 mmol) was added and the reaction mixture was stirred at 80 °C for 20 h. After concentration *in vacuo* the residue was treated with water and extracted with ethyl acetate (3x50mL). The combined organic layers were washed with water and brine, dried over sodium sulfate, filtered and concentrated to yield 0.919 g (87%) of the title compound.

### c) 4-(4-Amino-butyl)-piperidine-1-carboxylic acid tert-butyl ester

To a stirred solution of 4-(3-cyano-propyl)-piperidine-1-carboxylic acid *tert-*butyl ester (0.896 g, 3.55 mmol) and lithium hydroxide hydrate (0.447 g, 10.65 mmol) in a mixture of dioxane (56 mL) and water (14 mL) 10% Pd/C (90 mg) and Raney Ni (0.42 g) were added. The reaction mixture was hydrogenated at 50 °C for 3 h, then the catalysts were filtered off and the filtrate was concentrated. The residue was purified by column chromatography using Kieselgel 60 (0.015-0.040 mm) (Merck) as adsorbent, and methanol:ammonium hydroxide = 10:1 as eluent to yield 0.493 g (54 %) of the title compound. MS (EI) 257.2 (MH⁺).

### Reference Example 6

### 4-(3-Amino-propyl)-piperidine-1-carboxylic acid tert-butyl ester

### a) 4-(3-Azido-propyl)-piperidine-1-carboxylic acid tert-butyl ester

To a solution of 4-(3-methanesulfonyloxy-propyl)-piperidine-1-carboxylic acid *tert*-butyl ester (Reference Example 5/a) (1.35 g, 4.2 mmol) in dimethylformamide (30 mL) sodium azide (0.33 g, 5.1 mmol) was added and the reaction mixture was stirred at 80 °C for 20 h. After concentration *in vacuo* the residue was treated with water and extracted with ethyl acetate (3x50mL). The combined organic layers were washed with water and brine, dried over sodium sulfate, filtered and concentrated to yield 1.08 g (96 %) of the title. MS (EI) 291.3 (M+Na⁺).

### b) 4-(3-Amino-propyl)-piperidine-1-carboxylic acid tert-butyl ester

To a solution of 4-(3-azido-propyl)-piperidine-1-carboxylic acid *tert*-butyl ester (1.738 g, 6.48 mmol) in dry tetrahydrofuran (50 mL) water (0.49 mL) and triphenyl phosphine (3.4 g, 12.96 mmol) were added. The reaction mixture was stirred at room temperature overnight, then concentrated. The residue was purified by column chromatography using Kieselgel 60 (0.015-0.040 mm) (Merck) as adsorbent, and methanol:ammonium hydroxide = 10:1 as eluent to yield 1.498 g (95 %) of the title compound. MS (EI) 243.2 (MH⁺).

### Reference Example 7

### N-(4-Aminomethyl-benzyl)-guanidine dihydrochloride

### a) (4-(N,N'-Ditert-butoxycarbonyl-guanidinomethyl-benzyl))-carbamic acid tert-butyl ester

A mixture of (4-aminomethyl-benzyl)-carbamic acid *tert*-butyl ester (Aldrich) (0.47 g, 2 mmol), 1-methyl-di*tert*-butoxy-thiourea [J. Org. Chem. 52 (1987) 1700-1703] (0.6 g, 2 mmol), HgCl₂ (0.56 g, 2 mmol) and dimethylformamide (10 mL) was stirred at room temperature for 48 hours. The precipitated salts were filtered off and the filtrate was concentrated *in vacuo.* The remaining oil was dissolved in chloroform (70 mL), washed with water (3x40 mL), dried over sodium sulfate and concentrated to yield 0.65 g (68 %) of the title compound.

### b) N-(4-Aminomethyl-benzyl)-guanidine dihydrochloride

The title compound was prepared from (4-(*N*,*N'*-di*tert*-butoxycarbonyl-guanidinomethyl-benzyl))-carbamic acid *tert*-butyl ester according to the method described in Reference Example 3/b.

### Reference Example 8

### 2-(4-Pyridin-4-yl-piperazin-1-yl)-propylamine

### a) 2-[2-(4-Pyridin-4-yl-piperazin-1-yl)-propyl]-isoindole-1,3-dione

The title compound was prepared from 1-pyridin-4-yl-piperazine [Org. Lett. 4 (2002) 737-740] and N-(2-bromopropyl)-phthalimide according to the method described in Reference Example 1/a.

### b) 2-(4-Pyridin-4-yl-piperazin-1-yl)-propylamine

The title compound was prepared from 2-[2-(4-pyridin-4-yl-piperazin-1-yl)-propyl]-isoindole-1,3-dione according to the method described in Reference Example 1/b.

### Example 1

### 4-[2-(2,4-Dichloro-phenoxy)-phenylsulfamoyl]-N-{[(piperidin-4-ylmethyl)-carbamoyl]-methyl}-benzamide hydrochloride

### a) 2,4-Dichloro-1-(2-nitro-phenoxy)-benzene

A mixture of 1-fluoro-2-nitrobenzene (4.8 mL, 45.42 mmol), potassium carbonate (13.8 g, 0.1 mol) and 2,4-dichloro-phenol (8.16 g, 50.06 mmol) in dry dimethylformamide (70 mL) was stirred at 100 °C for 2 h. Solids were filtered off, and the filtrate was concentrated in vacuo. The residue was partitioned between diethyl ether and 1N sodium hydroxide, the organic layer was washed with 1N sodium hydroxide, water and brine, dried over sodium sulfate, filtered and concentrated *in vacuo* to yield 11.69 g (91 %) of the title compound as a yellowish oil, which solidifies on standing. Mp: 58-59 °C. MS (EI) 285.2 (MH⁺). Lit. [Chem. Heterocycl. Compd. (Engl. Transl.) 11 (1975) 1356-1358] Mp: 57-58 °C.

### b) 2-(2,4-Dichloro-phenoxy)-phenylamine [Chem. Abstr. 84 (1976) 164313q]

To a stirred solution of 2,4-dichloro-1-(2-nitro-phenoxy)-benzene (3.5 g, 12.32 mmol) in ethyl acetate (60 mL) stannous chloride dihydrate (13.89 g, 61.6 mmol) was added and the mixture was refluxed for 2 h before it was quenched with saturated sodium hydrogencarbonate solution (192 mL). The organic phase was separated and the aqueous phase was washed several times with ethyl acetate. The combined extracts were dried over sodium sulfate, filtered and concentrated in vacuo to yield 3.1 g (99 %) of the title compound as a yellowish oil: MS (EI) 255.2 (MH⁺).

### c) 4-[2-(2,4-Dichloro-phenoxy)-phenylsulfamoyl]-benzoic acid

Under an atmosphere of argon to an ice cooled solution of 2-(2,4-dichlorophenoxy)-phenylamine (0.5 g, 1.97 mmol) in dry pyridine (5 mL) 4-chlorosulfonyl benzoic acid (0.45 g, 1.97 mmol) was added portion-wise. The reaction mixture was stirred at room temperature overnight. The mixture was evaporated *in vacuo,* the residue was treated with 1N hydrochloric acid (20 mL), and extracted with ethyl acetate (3x50 mL). The combined organic layers were washed with 1N hydrochloric acid, water and brine, dried over sodium sulfate, filtered and concentrated in vacuo. The residue was submitted to flash column chromatography using Kieselgel 60 (0.015-0.040 mm) as adsorbent (Merck) and chloroform:methanol:acetic acid = 294:6:1 as eluent to yield 0.6 g (70 %) of the title compound as a light pink solid, which was crystallized from diethyl ether-petroleum ether. MS (EI) 439.3 (MH⁺).

### d) 4-[(2-{4-[2-(2,4-Dichloro-phenoxy)-phenylsulfamoyl]-benzoylamino}-acetylamino)-methyl]-piperidine-1-carboxylic acid tert-butyl ester

The solution of 4-[2-(2,4-dichloro-phenoxy)-phenylsulfamoyl]-benzoic acid (3.07 g, 7.0 mmol), triethylamine (1.0 mL, 7.1 mmol) and HBTU [O-benzotriazol-1-yl-*N,N,N',N*'-tetramethyluronium hexafluorophosphate (Advanced Chem. Tech.)] (3.21 g, 8.45 mmol) in dry dimethylformamide (100 mL) was stirred at room temperature for five minutes before 4-[(2-amino-acetylamino)-methyl]-piperidine-1-carboxylic acid *tert*-butyl ester (Reference Example 1) (1.9 g, 7.0 mmol) was added. The pH of the reaction mixture was adjusted to 8 by the addition of triethylamine, the so obtained mixture was stirred at room temperature overnight, then concentrated *in vacuo.* The residue was treated with saturated sodium hydrogencarbonate solution (100 mL), extracted with ethyl acetate (3x100 mL), the combined organic layers were washed with saturated sodium hydrogencarbonate solution, water and brine, dried over sodium sulfate, filtered and concentrated. The residue was purified by column chromatography using Kieselgel 60 (0.040-0.063 mm) as adsorbent (Merck) and toluene:acetone = 2:1 as eluent to yield 2.86 g (59 %) of the title compound as light yellowish amorphous solid. MS (EI) 714 (M+Na⁺).

### e) 4-[2-(2,4-Dichloro-phenoxy)-phenylsulfamoyl-N-{[(piperidin-4-ylmethyl)-carbamoyl]-methyl}-benzamide hydrochloride

To a stirred solution of 4-(2-{4-[2-(2,4-dichloro-phenoxy)-phenylsulfamoyl]-benzoylamino}-acetylamino)-methyl]-piperidine-1-carboxylic acid *tert*-butyl ester (1.07 g, 1.55 mmol) in dichloromethane (11 mL) 9 M hydrogen chloride in ethanol (0.91 mL) was added. The reaction mixture was stirred at room temperature for 2h, then diethyl ether (36 mL) was added, the precipitated crystals were filtered, washed with diethyl ether and dried to yield 0.655 g (67 %) of the title compound. MS (EI) 592.1 (MH⁺).

### Example 2

### 4-[2-(2,4-Dichloro-phenoxy)-phenylsulfamoyl]-N-[(3-piperidin-1-yl-propylcarbamoyl)-methyl]-benzamide

### a) {4-[2-(2,4-Dichloro-phenoxy)-phenylsulfamoyl]-benzoylamino}-acetic acid ethyl ester

The solution of 4-[2-(2,4-dichloro-phenoxy)-phenylsulfamoyl]-benzoic acid (Example 1/c) (8.207 g, 18.7 mmol), triethylamine (5.2 mL, 37.4 mmol) and HBTU (8.24 g, 21.7 mmol) in dry dimethyl formamide (150 mL) was stirred at room temperature for five minutes before glycine ethyl ester hydrochloride (Aldrich) (2.614 g, 18.7 mmol) was added. The pH of the reaction mixture was adjusted to 8 by the addition of triethylamine, the so obtained mixture was stirred at room temperature overnight, then concentrated *in vacuo.* The residue was treated with saturated sodium hydrogencarbonate solution (300 mL), the precipitated crystals were filtered off, washed with water and dried. The crude product was purified by column chromatography using Kieselgel 60 (0.040-0.063 mm) (Merck) as adsorbent, and n-hexane:ethyl acetate = 2:1 as eluent to yield 7.68 g (78 %) of the title compound. MS (EI) 524 (MH⁺).

### b) {4-[2-(2,4-Dichloro-phenoxy)-phenylsulfamoyl]-benzoylamino}-acetic acid

To a stirred solution of {4-[2-(2,4-dichloro-phenoxy)-phenylsulfamoyl]-benzoylamino}-acetic acid ethyl ester (7.68 g, 14.67 mmol) in a mixture of tetrahydrofuran (36 mL), water (18 mL) and methanol (18 mL) lithium hydroxide monohydrate (3.09 g, 73.64 mmol) was added and the reaction mixture was stirred at room temperature for 2 h. The mixture was concentrated, the residue was dissolved in water, acidified with 1M hydrochloric acid, the precipitated solid was filtered off, washed with water and dried to yield 6.76 g (93 %) of the title compound as a yellowish solid. MS (EI) 496.2 (MH⁺).

### c) 4-[2-(2,4-Dichloro-phenoxy)-phenylsulfamoyl]-N-[(3-piperidin-1-yl-propylcarbamoyl)-methyl]-benzamide

To a stirred solution of {4-[2-(2,4-dichloro-phenoxy)-phenylsulfamoyl]-benzoylamino}-acetic acid (42 mg, 0.085 mmol) in a mixture of dicloromethane (2 mL) and dimethylformamide (0.2 mL) 3-piperidin-1-yl-propylamine (EMKA-Chemie) (14 mg, 0.1 mmol), HBTU (46 mg, 0.12 mmol) and triethylamine (60 µL, 0.4 mmol) were added. The mixture was stirred at room temperature for 24 h, then purified by column chromatography using Kieselgel 60 (0.015-0.040 mm) as adsorbent (Merck) and gradient elution starting with 100% A eluent and processing to a mixture of 70% A and 30% B eluent over a period of 15 minutes (eluent A: chloroform; eluent B: methanol containing 5% of ammonium hydroxide) to yield 50.2 mg (95 %) of the title compound. MS (EI) 620.2 (MH⁺).

### Example 3

### 4-[2-(4-Bromo-phenoxy)-phenylsulfamoyl]-N-{[(piperidin-4-ylmethyl)-carbamoyl]-methyl}-benzamide trifluoroacetate

### a) 4-[2-(4-Bromo-phenoxy)-phenylsulfamoyl]-benzoic acid

To a stirred solution of 2-(4-bromo-phenoxy)-phenylamine [J. Chem.. Soc. (1930) 1202, 1206] (1.708 g, 6.5 mmol) in dry pyridine (10 mL) 4-chlorosulfonyl benzoic acid (1.66 g, 7.5 mmol) was added. The reaction mixture was stirred at room temperature overnight, then poured into ice-water (100 mL). The precipitated crystals were filtered off, washed with water and dried to yield 2.37 g (81 %) of the title compound. MS (EI) 449.2 (MH⁺).

### b) 4-[(2-{4-[2-(4-Bromo-phenoxy)-phenylsulfamoyl]-benzoylamino}-acetylamino)-methyl]-piperidine-1-carboxylic acid tert-butyl ester

The title compound was prepared from 4-[2-(4-bromo-phenoxy)-phenylsulfamoyl]-benzoic acid and 4-[(2-amino-acetylamino)-methyl]-piperidine-1-carboxylic acid *tert*-butyl ester (Reference Example 1) according to the method described in Example 1/d. MS (EI) 724.2 (M+Na⁺).

### c) 4-[2-(4-Bromo-phenoxy)-phenylsulfamoyl]-N-{[(piperidin-4-ylmethyl)-carbamoyl]-methyl}-benzamide trifluoroacetate

To an ice cold solution of 4-[(2-{4-[2-(4-bromo-phenoxy)-phenylsulfamoyl]-benzoylamino}-acetylamino)-methyl]-piperidine-1-carboxylic acid *tert*-butyl ester (0.80 g, 1.14 mmol) in dichloromethane (40 mL) trifluoroacetic acid (10 mL) was added and the reaction mixture was stirred at room temperature overnight, then concentrated. The residue was triturated with diethyl ether, filtered and dried to yield 0.748 g (82 %) of the title compound as a white amorphous solid. MS (EI) 602.1 (MH⁺).

### Example 4

### 4-[2-(4-Bromo-phenoxy)-5-fluoro-phenylsulfamoyl]-N-{[(piperidin-4-ylmethyl)-carbamoyl]-methyl}-benzamide trifluoroacetate

### a) 4-[2-(4-Bromo-phenoxy)-5-fluoro-phenylsulfamoyl]-benzoic acid

Under an atmosphere of argon to an ice cooled solution of 2-(4-bromophenoxy)-5-fluoro-phenylamine [Yakugaku Zasshi; 87 (1967) 591, 594; Chem.Abstr.; 67 (1967) 73282] (0.43 g, 1.52 mmol) in dry pyridine (10 mL) 4-chlorosulfonyl benzoic acid (0.34 g, 1.52 mmol) was added portion-wise. The reaction mixture was stirred at room temperature overnight. The mixture was concentrated *in vacuo,* the residue was treated with 1N hydrochloric acid (15 mL), and extracted with ethyl acetate (3x20 mL). The combined organic layers were washed with 1N hydrochloric acid, water and brine, dried over sodium sulfate, filtered and concentrated *in vacuo.* The residue was triturated with diethyl ether, filtered, washed with diethyl ether and dried to yield 0.31 g (43 %) of the title compound as a light pink solid. MS (EI) 467.9 (MH⁺).

### b) {4-[2-(4-Bromo-phenoxy)-5-fluoro-phenylsulfamoyl]-benzoylamino}-acetic acid ethyl ester

The title compound was prepared from 4-[2-(4-bromo-phenoxy)-5-fluorophenylsulfamoyl]-benzoic acid and glycine ethyl ester hydrochloride (Aldrich) according to the method described in Example 1/d. MS (EI) 552.0 (MH⁺).

### c) {4-[2-(4-Bromo-phenoxy)-5-fluoro-phoylsulfamoyl]-benzoylamino}-acetic acid

The title compound was prepared from {4-[2-(4-bromo-phenoxy)-5-fluorophenylsulfamoyl]-benzoylamino}-acetic acid ethyl ester according to the method described in Example 2/b. MS (EI) 524.2 (MH⁺).

### d) 4-[(2-{4-[2-(4-Bromo-phenoxy)-5-fluoro-phenylsulfamoyl]-benzoylamino}-acetylamino)-methyl]-piperidine-1-carboxylic acid tert-butyl ester

The title compound was prepared from {4-[2-(4-bromo-phenoxy)-5-fluoro-phenylsulfamoyl]-benzoylamino}-acetic acid and 4-aminomethyl-piperidine-1-carboxylic acid tert-butyl ester (Fluka) according to the method described in Example 1/d. MS (EI) 743.3 (M+Na⁺).

### e) 4-[2-(4-Bromo-phenoxy)-5-fluoro-phenylsulfamoyl]-N-{[(piperidin-4-ylmethyl)-carbamoyl]-methyl}-benzamide mono trifluoroacetate

The title compound was prepared from 4-[(2-{4-[2-(4-bromo-phenoxy)-5-fluoro-phenylsulfamoyl]-benzoylamino}-acetylamino)-methyl]-piperidine-1-carboxylic acid tert-butyl ester according to the method described in Example 3/c. MS (EI) 620.2 (MH⁺).

### Example 5

### 4-(2-Phenoxy-phenylsulfamoyl)-N-{[(piperidin-4-ylmethyl)-carbamoyl]-methyl}-benzamide hydrochloride

### a) 4-(2-Phenoxy-phenylsulfamoyl)-benzoic acid

The title compound was prepared from 2-phenoxy-phenylamine (Aldrich) according to the method described in Example 3/a. MS (EI) 370.2 (MH⁺).

### b) 4-({2-[4-(2-Phenoxy-phenylsulfamoyl)-benzoylamino]-acetylamino}-methyl)-piperidine-1-carboxylic acid tert-butyl ester

The title compound was prepared from 4-(2-phenoxy-phenylsulfamoyl)-benzoic acid and 4-[(2-amino-acetylamino)-methyl]-piperidine-1-carboxylic acid *tert*-butyl ester (Reference Example 1) according to the method described in Example 1/d. MS (EI) 645.2 (M+Na⁺).

### c) 4-(2-Phenoxy-phenylsulfamoyl)-N-{[(piperidin-4-ylmethyl)-carbamoyl]-methyl}-benzamide hydrochloride

The title compound was prepared from 4-({2-[4-(2-phenoxy-phenylsulfamoyl)-benzoylamino]-acetylamino}-methyl)-piperidine-1-carboxylic acid *tert*-butyl ester according to the method described in Example 1/e. MS (EI) 523.2 (MH⁺).

### Example 6

### 4-[2-(2,4-Dichloro-phenoxy)-phenylsulfamoyl]-N-{2-phenyl-1-[(piperidin-4-ylmethyl)-carbamoyl]-ethyl}-benzamide hydrochloride

### a) 2-{4-[2-(2,4-Dichloro-phenoxy)-phenylsulfamoyl]-benzoylamino}-3-phenylpropionic acid methyl ester

The title compound was prepared from 4-[2-(2,4-dichloro-phenoxy)-phenylsulfamoyl]-benzoic acid (Example 1/c) and (DL)-phenylalanine methyl ester hydrochloride according to the method described in Example 1/d. MS (EI) 600.2 (MH⁺).

### b) 2-{4-[2-(2,4-Dichloro-phenoxy)-phenylsulfamoyl]-benzoylamino}-3-phenylpropionic acid

The title compound was prepared from 2-{4-[2-(2,4-dichloro-phenoxy)-phenylsulfamoyl]-benzoylamino}-3-phenyl-propionic acid methyl ester according to the method described in Example 2/b. MS (EI) 586.2 (MH⁺).

### c) 4-[(2-{4-[2-(2,4-Dichloro-phenoxy)-phenylsulfamoyl]-benzoylamino}-3-phenyl-propionylamino)-methyl]-piperidine-1-carboxylic acid tert-butyl ester

The title compound was prepared from 2-{4-[2-(2,4-dichloro-phenoxy)-phenylsulfamoyl]-benzoylamino}-3-phenyl-propionic acid and 4-aminomethyl-piperidine-1-carboxylic acid *tert*-butyl ester according to the method described in Example 1/d. MS (EI) 804.1 (M+Na⁺).

### d) 4-[2-(2,4-Dichloro-phenoxy)-phenylsulfamoyl]-N-{2-phenyl-1-[(piperidin-4-ylmethyl)-carbamoyl]-ethyl}-benzamide hydrochloride

The title compound was prepared from 4-[(2-{4-[2-(2,4-dichloro-phenoxy)-phenylsulfamoyl]-benzoylamino}-3-phenyl-propionylamino)-methyl]-piperidine-1-carboxylic acid *tert*-butyl ester according to the method described in Example 1/e. MS (EI) 682.2 (MH⁺).

### Example 7

### 4-(5-Fluoro-2-phenoxy-phenylsulfamoyl)-N-{[(piperidin-4-ylmethyl)-carbamoyl]-methyl}-benzamide hydrochloride

### a) 4-(5-Fluoro-2-phenoxy-phenylsulfamoyl)-benzoic acid

The title compound was prepared from 5-fluoro-2-phenoxy-phenylamine [Bioorg. Med. Chem.; 12 (2004) 423-438] according to the method described in Example 1/c. MS (EI) 388.2 (MH⁺).

### b) 4-({2-[4-(5-Fluoro-2-phenoxy-phenylsulfamoyl)-benzoylamino]-acetylamino}-methyl)-piperidine-1-carboxylic acid tert-butyl ester

The title compound was prepared from 4-(5-fluoro-2-phenoxy-phenylsulfamoyl)-benzoic acid and 4-[(2-amino-acetylamino)-methyl]-piperidine-1-carboxylic acid *tert*-butyl ester (Reference Example 1) according to the method described in Example 1/d. MS (EI) 641.2 (MH⁺).

### c) 4-(5-Fluoro-2-phenoxy-phenylsulfamoyl)-N-{[(piperidin-4-ylmethyl)-carbamoyl]-methyl}-benzamide hydrochloride

The title compund was prepared from 4-({2-[4-(5-fluoro-2-phenoxy-phenylsulfamoyl)-benzoylamino]-acetylamino}-methyl)-piperidine-1-carboxylic acid *tert*-butyl ester according to the method described in Example 1/e. MS (EI) 541.2 (MH⁺).

### Example 8

### 4-[2-(2,4-Dichloro-phenoxy)-phenylsulfamoyl]-N-{(S)-1-[(piperidin-4-ylmethyl)-carbamoyl]-ethyl}-benzamide hydrochloride

### a) (S)-2-{4-[2-(2,4-Dichloro-phenoxy)-phenylsulfamoyl]-benzoylamino}-propionic acid benzyl ester

The title compound was prepared from 4-[2-(2,4-dichloro-phenoxy)-phenylsulfamoyl]-benzoic acid (Example 1/c) and (*S*)-2-amino-propionic acid benzyl ester according to the method described in Example 1/d. MS (EI) 600.2 (MH⁺).

### b (S)-2-{4-[2-(2,4-Dichloro-phenoxy)-phenylsulfamoyl]-benzoylamino}-propionic acid

The title compound was prepared from (*S*)-2-{4-[2-(2,4-dichloro-phenoxy)-phenylsulfamoyl]-benzoylamino}-propionic acid benzyl ester according to the method described in Example 2/b. MS (EI) 510.1 (MH⁺).

### c) 4-[((S)-2-{4-[2-(2,4-Dichloro-phenoxy)-phenylsulfamoyl]-benzoylamino}-propionylamino)-methyl]-piperidine-1-carboxylic acid tert-butyl ester

The title compound was prepared from (*S*)-2-{4-[2-(2,4-dichloro-phenoxy)-phenylsulfamoyl]-benzoylamino}-propionic acid and 4-aminomethyl-piperidine-1-carboxylic acid *tert*-butyl ester according to the method described in Example 1/d. MS (EI) 706.1 (MH⁺).

### d) 4-[2-(2,4-Dichloro-phenoxy)-phenylsulfamoyl]-N-{(S)-1-[(piperidin-4-ylmethyl)-carbamoyl]-ethyl}-benzamide hydrochloride

The title compound was prepared from 4-[((*S*)-2-{4-[2-(2,4-dichloro-phenoxy)-phenylsulfamoyl]-benzoylamino}-propionylamino)-methyl]-piperidine-1-carboxylic acid *tert*-butyl ester according to the method described in Example 1/e. MS (EI) 606.1 (MH⁺).

### Example 9

### 4-(Biphenyl-2-ylsulfamoyl)-N-{[(piperidin-4-ylmethyl)-carbamoyl]-methyl}-benzamide hydrochloride

### a) 4-(Biphenyl-2-ylsulfamoyl)-benzoic acid

The title compound was prepared from 2-amino-biphenyl (Aldrich) according to the method described in Example 1/c. MS (EI) 354.1 (MH⁺).

### b) 4-({2-[4-(Biphenyl-2-ylsulfamoyl)-benzoylamino]-acetylamino}-methyl)-piperidine-1-carboxylic acid tert-butyl ester

The title compound was prepared from 4-(biphenyl-2-ylsulfamoyl)-benzoic acid and 4-[(2-amino-acetylamino)-methyl]-piperidine-1-carboxylic acid *tert*-butyl ester (Reference Example 1) according to the method described in Example 1/d. MS (EI) 629.2 (M+Na⁺).

### c) 4-(Biphenyl-2-ylsulfamoyl)-N-{[(piperidin-4-ylmethyl)-carbamoyl]-methyl}-benzamide hydrochloride

The title compound was prepared from 4-({2-[4-(biphenyl-2-ylsulfamoyl)-benzoylamino]-acetylamino}-methyl)-piperidine-1-carboxylic acid *tert*-butyl ester according to the method described in Example 1/e. MS (EI) 507.2 (MH⁺).

### Example 10

### 4-(2-Phenoxy-phenylsulfamoyl)-N-[(2-piperidin-4-yl-ethylcarbamoyl)-methyl]-benzamide hydrochloride

### a) 4-(2-{2-[4-(2-Phenoxy-phenylsulfamoyl)-benzoylamino]-acetylamino}-ethyl)-piperidine-1-carboxylic acid tert-butyl ester

The title compound was prepared from 4-(2-phenoxy-phenylsulfamoyl)-benzoic acid (Example 5/a) and 4-[2-(2-amino-acetylamino)-ethyl]-piperidine-1-carboxylic acid *tert*-butyl ester (Reference Example 2) according to the method described in Example 1/d. MS (EI) 659.2 (M+Na⁺).

### b) 4-(2-Phenoxy-phenylsulfamoyl)-N-[(2-piperidin-4-yl-ethylcarbamoyl)-methyl]-benzamide hydrochloride

The title compound was prepared from 4-({2-[4-(2-phenoxy-phenylsulfamoyl)-benzoylamino]-acetylamino}-ethyl)-piperidine-1-carboxylic acid *tert*-butyl ester according to the method described in Example 1/e. MS (EI) 537.2 (MH⁺).

### Example 11

### 4-[2-(2,4-Dichloro-phenoxy)-4-methoxy-phenylsulfamoyl]-N-{[(piperidin-4-ylmethyl)-carbamoyl]-methyl}-benzamide hydrochloride

### a) 2,4-Dichloro-1-(5-methoxy-2-nitro-phenoxy)-benzene

The title compound was prepared from 2-fluoro-4-methoxy-1-nitro-benzene [J. Med. Chem.; 33 (1990) 286-291] and 2,4-dichloro-phenol according to the method described in Example 1/a. MS (EI) 315.1 (MH⁺).

### b) 2-(2,4-Dichloro-phenoxy)-4-methoxy-phenylamine

The title compound was prepared from 2,4-dichloro-1-(5-methoxy-2-nitrophenoxy)-benzene according to the method described in Example 1/b. MS (EI) 285.2 (MH⁺).

### c 4-[2-(2,4-Dichloro-phenoxy)-4-methoxy-phenylsulfamoyl]-benzoic acid

The title compound was prepared from 2-(2,4-dichloro-phenoxy)-4-methoxyphenylamine according to the method described in Example 1/c. MS (EI) 469.1 (MH⁺).

### d) 4-[(2-{4-[2-(2,4-Dichloro-phenoxy)-4-methox-phenylsulfamoyl]-benzoylamino}-acetylamino)-methyl]-piperidine-1-carboxylic acid tert-butyl ester

The title compound was prepared from 4-[2-(2,4-dichloro-phenoxy)-4-methoxy-phenylsulfamoyl]-benzoic acid and 4-[(2-amino-acetylamino)-methyl]-piperidine-1-carboxylic acid *tert*-butyl ester (Reference Example 1) according to the method described in Example 1/d. MS (EI) 744.2 (M+Na⁺).

### e) 4-[2-(2,4-Dichloro-phenoxy)-4-methoxy-phenylsulfamoyl]-N-{[(piperidin-4-ylmethyl)-carbamoyl]-methyl}-benzamide hydrochloride

The title compound was prepared from 4-[(2-{4-[2-(2,4-dichloro-phenoxy)-4-methoxy-phenylsulfamoyl]-benzoylamino}-acetylamino)-methyl]-piperidine-1-carboxylic acid *tert*-butyl ester according to the method described in Example 1/e. MS (EI) 622.2 (MH⁺).

### Example 12

### 4-[2-(4-Fluoro-phenoxy)-phenylsulfamoyl]-N-{[(piperidin-4-ylmethyl)-carbamoyl]-methyl}-benzamide hydrochloride

### a) 4-[2-(4-Fluoro-phenoxy)-phenylsulfamoyl]-benzoic acid

The title compound was prepared from 2-(4-fluoro-phenoxy)-phenylamine [Helv. Chim. Acta; 48 (1965) 336-347] according to the method described in Example 1/c. MS (EI) 388.2 (MH⁺).

### b) 4-[(2-{4-[2-(4-Fluoro-phenoxy)-phenylsulfamoyl]-benzoylamino}-acetylamino)-methyl]-piperidine-1-carboxylic acid tert-butyl ester

The title compound was prepared from 4-[2-(4-fluoro-phenoxy)-phenylsulfamoyl]-benzoic acid and 4-[(2-amino-acetylamino)-methyl]-piperidine-1-carboxylic acid *tert*-butyl ester (Reference Example 1) according to the method described in Example 1/d. MS (EI) 663.2 (M+Na⁺).

### c) 4-[2-(4-Fluoro-phenoxy)-phenylsulfamoyl]-N-{[(piperidin-4-ylmethyl)-carbamoyl]-methyl}-benzamide hydrochloride

The title compound was prepared from 4-[(2-{4-[2-(4-fluoro-phenoxy)-phenylsulfamoyl]-benzoylamino}-acetylamino)-methyl]-piperidine-1-carboxylic acid *tert*-butyl ester according to the method described in Example 1/e. MS (EI) 541.2 (MH⁺).

### Example 13

### 4-[5-Fluoro-2-(4-fluoro-phenoxy)-phenylsulfamoyl]-N-[(2-piperidin-4-yl-ethylcarbamoyl)-methyl]-benzamide hydrochloride

### a) 4-[5-Fluoro-2-(4-fluoro-phenoxy)-phenylsulfamoyl]-benzoic acid

The title compound was prepared from 5-fluoro-2-(4-fluoro-phenoxy)-phenylamine [Yakugaku Zasshi; 88 (1968) 1361, 1365; Chem.Abstr.; 70 (1969) 68312] according to the method described in Example 1/c. MS (EI) 406.3 (MH⁺).

### b) 4-[2-(2-{4-[5-Fluoro-2-(4-fluoro-phenoxy)-phenylsulfamoyl]-benzoylamino}-acetylamino)-ethyl]-piperidine-1-carboxylic acid tert-butyl ester

The title compound was prepared from 4-[5-fluoro-2-(4-fluoro-phenoxy)-phenylsulfamoyl]-benzoic acid and 4-[2-(2-amino-acetylamino)-ethyl]-piperidine-1-carboxylic acid *tert*-butyl ester (Reference Example 2) according to the method described in Example 1/d. MS (EI) 695.2 (M+Na⁺).

### c) 4-[5-Fluoro-2-(4-fluoro-phenoxy)-phenylsulfamoyl)-N-[(2-piperidin-4-yl-ethylcarbamoyl)-methyl]-benzamide hydrochloride

The title compound was prepared from 4-[2-(2-{4-[5-fluoro-2-(4-fluoro-phenoxy)-phenylsulfamoyl]-benzoylamino}-acetylamino)-ethyl]-piperidine-1-carboxylic acid *tert*-butyl ester according to the method described in Example 1/e. MS (EI) 573.2 (MH⁺).

### Example 14

### N-{[(1-Carbamimidoyl-piperidin-4-ylmethyl)-carbamoyl]-methyl}-4-[2-(2,4-dichloro-phenoxy)-phenylsulfamoyl]-benzamide hydrochloride

### a (tert-Butoxycarbonylimino-{4-[(2-{4-[2-(2,4-dichloro-phenoxy)-phenylsulfamoyl]-benzoylamino}-acetylamino)-methyl]-piperidin-1-yl}-methyl)-carbamic acid tert-butyl ester

To a stirred solution of 4-[2-(2,4-dichloro-phenoxy)-phenylsulfamoyl]-*N-*{[(piperidin-4-ylmethyl)-carbamoyl]-methyl}-benzamide hyrochloride (Example 1/e) (176 mg, 0.28 mmol) in dimethylformamide (3 mL) *N,N*'-bis(*tert*-butoxycarbonyl)-1*H-*pyrazole-1-carboxamidine (Aldrich) (160 mg, 0.5 mmol) and *N,N-*diisopropylethylamine (52 µL, 0.3 mmol) were added. After stirring at room temperature for 4 days the reaction mixture was concentrated and the residue was submitted to column chromatography using Kieselgel 60 (0.040-0.063 mm) (Merck) as adsorbent, and ethyl acetate:n-hexane = 5:1 as eluent to yield 150 mg (64 %) of the title compound.

### b) N-{[(1-Carbamimidoyl-piperidin-4-ylmethyl)-carbamoyl]-methyl}-4-[2-(2,4-dichloro-phenoxy)-phenylsulfamoyl]-benzamide hydrochloride

To a stirred solution of (*tert*-butoxycarbonylimino-{4-[(2-{4-[2-(2,4-dichloro-phenoxy)-phenylsulfamoyl]-benzoylamino}-acetylamino)-methyl]-piperidin-1-yl}-methyl)-carbamic acid *tert*-butyl ester (140 mg, 0.17 mmol) in ethyl acetate (0.5 mL) 2.5 M hydrogen chloride in ethyl acetate (3.0 mL) was added and the mixture was stirred at room temperature for 24 h. The precipitated product was filtered, washed with diethyl ether and dried in vacuum to yield 110 mg (98 %) of the title compound. MS (EI) 634.1 (MH⁺).

### Example 15

### 4-[5-Fluoro-2-(4-fluoro-phenoxy)-phenylsulfamoyl]-N-{[(piperidin-4-ylmethyl)-carbamoyl]-methyl}-benzamide hydrochloride

### a) 4-[2-(2-{4-[5-Fluoro-2-(4-fluoro-phenoxy)-phenylsulfamoyl]-benzoylamino}-acetylamino)-methyl]-piperidine-1-carboxylic acid tert-butyl ester

The title compound was prepared from 4-[5-fluoro-2-(4-fluoro-phenoxy)-phenylsulfamoyl]-benzoic acid and 4-[2-(2-amino-acetylamino)-methyl]-piperidine-1-carboxylic acid *tert*-butyl ester (Reference Example 1) according to the method described in Example 1/d. MS (EI) 681.3 (M+Na⁺).

### b) 4-[5-Fluoro-2-(4-fluoro-phenoxy)-phenylsulfamoyl]-N-{[(2-piperidin-4-ylmethyl)-carbamoyl]-methyl}-benzamide hydrochloride

The title compound was prepared from 4-[(2-{4-[5-fluoro-2-(4-fluoro-phenoxy)-phenylsulfamoyl]-benzoylamino}-acetylamino)-methyl]-piperidine-1-carboxylic acid *tert*-butyl ester according to the method described in Example 1/e. MS (EI) 559.4 (MH⁺).

### Example 16

### N-[(2-Oxo-2-piperazin-1-yl-ethylcarbamoyl)-methyl]-4-(2-phenoxy-phenylsulfamoyl)-benzamide hydrochloride

### a) 4-(2-Benzyloxycarbonylamino-acetyl)-piperazine-1-carboxylic acid tert-butyl ester

The title compound was prepared from Z-glycine and piperazine-1-carboxylic acid *tert*-butyl ester according to the method described in Example 1/d. MS (EI) 400.2 (M+Na⁺).

### b) 4-(2-Amino-acetyl)-piperazine-1-carboxylic acid tert-butyl ester acetate

The title compound was prepared from 4-(2-benzyloxycarbonylamino-acetyl)-piperazine-1-carboxylic acid *tert*-butyl ester according to the method described in Reference Example 1/b. MS (EI) 244.2 (MH⁺).

### c) 4-[2-(2-Benzyloxycarbonylamino-acetylamino)-acetyl]-piperazine-1-carboxylic acid tert-butyl ester

The title compound was prepared from Z-glycine and 4-(2-amino-acetyl)-piperazine-1-carboxylic acid *tert*-butyl ester according to the method described in Example 1/d. MS (EI) 457.1 (M+Na⁺).

### d) 4-[2-(2-Amino-acetylamino)-acetyl]piperazine-1-carboxylic acid tert-butyl ester acetate

The title compound was prepared from 4-[2-(2-benzyloxycarbonylamino-acetylamino)-acetyl]-piperazine-1-carboxylic acid *tert*-butyl ester according to the method described in Reference Example 1/b. MS (EI) 301.2 (MH⁺).

### e) 4-(2-{2-[4-(2-Phenoxyphenylsulfamoyl)-benzoylamino]-acetylamino}-acetyl)-piperazine-1-carboxylic acid tert-butyl ester

The title compound was prepared from 4-(2-phenoxy-phenylsulfamoyl)-benzoic acid (Example 5/a) and 4-[2-(2-amino-acetylamino)-acetyl]-piperazine-1-carboxylic acid *tert*-butyl ester acetate according to the method described in Example 1/d. MS (EI) 674.4 (M+Na⁺).

### f) N-[(2-Oxo-2-piperazin-1-yl-ethylcarbamoyl)-methyl]-4-(2-phenoxy-phenylsulfamoyl)-benzamide hydrochloride

The title compound was prepared from 4-(2-{2-[4-(2-phenoxy-phenylsulfamoyl)-benzoylamino]-acetylamino}-acetyl)-piperazine-1-carboxylic acid *tert*-butyl ester according to the method described in Example 1/e followed by preparative HPLC purification. MS (EI) 552.2 (MH⁺).

### Example 17

### 4-(2-Benzyl-phenylsulfamoyl)-N-{[(piperidin-4-ylmethyl)-carbamoyl]-methyl}-benzamide hydrochloride

### a) 4-(2-Benzyl-phenylsulfamoyl)-benzoic acid

The title compound was prepared from 2-benzyl-aniline (Aldrich) according to the method described in Example 1/c. MS (EI) 368.2 (MH⁺).

### b) 4-({2-[4-(2-Benzyl-phenylsulfamoyl)-benzoylamino]-acetylamino}-methyl)-piperidine-1-carboxylic acid tert-butyl ester

The title compound was prepared from 4-(2-benzyl-phenylsulfamoyl)-benzoic acid and 4-[(2-amino-acetylamino)-methyl]-piperidine-1-carboxylic acid *tert*-butyl ester (Reference Example 1) according to the method described in Example 1/d. MS (EI) 643.2 (M+Na⁺).

### c) 4-(2-Benzyl-phenylsulfamoyl)-N-{[(piperidin-4-ylmethyl)-carbamoyl]-methyl}-benzamide hydrochloride

The title compound was prepared from 4-({2-[4-(2-benzyl-phenylsulfamoyl)-benzoylamino]-acetylamino}-methyl)-piperidine-1-carboxylic acid *tert*-butyl ester according to the method described in Example 1/e. MS (EI) 521.5 (MH⁺).

### Example 18

### 4-(2-Benzoyl-phenylsulfamoyl)-N-{[(piperidin-4-ylmethyl)-carbamoyl]-methyl}-benzamide hydrochloride

### a) 4-(2-Benzoyl-phenylsulfamoyl)-benzoic acid

The title compound was prepared from 2-aminobenzophenone (Aldrich) according to the method described in Example 1/c. MS (EI) 382.2 (MH⁺).

### b) 4-({2-[4-(2-Benzoyl-phenylsulfamoyl)-benzoylamino]-acetylamino}-methyl)-piperidine-1-carboxylic acid tert-butyl ester

The title compound was prepared from 4-(2-benzoyl-phenylsulfamoyl)-benzoic acid and 4-[(2-amino-acetylamino)-methyl]-piperidine-l-carboxylic acid *tert*-butyl ester (Reference Example 1) according to the method described in Example 1/d. MS (EI) 657.2 (M+Na⁺).

### c) 4-(2-Benzoyl-phenylsulfamoyl)-N-{[(piperidin-4-ylmethyl)-carbamoyl]-methyl}-benzamide hydrochloride

The title compound was prepared from 4-({2-[4-(2-benzoyl-phenylsulfamoyl)-benzoylamino]-acetylamino}-methyl)-piperidine-1-carboxylic acid *tert*-butyl ester according to the method described in Example 1/e. MS (EI) 535.2 (MH⁺).

### Example 19

### N-Methyl-4-(2-phenoxv-phenylsulfamoyl)-N-{[(piperidin-4-ylmethyl)-carbamoyl]-methyl}-benzamide trifluoroacetate

### a) {Methyl-[4-(2-phenoxy-phenylsulfamoyl)-benzoyl]-amino}-acetic acid methyl ester

The title compound was prepared from 4-(2-phenoxy-phenylsulfamoyl)-benzoic acid (Example 5/a) and sarcosine methyl ester hydrochloride (Aldrich) according to the method described in Example 1/d. MS (EI) 455.2 (MH⁺).

### b) {Methyl-[4-(2-phenoxy-phenylsulfamoyl)-benzoyl]-amino}-acetic acid

The title compound was prepared from {methyl-[4-(2-phenoxy-phenylsulfamoyl)-benzoyl]-amino}-acetic acid methyl ester according to the method described in Example 2/b. MS (EI) 441.2 (MH⁺).

### c) 4-[(2-{Methyl-[4-(2-phenoxy-phenylsulfamoyl)-benzoyl]-amino}-acetylamino)-methyl]-piperidine-1-carboxylic acid tert-butyl ester

The title compound was prepared from {methyl-[4-(2-phenoxy-phenylsulfamoyl)-benzoyl]-amino}-acetic acid and 4-aminomethyl-piperidine-1-carboxylic acid *tert*-butyl ester according to the method described in Example 1/d. MS (EI) 659.2 (M+Na⁺).

### d) N-Methyl-4-(2-phenoxy-phenylsulfamoyl)-N-{[(piperidin-4-ylmethyl)-carbamoyl]-methyl}-benzamide trifluoroacetate

The title compound was prepared from 4-[(2-{methyl-[4-(2-phenoxy-phenylsulfamoyl)-benzoyl]-amino}-acetylamino)-methyl]-piperidine-1-carboxylic acid *tert*-butyl ester according to the method described in Example 3/c. MS (EI) 537.2 (MH⁺).

### Example 20

### N-{1-Methyl-1-[(piperidin-4-ylmethyl)-carbamoyl]-ethyl}-4-(2-phenoxy-phenylsulfamoyl)-benzamide hydrochloride

### a) 2-Methyl-2-[4-(2-phenoxy-phenylsulfamoyl)-benzoylamino]-propionic acid methyl ester

The title compound was prepared from 4-(2-phenoxy-phenylsulfamoyl)-benzoic acid (Example 5/a) and α-amino-isobutyric acid methyl ester hydrochloride [Collect. Czech. Chem. Commun.; 63 (1998) 85-93] according to the method described in Example 1/d. MS (EI) 469.1 (MH⁺).

### b) 2-Methyl-2-[4-(2-phenoxy-phenylsulfamoyl)-benzoylamino]-propionic acid

The title compound was prepared from 2-methyl-2-[4-(2-phenoxy-phenylsulfamoyl)-benzoylamino]-propionic acid methyl ester according to the method described in Example 2/b. MS (EI) 455.1 (MH⁺).

### c) 4-({2-Methyl-2-[4-(2-phenoxy-phenylsulfamoyl)-benzoylamino]-propionylamino}-methyl)piperidine-1-carboxylic acid tert-butyl ester

The title compound was prepared from 2-methyl-2-[4-(2-phenoxy-phenylsulfamoyl)-benzoylamino]-propionic acid and 4-aminomethyl-piperidine-1-carboxylic acid *tert*-butyl ester according to the method described in Example 1/d. MS (EI) 673.4 (M+Na⁺).

### d) N-{1-Methyl-1-[(piperidin-4-ylmethyl)-carbamoyl]-ethyl}-4-(2-phenoxy-phenylsulfamoyl)-benzamide hydrochloric

The title compound was prepared from 4-({2-methyl-2-[4-(2-phenoxy-phenylsulfamoyl)-benzoylamino]-propionylamino}-methyl)-piperidine-1-carboxylic acid *tert*-butyl ester according to the method described in Example 1/e. MS (EI) 551.2 (MH⁺).

### Example 21

### N-{(S)-2-Methyl-1-[(piperidin-4-ylmethyl)-carbamoyl]-propyl}-4-(2-phenoxy-phenylsulfamoyl)-benzamide trifluoroacetate

### a) (S)-3-Methyl-2-[4-(2-phenoxy-phenylsulfamoyl)-benzoylamino]-butyric acid tert-butyl ester

The title compound was prepared from 4-(2-phenoxy phenylsulfamoyl)-benzoic acid (Example 5/a) and L-valine *tert*-butyl ester hydrochloride (Aldrich) according to the method described in Example 1/d. MS (EI) 547.2 (M+Na⁺).

### b) (S)-3-Methyl-2-[4-(2-phenoxy-phenylsulfamoyl)-benzoylamino]-butyric acid

To a solution of (*S*)-3-methyl-2-[4-(2-phenoxy-phenylsulfamoyl)-benzoylamino]-butyric acid *tert*-butyl ester (0.56 g, 1.07 mmol) in dichloromethane (25 mL) trifluoroacetic acid (6.5 mL) was added and the reaction mixture was stirred at room temperature for 4 h, then concentrated to yield 0.467 g (93 %) of the title. MS (EI) 469.2 (MH⁺).

### c) 4-({(S)-3-Methyl-2-[4-(2-phenoxy-phenylsulfamoyl)-benzoylamino]-butyrylamino}-methyl)-piperidine-1-carboxylic acid tert-butyl ester

The title compound was prepared from (*S*)-3-methyl-2-[4-(2-phenoxy-phenylsulfamoyl)-benzoylamino]-butyric acid and 4-aminomethyl-piperidine-1-carboxylic acid *tert*-butyl ester according to the method described in Example 1/d. MS (EI) 687.2 (M+Na⁺).

### d) N-{(S)-2-Methyl-1-[(piperidin-4-ylmethyl)-carbamoyl]-propyl}-4-(2-phenoxy-phenylsulfamoyl)-benzamide trifluoroacetate

The title compound was prepared from 4-({(*S*)-3-methyl-2-[4-(2-phenoxy-phenylsulfamoyl)-benzoylamino]-butyrylamino}-methyl)-piperidine-1-carboxylic acid *tert*-butyl ester according to the method described in Example 3/c. MS (EI) 565.2 (MH⁺).

### Example 22

### 4-[2-(2,4-Dichloro-phenoxy)-phenylsulfamoyl]-N-[(2-piperidin-4-yl-ethylcarbamoyl)-methyl]-benzamide acetate

### a) 4-[2-(2-{4-[2-(2,4-Dichloro-phenoxy)-phenylsulfamoyl]-benzoylamino}-acetylamino)-ethyl]-piperidine-1-carboxylic acid tert-butyl ester

The title compound was prepared from 4-[2-(2,4-dichloro-phenoxy)-phenylsulfamoyl]-benzoic acid (Example 1/c) and 4-[2-(2-amino-acetylamino)-ethyl]-piperidine-1-carboxylic acid *tert*-butyl ester (Reference Example 2) according to the method described in Example 1/d. MS (EI) 728.2 (M+Na⁺).

### b) 4-[2-(2,4-Dichloro-phenoxy)-phenylsulfamoyl]-N-[(2-piperidin-4-yl-ethylcarbamoyl)-methyl]-benzamide acetate

The title compound was prepared from 4-[2-(2-{4-[2-(2,4-dichloro-phenoxy)-phenylsulfamoyl]-benzoylamino}-acetylamino)-ethyl]-piperidine-1-carboxylic acid *tert-*butyl ester according to the method described in Example 1/e followed by preparative HPLC purification. MS (EI) 606.2 (MH⁺).

### Example 23

### 4-[2-(2,4-Dichloro-phenoxy)-phenylsulfamoyl]-N-[(2-piperidin-1-yl-ethylcarbamoyl)-methyl]-benzamide

The title compound was prepared from {4-[2-(2,4-dichloro-phenoxy)-phenylsulfamoyl]-benzoylamino}-acetic acid (Example 2/b) and 2-piperidin-1-yl-ethylamine (EMKA-Chemie) according to the method described in Example 2/c. MS (EI) 606.2 (MH⁺).

### Example 24

### 4-[2-(2,4-Dichloro-phenoxy)-phenylsulfamoyl]-N-{[(2-dimethylamino-ethyl)-methyl-carbamoyl]-methyl}-benzamide

The title compound was prepared from {4-[2-(2,4-dichloro-phenoxy)-phenylsulfamoyl]-benzoylamino}-acetic acid (Example 2/b) and *N,N,N*'-trimethylethylenediamine (Aldrich) according to the method described in Example 2/c. MS (EI) 580.2 (MH⁺).

### Example 25

### 4-[2-(2,4-Dichloro-phenoxy)-phenylsulfamoyl]-N-[(3-dimethylamino-propylcarbamoyl)-methyl]-benzamide

The title compound was prepared from {4-[2-(2,4-dichloro-phenoxy)-phenylsulfamoyl]-benzoylamino}-acetic acid (Example 2/b) and 3-dimethylamino-1-propylamine (Aldrich) according to the method described in Example 2/c. MS (EI) 580.2 (MH⁺).

### Example 26

### 4-[2-(2,4-Dichloro-phenoxy)-phenylsulfamoyl]-N-[(3-diethylamino-propylcarbamoyl)-methyl]-benzamide

The title compound was prepared from {4-[2-(2,4-dichloro-phenoxy)-phenylsulfamoyl]-benzoylamino}-acetic acid (Example 2/b) and 3-diethylamino-1-propylamine (Fluka) according to the method described in Example 2/c. MS (EI) 608.2 (MH⁺).

### Example 27

### 4-[2-(2,4-Dichloro-phenoxy)-phenylsulfamoyl]-N-[(3-morpholin-4-yl-propylcarbamoyl)-methyl]-benzamide

The title compound was prepared from {4-[2-(2,4-dichloro-phenoxy)-phenylsulfamoyl]-benzoylamino}-acetic acid (Example 2/b) and 3-morpholin-4-yl propylamine (Aldrich) according to the method described in Example 2/c. MS (EI) 622.2 (MH⁺).

### Example 28

### 4-[2-(2,4-Dichloro-phenoxy)-phenylsulfamoyl]-N-{[3-(2-methyl-piperidin-1-yl)-propylcarbamoyl]-methyl}-benzamide

The title compound was prepared from {4-[2-(2,4-dichloro-phenoxy)-phenylsulfamoyl]-benzoylamino}-acetic acid (Example 2/b) and 3-(2-methyl-piperidin-1-yl)-propylamine (Aldrich) according to the method described in Example 2/c. MS (EI) 634.2 (MH⁺).

### Example 29

### 4-[2-(2,4-Dichloro-phenoxy)-phenylsulfamoyl]-N-[(3-imidazol-1-yl-propylcarbamoyl)-methyl]-benzamide

The title compound was prepared from {4-[2-(2,4-dichloro-phenoxy)-phenylsulfamoyl]-benzoylamino}-acetic acid (Example 2/b) and 3-imidazol-1-yl-propylamine (Aldrich) according to the method described in Example 2/c. MS (EI) 603.2 (MH⁺).

### Example 30

### 4-[2-(2,4-Dichloro-phenoxy)-phenylsulfamoyl]-N-[(2-morpholin-4-yl-ethylcarbamoyl)-methyl]-benzamide

The title compound was prepared from {4-[2-(2,4-dichloro-phenoxy)-phenylsulfamoyl]-benzoylamino}-acetic acid (Example 2/b) and 2-morpholin-4-yl-ethylamine (Aldrich) according to the method described in Example 2/c. MS (EI) 608.2 (MH⁺).

### Example 31

### 4-[2-(2,4-Dichloro-phenoxy)-phenylsulfamoyl]-N-[(2-pyrrolidin-1-yl-ethylcarbamoyl)-methyl]-benzamide

The title compound was prepared from {4-[2-(2,4-dichloro-phenoxy)-phenylsulfamoyl]-benzoylamino}-acetic acid (Example 2/b) and 2-pyrrolidin-1-yl-ethylamine (Aldrich) according to the method described in Example 2/c. MS (EI) 592.2 (MH⁺).

### Example 32

### 4-[2-(2,4-Dichloro-phenoxy)-phenylsulfamoyl]-N-[(2-diisopropylamino-ethylcarbamoyl)-methyl]-benzamide

The title compound was prepared from {4-[2-(2,4-dichloro-phenoxy)-phenylsulfamoyl]-benzoylamino}-acetic acid (Example 2/b) and 2-diisopropylamino-1-ethylamine (Fluka) according to the method described in Example 2/c. MS (EI) 622.2 (MH⁺).

### Example 33

### 4-[2-(2,4-Dichloro-phenoxy)-phenylsulfamoyl]-N-[(2-dimethylamino-1-methyl-ethylcarbamoyl)-methyl]-benzamide

The title compound was prepared from {4-[2-(2,4-dichloro-phenoxy)-phenylsulfamoyl]-benzoylamino}-acetic acid (Example 2/b) and 2-dimethylamino-1-methyl-ethylamine (Fluka) according to the method described in Example 2/c. MS (EI) 580.2 (MH⁺).

### Example 34

### 4-[2-(2,4-Dichloro-phenoxyl-phenylsulfamoyl]-N-[(2-methylamino-ethylcarbamoyl)-methyl]-benzamide hydrochloride

To a stirred solution of {4-[2-(2,4-dichloro-phenoxy)-phenylsulfamoyl]-benzoylamino}-acetic acid (Example 2/b) (42 mg, 0.085 mmol) in a mixture of dicloromethane (2 mL) and dimethylformamide (0.2 mL) and (2-amino-ethyl)-methyl-carbamic acid tert-butyl ester (Fluka) (17 mg, 0.1 mmol), HBTU (46 mg, 0.12 mmol) and triethylamine (30 µL, 0.2 mmol) were added. The mixture was stirred at room temperature for 24 h, then purified by column chromatography using Kieselgel 60 (0.015-0.040 mm) as adsorbent (Merck) and gradient elution starting with 100% A eluent and processing to 100% B eluent over a period of 20 minutes (eluent A: n-hexane; eluent B: ethyl acetate). The purified compound was dissolved in ethyl acetate (0.5 mL) 2.5 M hydrogen chloride in ethyl acetate (2.0 mL) was added and the mixture was stirred at room temperature for 24 h. The precipitated product was filtered, washed with diethyl ether and dried in vacuum to yield 31 mg (62 %) of the title compound. MS (EI) 552.2 (MH⁺).

### Example 35

### 4-[2-(2,4-Dichloro-phenoxy)-phenylsulfamoyl]-N-[(3-dimethylamino-2,2-dimethyl-propylcarbamoyl)-methyl]-benzamide

The title compound was prepared from {4-[2-(2,4-dichloro-phenoxy)-phenylsulfamoyl]-benzoylamino}-acetic acid (Example 2/b) and N,N,2,2-tetramethyl-1,3-propanediamine (Aldrich) according to the method described in Example 2/c. MS (EI) 608.2 (MH⁺).

### Example 36

### 4-[2-(3-Diethylamino-phenoxy)-phenylsulfamoyl]-N-{(piperidin-4-ylmethyl)-carbamoyl]-methyl}-benzamide hydrochloride

### a) 3-Diethylamino-1-(2-nitro-phenoxy)-benzene

The title compound was prepared from 3-diethylamino-phenol according to the method described in Example 1/a. MS (EI) 287.1 (MH⁺).

### b) 2-(3-Diethylamino-phenoxy)-phenylamine

The title compound was prepared from 3-diethylamino-1-(2-nitro-phenoxy)-benzene according to the method described in Example 1/b.

### c) 4-[2-(3-Diethylamino-phenoxy)-phenylsulfamoyl]-benzoic acid

The title compound was prepared from 2-(3-diethylamino-phenoxy)-phenylamine according to the method described in Example 1/c. MS (EI) 441.1 (MH⁺).

### d) 4-[2-(3-Diethylamino-phenoxy)-phenylsulfamoyl]-N-{[(piperidin-4-ylmethyl)-carbamoyl]-methyl}-benzamide hydrochloride

The title compound was prepared from 4-[2-(3-diethylamino-phenoxy)-phenylsulfamoyl]-benzoic acid and 4-[(2-amino-acetylamino)-methyl]-piperidine-1-carboxylic acid *tert*-butyl ester (Reference Example 1) according to the method described in Example 34. MS (EI) 594.3 (MH⁺).

### Example 37

### 4-[2-(4-Bromo-2-chloro-phenoxyl-phenylsulfamoyl]-N-{[(piperidin-4-ylmethyl)-carbamoyl]-methyl}-benzamide hydrochloride

### a) 4-Bromo-2-chloro-1-(2-nitro-phenoxy)-benzene

The title compound was prepared from 4-bromo-2-chloro-phenol according to the method described in Example 1/a. MS (EI) 329.3 (MH⁺).

### b) 2-(4-Bromo-2-chloro-phenoxy)-phenylamine

The title compound was prepared from 4-bromo-2-chloro-1-(2-nitro-phenoxy)-benzene according to the method described in Example 1/b. MS (EI) 300.2 (MH⁺).

### c) 4-[2-(4-Bromo-2-chloro-phenoxy)-phenylsulfamoyl)-benzoic acid

The title compound was prepared from 2-(4-bromo-2-chloro-phenoxy)-phenylamine according to the method described in Example 1/c.

### d) 4-[2-(4-Bromo-2-chloro-phenoxy)-phenylsulfamoyl]-N-{[(piperidin-4-ylmethyl)-carbamoyl]-methyl}-benzamide hydrochloride

The title compound was prepared from 4-[2-(4-bromo-2-chloro-phenoxy)-phenylsulfamoyl]-benzoic acid and 4-[(2-amino-acetylamino)-methyl]-piperidine-1-carboxylic acid *tert*-butyl ester (Reference Example 12 according to the method described in Example 34. MS (EI) 637.1 (MH⁺).

### Example 38

### Dimethyl-carbamic acid 4-[2-(2-{4-[2-(2,4-dichloro-phenoxy)-phenylsulfamoyl]-benzoylamino}-acetylamino)-ethyl]-phenyl ester

The title compound was prepared from {4-[2-(2,4-dichloro-phenoxy)-phenylsulfamoyl]-benzoylamino}-acetic acid (Example 2/b) and dimethyl-carbamic acid 4-(2-amino-ethyl)-phenyl ester [WO2003093245] according to the method described in Example 2/c. MS (EI) 686.2 (MH⁺).

### Example 39

### 4-[2-(2,4-Dichloro-phenoxy)-phenylsulfamoyl]-N-[(4-guanidinomethyl-benzylcarbamoyl)-methyl]-benzamide hydrochloride

The title compound was prepared from {4-[2-(2,4-dichloro-phenoxy)-phenylsulfamoyl]-benzoylamino}-acetic acid (Example 2/b) and N-(4-aminomethyl-benzyl)-guanidine dihydrochloride (Reference Example 7) according to the method described in Example 2/c. MS (EI) 656.2 (MH⁺).

### Example 40

### 4-[2-(2,4-Dichloro-phenoxy)-phenylsulfamoyl]-N-{[methyl-(2-pyridin-2-yl-ethyl)-carbamoyl]-methyl}-benzamide

The title compound was prepared from {4-[2-(2,4-dichloro-phenoxy)-phenylsulfamoyl]-benzoylamino}-acetic acid (Example 2/b) and methyl-(2-pyridin-2-yl-ethyl)-amine (Aldrich) according to the method described in Example 2/c. MS (EI) 614.3 (MH⁺).

### Example 41

### N-{[Bis-(3-dimethylamino-propyl)-carbamoyl]-methyl}-4-[2-(2,4-dichloro-phenoxy)-phenylsulfamoyl]-benzamide

The title compound was prepared from {4-[2-(2,4-dichloro-phenoxy)-phenylsulfamoyl]-benzoylamino}-acetic acid (Example 2/b) and N-(3-dimethylaminopropyl)-*N',N*'-dimethylpropane-1,3-diamine (Aldrich) according to the method described in Example 2/c. MS (EI) 665.2 (MH⁺).

### Example 42

### 2-(2-{4-[2-(2,4-Dichloro-phenoxy)-phenylsulfamoyl]-benzoylamino)-acetylamino)-(S)-3-(1H-imidazol-4-yl)-propionic acid methyl ester

The title compound was prepared from {4-[2-(2,4-dichloro-phenoxy)-phenylsulfamoyl]-benzoylamino}-acetic acid (Example 2/b) and L-histidine methyl ester dihydrochloride (Aldrich) according to the method described in Example 2/c. MS (EI) 647.2 (MH⁺).

### Example 43

### 4-[2-(2,4-Dichloro-phenoxy)-phenylsulfamoyl]-N-{[methyl-(2-pyridin-4-yl-ethyl)-carbamoyl]-methyl}-benzamide

The title compound was prepared from {4-[2-(2,4-dichloro-phenoxy)-phenylsulfamoyl]-benzoylamino}-acetic acid (Example 2/b) and methyl-(2-pyridine-4-yl-ethyl)-amine (Aldrich) according to the method described in Example 2/c. MS (EI) 614.2 (MH⁺).

### Example 44

### 3-(2-{4-[2-(2,4-Dichloro-phenoxyl-phenylsulfamoyl]-benzoylamino}-acetylamino)-(S)-5-guanidino-pentanoic acid methyl ester hydrochloride

The title compound was prepared from {4-[2-(2,4-dichloro-phenoxy)-phenylsulfamoyl]-benzoylamino}-acetic acid (Example 2/b) and L-arginine methyl ester dihydrochloride (Fluka) according to the method described in Example 2/c. MS (EI) 666.2 (MH⁺).

### Example 45

### (S)-2-Amino-6-(2-{4-[2-(2,4-dichloro-phenoxy)-phenylsulfamoyl]-benzoylamino}-acetylamino)-hexanoic acid methyl ester

The title compound was prepared from {4-[2-(2,4-dichloro-phenoxy)-phenylsulfamoyl]-benzoylamino}-acetic acid (Example 2/b) and L-lysine methyl ester dihydrochloride (Bachem) according to the method described in Example 2/c. MS (EI) 638.2 (MH⁺).

### Example 46

### (S)-2-(2-{4-[2-(2,4-Dichloro-phenoxy)-phenylsulfamoyl]-benzoylamino}-acetylamino)-succinamic acid methyl ester

The title compound was prepared from {4-[2-(2,4-dichloro-phenoxy)-phenylsulfamoyl]-benzoylamino}-acetic acid (Example 2/b) and L-asparagine methyl ester hydrochloride (Bachem) according to the method described in Example 2/c. MS (EI) 666.2 (MH⁺).

### Example 47

### 4-(2-Phenoxy-phenylsulfamoyl)-N-[(3-piperidin-1-yl-propylcarbamoyl)-methyl]-benzamide

### a) [4-(2-Phenoxy-phenylsulfamoyl)-benzoylamino]-acetic acid ethyl ester

The title compound was prepared from 4-(2-phenoxy-phenylsulfamoyl)-benzoic acid (Example 5/a) and glycine ethyl ester hydrochloride (Aldrich) according to the method described in Example 1/d. MS (EI) 455.2 (MH⁺).

### b) [4-(2-Phenoxy-phenylsulfamoyl)-benzoylamino]-acetic acid

The title compound was prepared from [4-(2-phenoxy-phenylsulfamoyl)-benzoylamino]-acetic acid ethyl ester according to the method described in Example 2/b. MS (EI) 427.2 (MH⁺).

### c) 4-(2-Phenoxy-phenylsulfamoyl)-N-[(3-piperidin-1-yl-propylcarbamoyl)-methyl]-benzamide

The title compound was prepared from [4-(2-phenoxy-phenylsulfamoyl)-benzoylamino]-acetic acid and 3-piperidin-1-yl-propylamine (EMKA-Chemie) according to the method described in Example 2/c. MS (EI) 551.2 (MH⁺).

### Example 48

### N-[(3-[1,4']Bipiperidinyl-1'-yl-propylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 3-[1,4']bipiperidinyl-1'-yl-propylamine trihydrochloride (Reference Example 3) according to the method described in Example 2/c. MS (EI) 634.2 (MH⁺).

### Example 49

### N-[(4-[1,4']Bipiperidinyl-1'-yl-phenylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 4-[1,4']bipiperidinyl-1'-yl-phenylamine [J. Med. Chem. 46 (2003) 1803-1806] according to the method described in Example 2/c. MS (EI) 668.4 (MH⁺).

### Example 50

### trans-4-(2-phenoxy-phenylsulfamoyl)-N-{[4-(2-pyrrolidin-1-yl-ethyl)-cyclohexyl-carbamoyl]-methyl}-benzamide

The title compound was prepared from [4-(2-phenoxy-phenylsulfamoyl)-benzoylamino]-acetic acid (Example 47/b) and trans-4-(2-pyrrolidin-1-yl-ethyl)-cyclohexylamine dihydrochloride (Reference Example 4) according to the method described in Example 2/c. MS (EI) 605.2 (MH⁺).

### Example 51

### 4-(2-Phenoxy-phenylsulfamoyl)-N-{[(3-(4-pyridin-4-yl-piperazin-1-yl)-propylcarbamoyl]-methyl}-benzamide

The title compound was prepared from [4-(2-phenoxy-phenylsulfamoyl)-benzoylamino]-acetic acid (Example 47/b) and 2-(4-pyridin-4-yl-piperazin-1-yl)-propylamine (Reference Example 8) according to the method described in Example 2/c. MS (EI) 629.2 (MH⁺).

### Example 52

### N-{[(2-Dimethylamino-ethyl)-methyl-carbamoyl]-methyl}-4-(2-phenoxy-phenylsulfamoyl)-benzamide

The title compound was prepared from [4-(2-phenoxy-phenylsulfamoyl)-benzoylamino]-acetic acid (Example 47/b) and *N,N,N*'-trimethylethylenediamine (Aldrich) according to the method described in Example 2/c. MS (EI) 511.2 (MH⁺).

### Example 53

### N-[(2-Methylamino-ethylcarbamoyl)-methyl]-4-(2-phenoxy-phenylsulfamoyl)-benzamide hydrochloride

The title compound was prepared from [4-(2-phenoxy-phenylsulfamoyl)-benzoylamino]-acetic acid (Example 47/b) and (2-amino-ethyl)-methyl-carbamic acid *tert*-butyl ester (Fluka) according to the method described in Example 34. MS (EI) 483.2 (MH⁺).

### Example 54

### N-[(3-Dimethylamino-propylcarbamoyl)-methyl]-4-(2-phenox-phenylsulfamoyl)-benzamide

The title compound was prepared from [4-(2-phenoxy-phenylsulfamoyl)-benzoylamino]-acetic acid (Example 47/b) and 3-dimethylamino-1-propylamine (Aldrich) according to the method described in Example 2/c. MS (EI) 511.2 (MH⁺).

### Example 55

### N-[(3-Diethylamino-propylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 3-diethylamino-1-propylamine (Fluka) according to the method described in Example 2/c. MS (EI) 539.2 (MH⁺).

### Example 56

### N-{[3-(2-Methyl-piperidin-1-yl)-propylcarbamoyl]-methyl}-4-(2-phenoxy-phenylsulfamoyl)-benzamide

The title compound was prepared from [4-(2-phenoxy-phenylsulfamoyl)-benzoylamino]-acetic acid (Example 47/b) and 3-(2-methyl-piperidin-1-yl)-propylamine (Aldrich) according to the method described in Example 2/c. MS (EI) 565.2 (MH⁺).

### Example 57

### N-[(3-Imidazol-1-yl-propylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 3-imidazol-1-yl-propylamine (Aldrich) according to the method described in Example 2/c. MS (EI) 534.2 (MH⁺).

### Example 58

### 4-(2-Phenoxy-phenylsulfamoyl)-N-[(2-pyrrolidin-1-yl-ethylcarbamoyl)-methyl]-benzamide

The title compound was prepared from [4-(2-phenoxy-phenylsulfamoyl)-benzoylamino]-acetic acid (Example 47/b) and 2-pyrrolidin-1-yl-ethylamine (Aldrich) according to the method described in Example 2/c. MS (EI) 523.2 (MH⁺).

### Example 59

### 4-[2-(2,4-Dichloro-phenoxy)-phenylsulfamoyl]-N-[(4-piperidin-4-yl-butylcarbamoyl)-methyl]-benzamide hydrochloride

The title compound was prepared from {4-[2-(2,4-dichloro-phenoxy)-phenylsulfamoyl]-benzoylamino}-acetic acid (Example 2/b) and 4-(4-amino-butyl)-piperidine-1-carboxylic acid *tert*-butyl ester (Reference Example 5) according to the method described in Example 34. MS (EI) 634.2 (MH⁺).

### Example 60

### 4-[2-(2,4-Dichloro-phenoxy)-phenylsulfamoyl]-N-(piperidin-4-ylcarbamoylmethyl)-benzamide hydrochloride

The title compound was prepared from {4-[2-(2,4-dichloro-phenoxy)-phenylsulfamoyl]-benzoylamino}-acetic acid (Example 2/b) and 4-amino-piperidine-1-carboxylic acid *tert*-butyl ester (Fluka) according to the method described in Example 34. MS (EI) 578.2 (MH⁺).

### Example 61

### 4-[2-(2,4-Dichloro-phenoxy)-phenylsulfamoyl]-N-[(3-piperidin-4-yl-propylcarbamoyl)-methyl]-benzamide hydrochloride

The title compound was prepared from {4-[2-(2,4-dichloro-phenoxy)-phenylsulfamoyl]-benzoylamino}-acetic acid (Example 2/b) and 4-(3-amino-propyl)-piperidine-1-carboxylic acid *tert*-butyl ester (Reference Example 6) according to the method described in Example 34. MS (EI) 620.2 (MH⁺)

### Example 62

### 4-[2-(2,4-Dichloro-phenoxyl-phenylsulfamoyl]-N-[(4-piperidin-1-yl-cyclohexyl-carbamoyl)-methyl]-benzamide

The title compound was prepared from {4-[2-(2,4-dichloro-phenoxy)-phenylsulfamoyl]-benzoylamino}-acetic acid (Example 2/b) and 4-piperidin-1-yl-cyclohexylamine [J. Am. Chem. Soc. 68 (1946) 1296] according to the method described in Example 2/c. MS (EI) 660.2 (MH⁺).

### Example 63

### 4-[2-(2,4-Dichloro-phenoxy)-phenylsulfamoyl]-N-[(4-piperidin-1-yl-butylcarbamoyl)-methyl]-benzamide

The title compound was prepared from {4-[2-(2,4-dichloro-phenoxy)-phenylsulfamoyl]-benzoylamino}-acetic acid (Example 2/b) and 4-piperidin-1-yl-butylamine [J. Med. Chem. 45 (2002) 1128-1141] according to the method described in Example 2/c. MS (EI) 634.2 (MH⁺).

### Example 64

### N-{[(Piperidin-4-ylmethyl)-carbamoyl]-methyl}-4-[2-(4-trifluoromethyl-phenoxy)-phenylsulfamoyl]-benzamide hydrochloride

### a) 4-[2-(4-Trifluormethyl-phenoxy)-phenylsulfamoyl]-benzoic acid

The title compound was prepared from 2-(4-trifluoromethyl-phenoxy)-phenylamine [J. Chem. Soc. Perkin Trans. 1. (1976) 1279-1285] according to the method described in Example 3/a. MS (EI) 438.0 (MH⁺).

### b) N-{[Piperidin-4-ylmethyl)-carbamoyl-methyl}-4-[2-(4-trifluoromethyl-phenoxy)-phenylsulfamoyl]-benzamide hydrochloride

The title compound was prepared from 4-[2-(4-trifluormethyl-phenoxy)-phenylsulfamoyl]-benzoic acid and 4-[(2-amino-acetylamino)-methyl]-piperidine-1-carboxylic acid *tert*-butyl ester (Reference Example 1) according to the method described in Example 34. MS (EI) 591.2 (MH⁺).

### Example 65

### N-{[(Piperidin-4-ylmethyl)-carbamoyl]-methy}-4-[2-(4-trifluoromethoxy-phenoxy)-phenylsulfamoyl]-benzamide hydrochloride

### a) 4-[2-(4-Trifluormethoxy-phenoxy)-phenylsulfamoyl]-benzoic acid

The title compound was prepared from 2-(4-trifluoromethoxy-phenoxy)-phenylamine [J. Med. Chem. 13 (1970) 295-297] according to method described in Example 3/a. MS (EI) 454.1 (MH⁺).

### b) N-{[(Piperidin-4-ylmethyl)-carbamoyl]-methyl}-(4-[2-trifluoromethoxy-phenoxy)-phenylsulfamoyl]-benzamide hydrochloride

The title compound was prepared from 4-[2-(4-trifluormethoxy-phenoxy)-phenylsulfamoyl]-benzoic acid and 4-[(2-amino-acetylamino)-methyl]-piperidine-1-carboxylic acid *tert*-butyl ester (Reference Example 1) according to the method described in Example 34. MS (EI) 607.2 (MH⁺).

### Example 66

### 4-(2-Phenoxy-phenylsulfamoyl)-N-(piperidin-4-ylcarbamoylmethyl)-benzamide hydrochloride

The title compound was prepared from [4-(2-phenoxy-phenylsulfamoyl)-benzoylamino]-acetic acid (Example 47/b) and 4-amino-piperidine-1-carboxylic acid ***tert***-butyl ester (Aldrich) according to the method described in Example 34. MS (EI) 509.2 (MH⁺).

### Example 67

### (S)-6-Amino-2-{2-[4-(2-phenoxy-phenylsulfamoyl)-benzoylamino]-acetylamino}-hexanoic acid methyl ester hydrochloride

The title compound was prepared from [4-(2-phenoxy-phenylsulfamoyl)-benzoylamino]-acetic acid (Example 47/b) and *N*-(tert-butoxycarbonyl)-L-lysine methyl ester hydrochloride (Fluka) according to the method described in Example 34. MS (EI) 569.2 (MH⁺).

### Example 68

### N-Carbamoylmethyl-4-(2-phenoxy-phenylsulfamoyl)-benzamide

The title compound was prepared from [4-(2-phenoxy-phenylsulfamoyl)-benzoylamino]-acetic acid (Example 47/b) and ammonium carbonate according to the method described in Example 2/c. MS (EI) 426.2 (MH⁺).

### Example 69

### N-[(2-Hydroxy-ethylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and ethanolamine hydrochloride (Aldrich) according to the method described in Example 2/c. MS (EI) 470.2 (MH⁺).

### Example 70

### N-[(3-Hydroxy-propylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 3-amino-1-propanol hydrochloride **(Aldrich) according to the method described in Example 2/c. MS (EI) 484.2 (MH⁺).**

### Example 71

### N-[(4-Hydroxy-butylcarbamoyl)-methyl}-4-(2-phenoxy-phenylsulfamoyl)-benzamide

The title compound was prepared from [4-(2-phenoxy-phenylsulfamoyl)-benzoylamino]-acetic acid (Example 47/b) and 4-amino-1-butanol hydrochloride (Aldrich) according to the method described in Example 2/c. MS (EI) 498.2 (MH⁺).

### Example 72

### N-Cycloheptylcarbamoylmethyl-4-(2-phenoxy-phenylsulfamoyl)-benzamide

The title compound was prepared from [4-(2-phenoxy-phenylsulfamoyl)-benzoylamino]-acetic acid (Example 47/b) and cycloheptylamine (Aldrich) according to the method described in Example 2/c. MS (EI) 522.2 (MH⁺).

### Example 73

### N-[(4-Cyanomethyl-phenylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and (4-amino-phenyl)-acetonitrile (Aldrich) according to the method described in Example 2/c. MS (EI) 541.2 (MH⁺).

### Example 74

### N-Cyclohexylcarbamoylmethyl-4-(2-phenoxy-phenylsulfamoyl)-benzamide

The title compound was prepared from [4-(2-phenoxy-phenylsulfamoyl)-benzoylamino]-acetic acid (Example 47/b) and cyclohexylamine (Aldrich) according to the method described in Example 2/c. MS (EI) 508.2 (MH⁺).

### Example 75

### N-Cyclopentylcarbamoylmethyl-4-(2-phenoxy-phenylsulfamoyl)-benzamide

The title compound was prepared from [4-(2-phenoxy-phenylsulfamoyl)-benzoylamino]-acetic acid (Example 47/b) and cyclopentylamine (Aldrich) according to the method described in Example 2/c. MS (EI) 494.2 (MH⁺).

### Example 76

### N-[(2-Acetylamino-phenylcarbamoyl)-methyl]-4-(2-phenoxyphenylsulfamoyl)-benzamide

The title compound was prepared from [4-(2-phenoxy-phenylsulfamoyl)-benzoylamino]-acetic acid (Example 47/b) and N-(2-amino-phenyl)-acetamide (Aldrich) according to the method described in Example 2/c. MS (EI) 559.2 (MH⁺).

### Example 77

### 4-(2-Benzoyl-phenylsulfamoyl)-N-cyclohexylcarbamoylmethyl-benzamide

### a) 4-(2-Benzoyl-phenylsulfamoyl)-benzoic acid

The title compound was prepared from 2-amino-benzophenone according to the method described in Example 1/c. MS (EI) 382.2 (MH⁺).

### b) [4-(2-Benzoyl-phenylsulfamoyl)-benzoylamino]-acetic acid ethyl ester

The title compound was prepared from 4-(2-benzoyl-phenylsulfamoyl)-benzoic acid and glycine ethyl ester hydrochloride (Aldrich) according to the method described in Example 1/d. MS (EI) 867.2 (MH⁺).

### c) [4-(2-Benzoyl-phenylsulfamoyl)-benzoylamino]-acetic acid

The title compound was prepared from [4-(2-benzoyl-phenylsulfamoyl)-benzoylamino]-acetic acid ethyl ester according to the method described in Example 1/e. MS (EI) 439.2 (MH⁺).

### d) 4-(2-Benzoyl-phenylsulfamoyl)-N-cyclohexylcarbamoylmethyl-benzamide

The title compound was prepared from [4-(2-benzoyl-phenylsulfamoyl)-benzoylamino]-acetic acid and cyclohexylamine (Aldrich) according to the method described in Example 2/c. MS (EI) 520.2 (MH⁺).

### Example 78

### 4-(2-Benzoyl-phenylsulfamoyl)-N-[(4-[1,4']bipiperidinyl-1'-yl-phenylcarbamoyl)-methyl]-benzamide

The title compound was prepared from [4-(2-benzoyl-phenylsulfamoyl)-benzoylamino]-acetic acid (Example 77/c) and 4-[1,4']bipiperidinyl-1'-yl-phenylamine [J. Med. Chem. 46 (2003) 1803-1806] according to the method described in Example 2/c. MS (EI) 680.2 (MH⁺).

### Example 79

### trans-4-(2-Benzoyl-phenylsulfamoyl)-N-{[4-(2-pyrrolidin-1-yl-ethyl)-cyclohexylcarbamoyl]-methyl}-benzamide

The title compound was prepared from [4-(2-benzoyl-phenylsulfamoyl)-benzoylamino]-acetic acid (Example 77/c) and trans-4-(2-pyrrolidin-1-yl-ethyl)-cyclohexylamine dihydrochloride (Reference Example 4) according to the method described in Example 2/c. MS (EI) 617.2 (MH⁺).

### Example 80

### 4-(2-Benzoyl-phenylsulfamoyl)-N-(piperidin-4-ylcarbamoylmethyl)-benzamide hydrochloride

The title compound was prepared from [4-(2-benzoyl-phenylsulfamoyl)-benzoylamino]-acetic acid (Example 77/c) and 4-amino-piperidine-1-carboxylic acid *tert*-butyl ester (Fluka) according to the method described in Example 34. MS (EI) 521.2 (MH⁺).

### Example 81

### 4-(2-Benzoyl-phenylsulfamoyl)-N-[(4-piperidin-1-yl-cyclohexylcarbamoyl)-methyl]-benzamide

The title compound was prepared from [4-(2-benzoyl-phenylsulfamoyl)-benzoylamino]-acetic acid (Example 77/c) and 4-piperidin-1-yl-cyclohexylamine [J. Am. Chem. Soc. 68 (1946) 1296] according to the method described in Example 2/c. MS (EI) 603.2 (MH⁺).

### Example 82

### N-{1-[(1-Ethyl-piperidin-4-ylmethyl)-carbamoyl]-1-methyl-ethyl}-4-(2-phenoxy

### phenylsulfamoyl)-benzamide

The title compound was prepared from 2-methyl-2-[4-(2-phenoxy-phenylsulfamoyl)-benzoylamino]-propionic acid (Example 20/b) and *C*-(1-ethyl-piperidin-4-yl)-methylamine [Eur. J. Med. Chem. Chim. Ther. 34 (1999) 329-342] according to the method described in Example 1/d. MS (EI) 579.2 (MH⁺).

### Example 83

### 4-(2-Phenoxy-phenylsulfamoyl)-N-[(4-piperidin-4-yl-butylcarbamoyl)-methyl]-benzamide hydrochloride

The title compound was prepared from [4-(2-phenoxy-phenylsulfamoyl)-benzoylamino]-acetic acid (Example 47/b) and 4-(4-amino-butyl)-piperidine-1-carboxylic acid *tert*-butyl ester (Reference Example 5) according to the method described in Example 34. MS (EI) 565.2 (MH⁺).

### Example 84

### N-{[(S)-2-(4-Hydroxy-phenyl)-1-[(piperidin-4-ylmethyl)-carbamoyl]-ethyl}-4-(2-phenoxy-phenylsulfamoyl)-benzamide trifluoroacetate

### a) (S)-3-(4-Hydroxy-phenyl)-2-[4-(2-phenoxy-phenylsulfamoyl)-benzoylamino]-propionic acid methyl ester

The title compound was prepared from 4-(2-phenoxy-phenylsulfamoyl)-benzoic acid (Example 5/a) and L-tyrosine methyl ester (Aldrich) according to the method described in Example 1/d. MS (EI) 547.2 (MH⁺).

### b) (S)-3-(4-Hydroxy-phenyl)-2-[4-(2-phenoxy-phenylsulfamoyl)-benzoylamino]-propionic acid

The title compound was prepared from (S)-3-(4-hydroxy-phenyl)-2-[4-(2-phenoxy-phenylsulfamoyl)-benzoylamino]-propionic acid methyl ester according to the method described in Example 2/b. MS (EI) 533.2 (MH⁺).

### c) 4-({(S)-3-(4-Hydroxy-phenyl)-2-[4-(2-phenoxy-phenylsulfamoyl)-benzoylamino]-_ propionylamino}-methyl)-piperidine-1-carboxylic acid tert-butyl ester

The title compound was prepared from (*S*)-3-(4-hydroxy-phenyl)-2-[4-(2-phenoxy-phenylsulfamoyl)-benzoylamino]-propionic acid and 4-aminomethyl-piperidine-1-carboxylic acid *tert*-butyl ester according to the method described in Example 1/d. MS (EI) 751.2 (M+Na⁺).

### d) N-{(S)-2-(4-Hydroxy-phenyl)-1-[(piperidin-4-ylmethyl)-carbamoyl]-ethyl}-4-(2-phenoxy-phenylsulfamoyl)-benzamide trifluoroacetate

The title compound was prepared from 4-({(*S*)-3-(4-hydroxy-phenyl)-2-[4-(2-phenoxy-phenylsulfamoyl)-benzoylamino]-propionylamino}-methyl)-piperidine-1-carboxylic acid *tert*-butyl ester according to the method described in Example 3/c. MS (EI) 629.2 (MH⁺).

### Example 85

### N-{(S)-5-Amino-1-[(piperidin-4-ylmethyl)-carbamoyl]-pentyl}-4-(2-phenoxy-phenylsulfamoyl)-benzamide ditrifluoroacetate

### a) (S)-6-tert-Butoxy-carbonylamino-2-[4-(2-phenoxy-phenylsulfamoyl)-benzoylamino]-hexanoic acid methyl ester

The title compound was prepared from 4-(2-phenoxy-phenylsulfamoyl)-benzoic acid (Example 5/a) and *N*-(*tert*-butoxycarbonyl)-L-lysine methyl ester [Bull. Chem. Soc. Jpn. 37 (1964) 1471-1477] according to the method described in Example 1/d. MS (EI) 634.4 (M+Na⁺).

### b) (S)-6-tert-Butoxycarbonylamino-2-[4-(2-phenoxy-phenylsulfamoyl)-benzoylamino]-hexanoic acid

The title compound was prepared from (S)-6-tert-butoxycarbonylamino-2-[4-(2-phenoxy-phenylsulfamoyl)-benzoylamino]-hexanoic acid methyl ester according to the method described in Example 2/b. MS (EI) 598.2 (MH⁺).

### c) 4-({(S)-6-tert-Butoxycarbonylamino-2-[4-(2-phenoxy-phenylsulfamoyl)-benzoylamino]-hexanoylamino}-methyl)-piperidine-1-carboxylic acid tert-butyl ester

The title compound was prepared from (S)-6-tert-butoxycarbonylamino-2-[4-(2-phenoxy-phenylsulfamoyl)-benzoylamino]-hexanoic acid and 4-aminomethyl-piperidine-1-carboxylic acid tert-butyl ester according to the method described in Example 1/d. MS (EI) 816.2 (M+Na⁺).

### d) N-{(S)-5-Amino-1-[(piperidin-4-ylmethyl)-carbamoyl]-pentyl}-4-(2-phenoxy-phenylsulfamoyl)-benzamide ditrifluoroacetate

The title compound was prepared from 4-({(*S*)-6-tert-butoxycarbonylamino-2-[4-(2-phenoxy-phenylsulfamoyl)-benzoylamino]-hexanoylamino}-methyl)-piperidine-1-carboxylic acid *tert*-butyl ester according to the method described in Example 3/c. MS (EI) 594.2 (MH⁺).

### Example 86

### 4-(2-Phenoxy-phenylsulfamoyl)-N-{1-[(piperidin-4-ylmethyl)-carbamoyl]-cyclopropyl}-benzamide trifluoroacetate

### a) 1-[4-(2-Phenoxy-phenylsulfamoyl)-benzoylamino]-cyclopropanecarboxylic acid methyl ester

The title compound was prepared from 4-(2-phenoxy-phenylsulfamoyl)-benzoic acid (Example 5/a) and methyl 1-amino-cyclopropane-carboxylate hydrochloride (Sigma) according to the method described in Example 1/d. MS (EI) 467.1 (MH⁺).

### b) 1-[4-(2-Phenoxy-phenylsulfamoyl)-benzoylamino]-cyclopropanecarboxylic acid

The title compound was prepared from 1-[4-(2-phenoxy-phenylsulfamoyl)-benzoylamino]-cyclopropanecarboxylic acid methyl ester according to the method described in Example 2/b. MS (EI) 453.1 (MH⁺).

### c) 4-[({1-[4-(2-Phenoxy-phenylsulfamoyl)-benzoylamino]-cyclopropanecarbonyl}-amino)-methyl]-piperidine-1-carboxylic acid tert-butyl ester

The title compound was prepared from 1-[4-(2-phenoxy-phenylsulfamoyl)-benzoylamino]-cyclopropanecarboxylic acid and 4-aminomethyl-piperidine-1-carboxylic acid *tert*-butyl ester according to the method described in Example 1/d. MS (EI) 671.2 (M+Na⁺).

### d) 4-(2-Phenoxy-phenylsulfamoyl)-N-{1-[(piperidin-4-ylmethyl)-carbamoyl]-cyclopropyl}-benzamide trifluoroacetate

The title compound was prepared from 4-[({1-[4-(2-phenoxy-phenylsulfamoyl)-benzoylamino]-cyclopropanecarbonyl}-amino)-methyl]-piperidine-1-carboxylic acid *tert*-butyl ester according to the method described in Example 3/c. MS (EI) 549.2 (MH⁺).

### Example 87

### 4-(2-Phenoxy-phenylsulfamoyl)-N-phenylcarbamoylmethyl-benzamide

The title compound was prepared from [4-(2-phenoxy-phenylsulfamoyl)-benzoylamino]-acetic acid (Example 47/b) and aniline according to the method described in Example 2/c. MS (EI) 502.1 (MH⁺).

### Example 88

### 4-(2-Phenoxy-phenylsulfamoyl)-N-[(5-trifluoromethyl-[1,3,4]thiadiazol-2-ylcarbamoyl)-methyl]-benzamide

The title compound was prepared from [4-(2-phenoxy-phenylsulfamoyl)-benzoylamino]-acetic acid (Example 47/b) and 5-trifluoromethyl-[1,3,4]thiadiazol-2-ylamine (Aldrich) according to the method described in Example 2/c. MS (EI) 578.2 (MH⁺).

### Example 89

### 4-(2-Phenoxy-phenylsulfamoyl)-N-([1,3,4]thiadiazol-2-ylcarbamoylmethyl)-benzamide

The title compound was prepared from [4-(2-phenoxy-phenylsulfamoyl)-benzoylamino]-acetic acid (Example 47/b) and [1,3,4]thiadiazol-2-ylamine (Aldrich) according to the method described in Example 2/c. MS (EI) 510.2 (MH⁺).

### Example 90

### N-[(1H-Indol-6-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 1*H*-indol-6-ylamine (Aldrich) according to the method described in Example 2/c. MS (EI) 541.2 (MH⁺).

### Example 91

### N-[(1H-Benzoimidazol-2-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 1*H*-benzimidazol-2-ylamine (Aldrich) according to the method described in Example 2/c. MS (EI) 542.2 (MH⁺).

### Example 92

### N-[(2,2-Difluoro-benzo[1,3]dioxol-4-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 2,2-difluoro-benzo[1,3]dioxol-4-ylamine (ABCR) according to the method described in Example 2/c. MS (EI) 582.2 (MH⁺).

### Example 93

### N-[(4-Methyl-thiazol-2-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 4-methyl-thiazol-2-ylamine (Aldrich) according to the method described in Example 2/c. MS (EI) 523.2 (MH⁺).

### Example 94

### N-(Benzothiazol-2-ylcarbamoylmethyl)-4-(2-phenoxy-phenylsulfamoyl)-benzamide

The title compound was prepared from [4-(2-phenoxy-phenylsulfamoyl)-benzoylamino]-acetic acid (Example 47/b) and benzothiazol-2-ylamine (Aldrich) according to the method described in Example 2/c. MS (EI) 559.2 (MH⁺).

### Example 95

### N-[(4-Cyano-1H-pyrazol-3-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 3-amino-1*H*-pyrazole-4-carbonitrile (Aldrich) according to the method described in Example 2/c. MS (EI) 517.2 (MH⁺).

### Example 96

### N-[(5-Methyl-thiazol-2-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 5-methyl-thiazol-2-ylamine (Aldrich) according to the method described in Example 2/c. MS (EI) 523.2 (MH⁺).

### Example 97

### N-[(5-Methyl-2H-pyrazol-3-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 5-methyl-2*H*-pyrazol-3-ylamine (Aldrich) according to the method described in Example 2/c. MS (EI) 506.1 (MH⁺).

### Example 98

### N-[(6-Acetylamino-pyridin-3-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and *N*-(5-amino-pyridin-2-yl)-acetamide (Aldrich) according to the method described in Example 2/c. MS (EI) 560.1 (MH⁺).

### Example 99

### N-[(1H-Indol-4-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 1*H*-indol-4-ylamine (Aldrich) according to the method described in Example 2/c. MS (EI) 541.1 (MH⁺).

### Example 100

### N-(Benzothiazol-6-ylcarbamoylmethyl)-4-(2-phenoxy-phenylsulfamoyl)-benzamide

The title compound was prepared from [4-(2-phenoxy-phenylsulfamoyl)-benzoylamino]-acetic acid (Example 47/b) and benzothiazol-6-ylamine (Aldrich) according to the method described in Example 2/c. MS (EI) 559.2 (MH⁺).

### Example 101

### N-[(1H-Indol-5-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 1*H*-indol-5-ylamine (Aldrich) according to the method described in Example 2/c. MS (EI) 541.2 (MH⁺).

### Example 102

### N-[(3,5-Dimethyl-isoxazol-4-ylcarbamoyl)-methyl]-4-(2-phenoxy-phenylsulfamoyl)-benzamide

The title compound was prepared from [4-(2-phenoxy-phenylsulfamoyl)-benzoylamino]-acetic acid (Example 47/b) and 3,5-dimethyl-isoxazol-4-ylamine (Aldrich) according to the method described in Example 2/c. MS (EI) 521.2 (MH⁺).

### Example 103

### 4-(2-Phenylamino-phenylsulfamoyl)-N-{[(piperidin-4-ylmethyl)-carbamoyl]-methyl}-benzamide hydrochloride

### a) 4-(2-Phenylamino-phenylsulfamoyl)-benzoic acid

The title compound was prepared from 2-amino-diphenylamine (Aldrich) according to the method described in Example 1/c. MS (EI) 369.2 (MH⁺).

### b) 4-({2-[4-(2-Phenylamino-phenylsulfamoyl)-benzoylamino]-acetylamino}-methyl)-piperidine-1-carboxylic acid tert-butyl ester

The title compound was prepared from 4-(2-phenylamino-phenylsulfamoyl)-benzoic acid and 4-[(2-amino-acetylamino)-methyl]-piperidine-1-carboxylic acid *tert-*butyl ester (Reference Example 1) according to the method described in Example 1/d. MS (EI) 622.2 (MH⁺).

### c) 4-(2-Phenylamino-phenylsulfamoyl)-N-{[(piperidin-4-ylmethyl)-carbamoyl]-methyl}-benzamide hydrochloride

The title compound was prepared from 4-({2-[4-(2-phenylamino-phenylsulfamoyl)-benzoylamino]-acetylamino}-methyl)-piperidine-1-carboxylic acid *tert*-butyl ester according to the method described in Example 1/e. MS (EI) 522.2 (MH⁺).

### Example 104

### N-{(S)-2-Hydroxy-1-[(piperidin-4-ylmethyl)-carbamoyl]-ethyl}-4-(2-phenoxy-phenylsulfamoyl)-benzamide trifluoroacetate

### a) (S)-3-Hydroxy-2-[4-(2-phenoxy-phenylsulfamoyl)-benzoylamino]-propionic acid methyl ester

The title compound was prepared from 4-(2-phenoxy-phenylsulfamoyl)-benzoic acid (Example 5/a) and L-serine methyl ester hydrochloride (Aldrich) according to the method described Example 1/d.

### b) (S)-3-Hydroxy-2-[4-(2-phenoxy-phenylsulfamoyl)-benzoylamino]-propionic acid

The title compound was prepared from (*S*)-3-hydroxy-2-[4-(2-phenoxy-phenylsulfamoyl)-benzoylamino]-propionic acid methyl ester according to the method described Example 2/b. MS (EI) 457.3 (MH⁺).

### c) 4-({(S)-3-Hydroxy-2-[4-(2-phenoxy-phenylsulfamoyl)-benzoylamino]-propionylamino}-methyl)-piperidine-1-carboxylic acid tert-butyl ester

The title compound was prepared from (*S*)-3-hydroxy-2-[4-(2-phenoxy-phenylsulfamoyl)-benzoylamino]-propionic acid and 4-aminomethyl-piperidine-1-carboxylic acid *tert*-butyl ester according to the method described in Example 1/d. MS (EI) 675.2 (M+Na⁺).

### d) N-{(S)-2-Hydroxy-1-[(piperidin-4-ylmethyl)-carbamoyl]-ethyl}-4-(2-phenoxy-phenylsulfamoyl)-benzamide trifluoroacetate

The title compound was prepared from 4-({(*S*)-3-hydroxy-2-[4-(2-phenoxy-phenylsulfamoyl)-benzoylamino]-propionylamino}-methyl)-piperidine-1-carboxylic acid tert-butyl ester according to the method described in Example 3/c. MS (EI) 553.2 (MH⁺).

### Example 105

### (S)-2-[4-(2-Phenoxy-phenylsulfamoyl)-benzoylamino]-pentanedioic acid 5-amide 1-[(piperidin-4-ylmethyl)-amide] hydrochloride

### a) (S)-4-Carbamoyl-2-[4-(2-phenoxy-phenylsulfamoyl)-benzoylamino]-butyric acid methyl ester

The title compound was prepared from 4-(2-phenoxy-phenylsulfamoyl)-benzoic acid (Example 5/a) and L-glutamine methyl ester hydrochloride (Senn Chemicals) according to the method described Example 1/d.

### b) (S)-4-Carbamoyl-2-[4-(2-phenoxy-phenylsulfamoyl)-benzoylamino]-butyric acid

The title compound was prepared from (*S*)-4-carbamoyl-2-[4-(2-phenoxy-phenylsulfamoyl)-benzoylamino]-butyric acid methyl ester according to the method described Example 2/b. MS (EI) 498.3 (MH⁺).

### c) 4-({(S)-4-Carbamoyl-2-[4-(2-phenoxy-phenylsulfamoyl)-benzoylamino]-butyrylamino}-methyl)-piperidine-1-carboxylic acid tert-butyl ester

The title compound was prepared from (*S*)-4-carbamoyl-2-[4-(2-phenoxy-phenylsulfamoyl)-benzoylamino]-butyric acid and 4-aminomethyl-piperidine-1-carboxylic acid *tert*-butyl ester according to the method described in Example 1/d. MS (EI) 716.2 (M+Na⁺).

### d) (S)-2-[4-(2-Phenoxy-phenylsulfamoyl)-benzoylamino]-pentanedioic acid 5-amide 1-[(piperidin-4-ylmethyl)-amide hydrochloride

The title compound was prepared from 4-({(*S*)-4-carbamoyl-2-[4-(2-phenoxy-phenylsulfamoyl)-benzoylamino]-butyrylamino}-methyl)-piperidine-1-carboxylic acid tert-butyl ester according to the method described in Example 1/e. MS (EI) 594.5 (MH⁺).

### Example 106

### N-Cyclooctylcarbamoylmethyl-4-(2-phenoxy-phenylsulfamoyl)-benzamide

The title compound was prepared from [4-(2-phenoxy-phenylsulfamoyl)-benzoylamino]-acetic acid (Example 47/b) and cyclooctylamine (Aldrich) according to the method described in Example 2/c. MS (El) 536.2 (MH⁺).

### Example 107

### 4-(2-Benzoyl-phenylsulfamoyl)-N-[(2-piperidin-4-yl-ethylcarbamoyl)-methyl]-benzamide hydrochloride

The title compound was prepared from [4-(2-benzoyl-phenylsulfamoyl)-benzoylamino]-acetic acid (Example 77/c) and 4-(2-amino-ethyl)-piperidine-1-carboxylic acid tert-butyl ester [Bioorg. Med. Chem. Lett.; 13 (2003) 2167-2172.] according to the method described in Example 34. MS (EI) 549.2 (MH⁺).

### Example 108

### 4-[2-(2,4-Dichloro-benzoyl)-phenylsulfamoyl]-N-[(2-piperidin-4-yl-ethylcarbamoyl)-methyl]-benzamide hydrochloride

### a) 4-[2-(2,4-Dichloro-benzoyl)-phenylsulfamoyl]-benzoic acid

The title compound was prepared from (2-amino-phenyl)-(2,4-dichloro-phenyl)-methanone [Synthesis, (1980) 677-688] and 4-chlorosulfonyl-benzoic acid according to the method described in Example 1/c. MS (EI) 451 (MH⁺).

### b) {4-[2-(2,4-Dichloro-benzoyl)-phenylsulfamoyl]-benzoylamino}-acetic acid ethyl ester

The title compound was prepared from 4-[2-(2,4-dichloro-benzoyl)-phenylsulfamoyl]-benzoic acid and glycine ethyl ester hydrochloride according to the method described in Example 1/d. MS (EI) 536.1 (MH⁺).

### c) {4-[2-(2,4-Dichloro-benzoyl)-phenylsulfamoyl-benzoylamino}-acetic acid

The title compound was prepared from {4-[2-(2,4-dichloro-benzoyl)-phenylsulfamoyl]-benzoylamino}-acetic acid ethyl ester according to the method described in Example 2/b. MS (EI) 508 (MH⁺).

### d) 4-[2-(2,4-Dichloro-benzoyl)-phenylsulfamoyl]-N-[(2-piperidin-4-yl-ethylcarbamoyl)-methyl]-benzamide hydrochloride

The title compound was prepared from {4-[2-(2,4-dichloro-benzoyl)-phenylsulfamoyl]-benzoylamino}-acetic acid and 4-(2-amino-ethyl)-piperidine-1-carboxylic acid tert-butyl ester [Bioorg. Med. Chem. Lett.; 13 (2003) 2167-2172.] according to the method described in Example 34. MS (EI) 618.4 (MH⁺).

### Example 109

### trans-4-[2-(2,4-Dichloro-benzoyl)-phenylsulfamoyl]-N-{[4-(2-pyrrolidin-1-yl-ethyl)-cyclohexylcarbamoyl]-methyl}-benzamide

The title compound was prepared from {4-[2-(2,4-dichloro-benzoyl)-phenylsulfamoyl]-benzoylamino}-acetic acid (Example 108/c) and trans-4-(2-pyrrolidin-1-yl-ethyl)-cyclohexylamine dihydrochloride (Reference Example 4) according to the method described in Example 2/c. MS (EI) 686.2 (MH⁺).

### Example 110

### 4-[2-(2,4-Dichloro-benzoyl)-phenylsulfamoyl]-N-(piperidin-4-ylcarbamoylmethyl)-benzamide hydrochloride

The title compound was prepared from {4-[2-(2,4-dichloro-benzoyl)-phenylsulfamoyl]-benzoylamino}-acetic acid (Example 108/c) and 4-amino-piperidine-1-carboxylic acid *tert*-butyl ester (Fluka) according to the method described in Example 34. MS (EI) 589.3 (MH⁺).

### Example 111

### trans-4-[2-(2-Chloro-4-fluoro-phenoxy)-phenylsulfamoyl]-N-{[4-(2-pyrrolidin-1-yl-ethyl)-cyclohexylcarbamoyl]-methyl}-benzamide

### a) 2-Chloro-4-fluoro-1-(2-nitro-phenoxy)-benzene

The title compound was prepared from 2-chloro-4-fluoro-phenol according to the method described in Example 1/a.

### b) 2-(2-Chloro-4-fluoro-phenoxy)-phenylamine

The title compound was prepared from 2-chloro-4-fluoro-1-(2-nitro-phenoxy)-benzene according to the method described in Example 1/b.

### c) 4-[2-(2-Chloro-4-fluoro-phenoxy)-phenylsulfamoyl]-benzoic acid

The title compound was prepared from 2-(2-chloro-4-fluoro-phenoxy)-phenylamine according to the method described in Example 1/c. MS (EI) 422.1 (MH⁺).

### d) {4-[2-(2-Chloro-4-fluoro-phenoxy)-phenylsulfamoyl]-benzoylamino}-acetic acid

### ethyl ester

The title compound was prepared from 4-[2-(2-chloro-4-fluoro-phenoxy)-phenylsulfamoyl]-benzoic acid and glycine ethyl ester hydrochloride according to the method described in Example 1/d.

### e) {4-[2-(2-Chloro-4-fluoro-phenoxy)-phenylsulfamoyl]-benzoylamino}-acetic acid

The title compound was prepared from {4-[2-(2-chloro-4-fluoro-phenoxy)-phenylsulfamoyl]-benzoylamino}-acetic acid ethyl ester according to the method described in Example 2/b. MS (EI) 479 (MH⁺).

### f) trans-4-[2-(2-Chloro-4-fluoro-phenoxy)-phenylsulfamoyl]-N-{[4-(2-pyrrolidin-1-yl-ethyl)-cyclohexylcarbamoyl]-methyl}-benzamide

The title compound was prepared from {4-[2-(2-chloro-4-fluoro-phenoxy)-phenylsulfamoyl]-benzoylamino}-acetic acid and trans-4-(2-pyrrolidin-1-yl-ethyl)-cyclohexylamine dihydrochloride (Reference Example 4) according to the method described in Example 2/c. MS (EI) 657.4 (MH⁺).

### Example 112

### trans-4-[2-(4-Bromo-2-chloro-phenoxy)-phenylsulfamoyl-N-{[4-(2-pyrrolidin-1-yl-ethyl)-cyclohexylcarbamoyl]-methyl]-benzamide

### a) 4-Bromo-2-chloro-1-(2-nitro-phenoxy)-benzene

The title compound was prepared from 4-bromo-2-chloro-phenol according to the method described in Example 1/a. MS (EI) 329.3 (MH⁺).

### b) 2-(4-Bromo-2-chloro-phenoxy)-phenylamine

The title compound was prepared from 4-bromo-2-chloro-1-(2-nitro-phenoxy)-benzene according to the method described in Example 1/b. MS (EI) 300.2 (MH⁺).

### c) 4-[2-(4-Bromo-2-chloro-phenoxy)-phenylsulfamol]-benzoic acid

The title compound was prepared from 2-(4-bromo-2-chloro-phenoxy)-phenylamine according to the method described in Example 1/c. MS (EI) 483.4 (MH⁺).

### d) {4-[2-(4-Bromo-2-chloro-phenoxy)-phenylsulfamoyl]-benzoylamino}-acetic acid

### ethyl ester

The title compound was prepared from 4-[2-(4-bromo-2-chloro-phenoxy)-phenylsulfamoyl]-benzoic acid and glycine ethyl ester hydrochloride according to the method described in Example 1/d.

### e) {4-[2-(4-Bromo-2-chloro-phenoxy)-phenylsulfamoyl]-benzoylamino}-acetic acid

The title compound was prepared from {4-[2-(4-bromo-2-chloro-phenoxy)-phenylsulfamoyl]-benzoylamino}-acetic acid ethyl ester according to the method described in Example 2/b. MS (EI) 541.1 (MH⁺).

### f) trans-4-[2-(4-Bromo-2-chloro-phenoxy)-phenylsulfamoyl]-N-{[4-(2-pyrrolidin-1-yl-ethyl)-cyclohexylcarbamoyl]-methyl}-benzamide

The title compound was prepared from {4-[2-(4-bromo-2-chloro-phenoxy)-phenylsulfamoyl]-benzoylamino}-acetic acid and trans-4-(2-pyrrolidin-1-yl-ethyl)-cyclohexylamine dihydrochloride (Reference Example 4) according to the method described in Example 2/c. MS (EI) 719.4 (MH⁺).

### Example 113

### Preparation of pharmaceutical compositions:

### a) Tablets:

0.01-50 % of active ingredient of formula (I), 15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin were mixed, then granulated by wet granulation and pressed to tablets.

### b) Dragées, filmcoated tablets:

The tablets made according to the method described above were coated by a layer consisting of entero- or gastrosolvent film, or of sugar and talc. The dragées were polished by a mixture of beeswax and carnuba wax.

### c) Capsules:

0.01-50 % of active ingredient of formula (I), 1-5 % of sodium lauryl sulfate, 15-50 % of starch, 15-50 % of lactose, 1-3 % of colloid silicon dioxide and 0.01-3 % of magnesium stearate were thoroughly mixed, the mixture was passed through a sieve and filled in hard gelatin capsules.

### d) Suspensions:

Ingredients: 0.01-15 % of active ingredient of formula (I), 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol was added in small portions under vigorous stirring, and the solution was left to stand for 10-12 h. Then the sodium hydroxide in 1 ml of distilled water, the aqueous solution of sorbitol and finally the ethanolic raspberry flavor were added with stirring. To this carrier the active ingredient was added in small portions and suspended with an immersing homogenizator. Finally the suspension was filled up to the desired final volume with distilled water and the suspension syrup was passed through a colloid milling equipment.

### e) Suppositories:

For each suppository 0.01-15% of active ingredient of formula (I) and 1-20% of lactose were thoroughly mixed, then 50-95% of adeps pro suppository (for example Witepsol 4) was melted, cooled to 35 °C and the mixture of active ingredient and lactose was mixed in it with homogenizator. The obtained mixture was mould in cooled forms.

### f) Lyophilized powder ampoule compositions:

A 5 % solution of mannitol or lactose was made with bidistilled water for injection use, and the solution was filtered so as to have sterile solution. A 0.01-5 % solution of the active ingredient of formula (I) was also made with bidistilled water for injection use, and this solution was filtered so as to have sterile solution. These two solutions were mixed under aseptic conditions, filled in 1 ml portions into ampoules, the content of the ampoules was lyophilized, and the ampoules were sealed under nitrogen. The contents of the ampoules were dissolved in sterile water or 0.9 % (physiological) sterile aqueous sodium chloride solution before administration.

## Claims

1. Bradykinin B1 receptor antagonist phenylsulfamoyl benzamide derivatives of A formula (I) 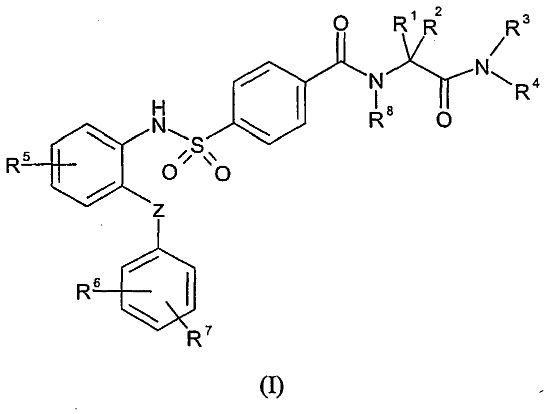 wherein
R¹ is hydrogen atom or C₁-C₄ alkyl group;
R² is selected from (I) hydrogen atom; (2) C₁-C₆ straight or branched alkyl group; (3) -(CH₂)ₙ-NH₂; (4) -(CH₂)ₙ-OH; (5) -(CH₂)ₙ-CO-NH₂; (6) -(CH₂)ₙ-COOR^{c}; (7) benzyl optionally substituted with one or more hydroxy group or halogen atom; or
R¹, R² and the carbon atom to which they are both attached together form a 3-7 membered cycloalkyl ring;
R³ is (1) hydrogen atom, (2) C₁-C₈ straight or branched alkyl group, optionally substituted with one or more substituents independently selected from amino, hydroxy, 1*H*-imidazol-4-yl, -NR^{a}R^{b}, -COOR^{c}, -NH-C(=NH)-NH₂, -CO-NH₂ group;
R⁴ is (1) hydrogen atom, (2) -(CH₂)ₙ-NR^{a}R^{b} group; (3) -(CH₂)ₘ-X-P group;
R⁵, R⁶ and R⁷ are independently of each other hydrogen atom, halogen atom, cyano, nitro, amino, or amino substituted with one or more C₁-C₄ alkyl group; trifluoromethyl; C₁-C₄ alkyl, C₁-C₄ alkoxy, -C(=O)-NH₂,-C₁-C₄ alkoxycarbonyl, trifluoromethoxy or hydroxy group;
R⁸ is hydrogen atom or C₁-C₄ alkyl group;
Z is selected from (1) single bond; (2) oxygen atom; (3) CH₂ group; (4) CO group; (5) NR^{c} group; (6) S atom; (7) SO₂ group;
n is an integer from 1 to 6;
m is an integer from 0 to 6;
X is selected from (1) single bond; (2) oxygen atom; (3) -CO-NR^{c} group; (4) CO or SO₂ group;
P is selected from (1) phenyl group, optionally substituted with one or more halogen atom, hydroxy, -(CH₂)ₘ-CN, -O-CO-NR^{c}R^{c}, -NH-CO-R^{c}, C₁-C₄ alkyl, C₁-C₄ alkoxy, cyano, amino, [1,4']bipiperidinyl-1'-yl or -(CH₂)ₙ-NH-C(=NH)-NH₂ group; (2) a saturated, partially unsaturated or aromatic 4-7 membered ring containing 1-3 heteroatom selected from O, S, SO₂ and N; wherein said ring is optionally substituted with one or more halogen atom, oxo, hydroxy, cyano, amino, trifluoromethyl, -NH-CO-R^{c}, -C(=NH)-NH₂, C₁-C₄ alkyl, pyridin-4-yl, piperidin-1-yl or pyridine-2-yl group; (3) a saturated, partially unsaturated or aromatic 8-10 membered bicyclic ring system containing 1-3 heteroatom selected from O, S, SO₂ and N; wherein said ring system is optionally substituted with one or more halogen atom, oxo, hydroxy, cyano, amino, -NH-CO-R^{c}, trifluoromethyl or C₁-C₄ alkyl group; (4) C₅-C₈ cycloalkyl group, optionally substituted with -(CH₂)ₘ-NR^{a}R^{b} group;
R^{a} and R^{b} are (1) hydrogen atom; (2) straight or branched C₁-C₆ alkyl group; (3) R^{a}, R^{b} and the nitrogen atom to which they are both attached together form a saturated, partially unsaturated or aromatic 4-7 membered ring containing 0-3 heteroatom (in addition to the nitrogen atom to which R^{a} and R^{b} attached) selected from O, S, SO₂ and N; wherein said ring is optionally substituted with one or more halogen atom, oxo, cyano, hydroxy or C₁-C₄ alkyl group;
R^{c} is hydrogen atom or C₁-C₄ alkyl group;
and optical antipodes or racemates and/or salts and/or hydrates and/or solvates thereof.

2. A compound of Claim 1 selected from the group of 4-[2-(2,4-dichloro-phenoxy)-phenylsulfamoyl]-*N-*{[(piperidin-4-ylmethyl)-carbamoyl]-methyl}-benzamide hydrochloride; 4-[2-(2,4-dichloro-phenoxy)-phenylsulfamoyl]-*N*-[(3-piperidin-1-yl-propylcarbamoyl)-methyl]-benzamide; 4-[2-(4-bromo-phenoxy)-phenylsulfamoyl]-*N*-{[(piperidin-4-ylmethyl)-carbamoyl]-methyl} -benzamide trifluoroacetate; 4-[2-(4-bromo-phenoxy)-5-fluoro-phenylsulfamoyl]-*N-*{[(piperidin-4-ylmethyl)-carbamoyl]-methyl}-benzamide mono trifluoroacetate; 4-(2-phenoxy-phenylsulfamoyl)-*N*- {[(piperidin-4-ylmethyl)-carbamoyl]-methyl}-benzamide hydrochloride; 4-[2-(2,4-dichloro-phenoxy)-phenylsulfamoyl]-*N*-{2-phenyl-1-[(piperidin-4-ylmethyl)-carbamoyl]-ethyl}-benzamide hydrochloride; 4-(5-fluoro-2-phenoxy-phenylsulfamoyl)-*N*-{[(piperidin-4-ylmethyl)-carbamoyl]-methyl}-benzamide hydrochloride; 4-[2-(2,4-dichloro-phenoxy)-phenylsulfamoyl]-*N*-{(*S*)-1-[(piperidin-4-ylmethyl)-carbamoyl]-ethyl}-benzamide hydrochloride; 4-(2-phenoxy-phenylsulfamoyl)-*N*-[(2-piperidin-4-yl-ethylcarbamoyl)-methyl]-benzamide hydrochloride; 4-[2-(2,4-dichloro-phenoxy)-4-methoxy-phenylsulfamoyl]-*N*-{[(piperidin-4-ylmethyl)-carbamoyl]-methyl}-benzamide hydrochloride; 4-[2-(4-fluoro-phenoxy)-phenylsulfamoyl]-*N-*{[(piperidin-4-ylmethyl)-carbamoyl]-methyl}-benzamide hydrochloride; 4-[5-fluoro-2-(4-fluoro-phenoxy)-phenylsulfamoyl]-*N*-[(2-piperidin-4-yl-ethylcarbamoyl)-methyl]-benzamide hydrochloride; *N*-{[(1-carbamimidoyl-piperidin-4-ylmethyl)-carbamoyl]-methyl}-4-[2-(2,4-dichloro-phenoxy)-phenylsulfamoyl]-benzamide hydrochloride; 4-[5-fluoro-2-(4-fluoro-phenoxy)-phenylsulfamoyl]-*N*-{[(2-piperidin-4-ylmethyl)-carbamoyl]-methyl}-benzamide hydrochloride; *N*-[(2-oxo-2-piperazin-1-yl-ethylcarbamoyl)-methyl]-4-(2-phenoxy-phenylsulfamoyl)-benzamide hydrochloride; 4-(2-benzoyl-phenylsulfamoyl)-*N*-{[(piperidin-4-ylmethyl)-carbamoyl]-methyl}-benzamide hydrochloride; 4-[2-(2,4-dichloro-phenoxy)-phenylsulfamoyl]-*N*-[(2-piperidin-4-yl-ethylcarbamoyl)-methyl]-benzamide acetate; 4-[2-(2,4-dichloro-phenoxy)-phenylsulfamoyl]-*N*-[(2-piperidin-1-yl-ethylearbamoyl)-methyl]-benzamide; 4-[2-(2,4-dichloro-phenoxy)-phenylsulfamoyl]-*N*-[(3-dimethylamino-propylcarbamoyl)-methyl]-benzamide; 4-[2-(2,4-dichloro-phenoxy)-phenylsulfamoyl]-*N*-[(3-morpholin-4-yl-propylcarbatnoyl)-methyl]-benzamide; 4-[2-(2,4-dichloro-phenoxy)-phenylsulfamoyl]-*N*-{[3-(2-methyl-piperidin-1-yl)-propylcarbamoyl]-methyl} -benzamide; 4-[2-(2,4-dichloro-phenoxy)-phenylsulfamoyl]-*N*-[(3-imidazol-1-yl-propylcarbamoyl)-methyl]-benzamide; 4-[2-(2,4-dichloro-phenoxy)-phenylsulfamoyl]-*N*-[(2-morpholin-4-yl-ethylcarbamoyl)-methyl]-benzamide; 4-[2-(2,4-dichloro-phenoxy)-phenylsulfamoyl]-*N*-[(2-pyrrolidin-1-yl-ethylcarbamoyl)-methyl]-benzamide; 4-[2-(2,4-dichloro-phenoxy)-phenylsulfarnoyl]-*N*-[(3-dimethylamino-2,2-dimethyl-propylcarbamoyl)-methyl]-benzamide; 4-[2-(4-bromo-2-chloro-phenoxy)-phenylsulfamoyl]-*N*-{[(piperidin-4-ylmethyl)-carbamoyl]-methyl}-benzamide hydrochloride; 4-[2-(2,4-dichloro-phenoxy)-phenylsulfamoyl]-*N*-[(4-guanidinomethyl-benzylcarbamoyl)-methyl]-benzamide hydrochloride; 4-[2-(2,4-dichloro-phenoxy)-phenylsulfamoyl]-*N*-{[methyl-(2-pyridin-4-yl-ethyl)-carbamoyl]-methyl}-benzamide; *N*-[(3-[1,4']bipiperidinyl-1'-yl-propylcarbamoyl)-methyl]-4-(2-phenoxy-phenylsulfamoyl)-benzamide; *N*-[(4-[1,4']bipiperidinyl-1'-yl-phenylcarbamoyl)-methyl]-4-(2-phenoxy-phenylsulfamoyl)-benzamide; trans-4-(2-phenoxy-phenylsulfamoyl)-*N*-([4-(2-pyrrolidin-1-yl-ethyl)-cyclohexyl-carbamoyl]-methyl}-benzamide; *N*-[(3-dimethylamino-propylcarbamoyl)-methyl]-4-(2-phenoxy-phenylsulfamoyl)-benzamide; *N*-{[3-(2-methyl-piperidin-1-yl)-propylcarbamoyl]-methyl}-4-(2-phenoxy-phenylsulfamoyl)-benzamide; 4-[2-(2,4-dichloro-phenoxy)-phenylsulfamoyl]-*N*-[(4-piperidin-4-yl-butylcarbamoyl)-methyl]-benzamide hydrochloride; 4-[2-(2,4-dichloro-phenoxy)-phenylsulfamoyl]-*N*-(piperidin-4-ylcarbamoylmethyl)-benzamide hydrochloride; 4-[2-(2,4-dichloro-phenoxy)-phenylsulfamoyl]-*N*-[(3-piperidin-4-yl-propylcarbamoyl)-methyl]-benzamide hydrochloride; 4-[2-(2,4-dichloro-phenoxy)-phenylsulfamoyl]-*N*-[(4-piperidin-1-yl-cyclohexyl-carbamoyl)-methyl]-benzamide; 4-[2-(2,4-dichloro-phenoxy)-phenylsulfamoyl]-*N*-[(4-piperidin-1-yl-butylcarbamoyl)-methyl]-benzamide; 4-(2-phenoxy-phenylsulfamoyl)-*N-*(piperidin-4-ylcarbamoylmethyl)-benzamide hydrochloride; 4-(2-phenylamino-phenylsulfamoyl)-*N*-{[(piperidin-4-ylmethyl)-carbamoyl]-methyl}-benzamide hydrochloride or *N*-[(4-cyanomethyl-phenylcarbamoyl)-methyl]-4-(2-phenoxy-phenylsulfamoyl)-benzamide.

3. A process for preparing the compounds of formula (I) as claimed in Claim 1 which comprises
Method a)
reacting an amine derivative of formula (II) 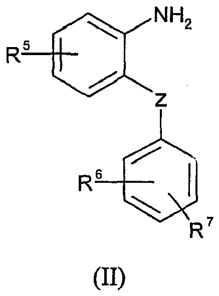 - wherein the meaning of R⁵, R⁶ and R⁷ is as described above for the formula (I) - with the sulfonyl chloride of formula (III) 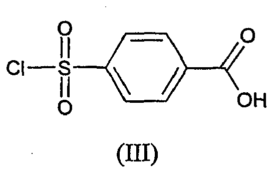 then reacting the so obtained phenylsulfamoyl benzoic acid derivative of formula (IV) 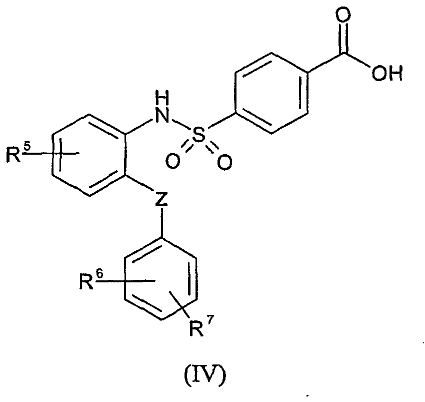 - wherein the meaning of R⁵, R⁶ and R⁷ is as defined above - with an amine derivative of formula (V) 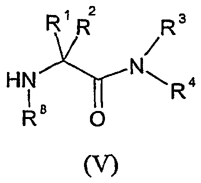 - wherein the meaning of R¹, R², R³, R⁴ and R⁸ is as defined above - to obtain a phenylsulfamoyl benzamide derivative of formula (I) or optical antipodes or racemates and/or pharmaceutically acceptable salts and/or hydrates and/or solvates thereof.
Method b)
Reacting the phenylsulfamoyl benzoic acid derivative of formula (IV) - obtained according to the method described in method a) - with an amino acid derivative of formula (VI) 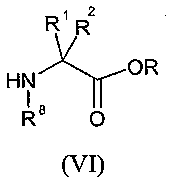 - wherein the meaning of R¹, R² and R⁸ is as defined above, and R is C₁-C₄ alkyl group - and hydrolyzing the so obtained compound of formula (VII) 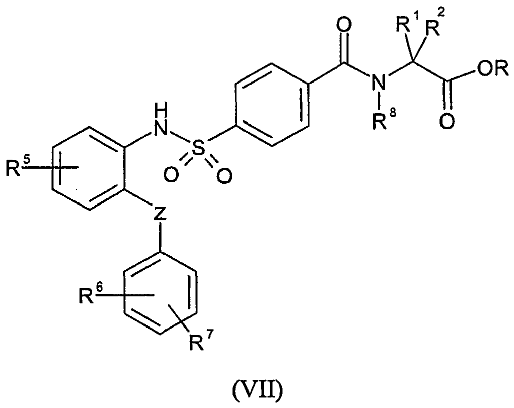 - wherein the meaning of R¹, R², R⁵, R⁶, R⁷, R⁸ and R is as defined above - to furnish a carboxylic acid derivative of formula (VIII) 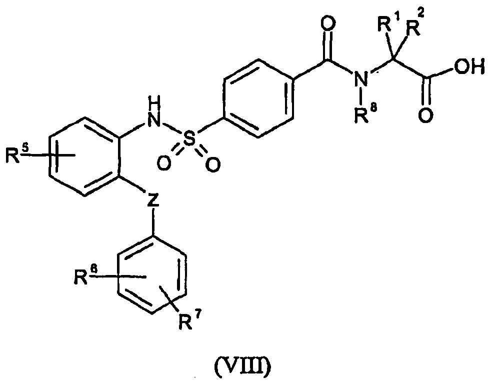 - wherein the meaning of R¹, R², R⁵, R⁶, R⁷ and R⁸ is as defined above - finally reacting the latter with an amine derivative of formula (IX) 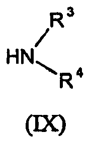 - wherein the meaning of R³ and R⁴ is as defined above - to obtain a phenylsulfamoyl benzamide derivative of formula (I) or optical antipodes or racemates and/or pharmaceutically acceptable salts and/or hydrates and/or solvates thereof.

4. A process for preparing the compounds of formula (I) as claimed in Claim 1 which comprises transforming a compound of formula (I) into an other compound of formula (I) by introducing new substituents and/or modifying or removing the existing ones, and/or salt formation and/or liberating the compound from salts.

5. A compound of formula (V), which is 4-[2-(2-amino-acetylamino)-ethyl]-piperidine-1-carboxylic acid *tert*-butyl ester.

6. A compound of formula (IX) selected from the group of *N*-(4-aminomethyl-benzyl)-guanidine dihydrochloride or 2-(4-pyridin-4-yl-piperazin-1-yl)-propylamine.

7. A pharmaceutical composition comprising a therapeutically effective amount of a compound of formula (I) as claimed in Claim 1 or optical antipodes or racemates or pharmaceutically acceptable salt or hydrate or solvate thereof and one or more pharmaceutically acceptable excipients.

8. Use of a compound of formula (I) as claimed in Claim 1 or optical antipodes or racemates or a pharmaceutically acceptable salt or hydrate or solvate thereof for the manufacture of a medicament for prevention and/or treatment of a condition which requires inhibition of a bradykinin receptor.

9. Use according to Claim 8 wherein the bradykinin receptor is bradykinin B1 receptor.

10. The compound of formula (I) as claimed in Claim 1 or optical antipodes or racemates or pharmaceutically acceptable salt or hydrate or solvate thereof for use in a method of treating and/or preventing a condition which requires inhibition of a bradykinin receptor.

11. The compound for use according to Claim 10 wherein the bradykinin receptor is bradykinin B1 receptor.

## Patentansprüche

1. Bradykinin B1-Rezeptor-Antagonist-Phenylsulfamoylbenzamidderivat der Formel (I) 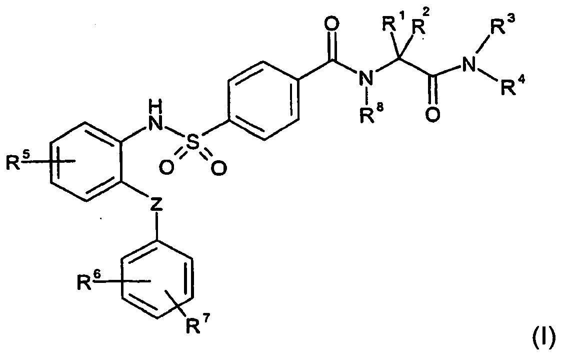 worin:
R¹ ein Wasserstoffatom oder eine C₁₋₄-Alkylgruppe ist;
R² aus (1) einem Wasserstoffatom; (2) einer geradkettigen oder verzweigten C₁₋₆-Alkylgruppe; (3) -(CH₂)ₙ-NH₂; (4) -(CH₂)ₙ-OH; (5) -(CH₂)ₙ-CO-NH₂; (6) -(CH₂)ₙ-COOR^{c}; (7) gegebenenfalls mit einer/einem oder mehreren Hydroxygruppe(n) oder Halogenatom(en) substituiertem Benzyl ausgewählt ist; oder
R¹, R² und das Kohlenstoffatom, an das sie beide gebunden sind, einen 3- bis 7-gliedrigen Cycloalkylring bilden;
R³ (1) ein Wasserstoffatom, (2) eine geradkettige oder verzweigte C₁₋₈-Alkylgruppe, gegebenenfalls substituiert mit einem oder mehreren Substituenten, unabhängig ausgewählt aus Amino, Hydroxy, 1H-Imidazol-4-yl, -NR^{a}R^{b}, -COOR^{c}, -NH-C(=NH)-NH₂, einer -CO-NH₂-Gruppe, ist;
R⁴ (1) ein Wasserstoffatom, (2) eine -(CH₂)ₙ-NR^{a}R^{b}-Gruppe; (3) eine -(CH₂)ₘ-X-P-Gruppe ist;
R⁵, R⁶ und R⁷ jeweils unabhängig voneinander ein Wasserstoffatom, ein Halogenatom, Cyano, Nitro, Amino oder mit einer oder mehreren C₁₋₄-Alkylgruppe(n) substituiertes Amino; Trifluormethyl; C₁₋₄-Alkyl, C₁₋₄-Alkoxy, -C(=O)-NH₂, C₁₋₄-Alkoxycarbonyl, Trifluormethoxy oder eine Hydroxygruppe sind;
R⁸ ein Wasserstoffatom oder eine C₁₋₄-Alkylgruppe ist;
Z aus (1) einer Einfachbindung; (2) einem Sauerstoffatom; (3) einer CH₂-Gruppe; (4) einer CO-Gruppe; (5) einer NR^{c}-Gruppe; (6) einem S-Atom; (7) einer SO₂-Gruppe ausgewählt ist;
n eine ganze Zahl von 1 bis 6 ist;
m eine ganze Zahl von 0 bis 6 ist;
X aus (1) einer Einfachbindung; (2) einem Sauerstoffatom; (3) einer -CO-NR^{c}-Gruppe; (4) einer CO- oder SO₂-Gruppe ausgewählt ist;
P aus (1) einer Phenylgruppe, gegebenenfalls substituiert mit einem oder mehreren Halogenatom(en), Hydroxy, -(CH₂)ₘ-CN, -O-CO-NR^{c}R^{c}, -NH-CO-R^{c}, C₁₋₄-Alkyl, C₁₋₄-Alkoxy, Cyano, Amino, [1,4']-Bipiperidinyl-1'-yl oder einer -(CH₂)ₙ-NH-C(=NH)-NH₂-Gruppe; (2) einem gesättigten, teilweise ungesättigten oder aromatischen 4- bis 7-gliedrigen Ring, enthaltend 1 bis 3 Heteroatom(e), ausgewählt aus O, S, SO₂ und N; wobei dieser Ring gegebenenfalls substituiert ist mit einem oder mehreren Halogenatom(en), Oxo, Hydroxy, Cyano, Amino, Trifluormethyl, -NH-CO-R^{c}, -C(=NH)-NH₂, C₁₋₄-Alkyl, Pyridin-4-yl, Piperidin-1-yl oder einer Pyridin-2-yl-Gruppe; (3) einem gesättigten, teilweise ungesättigten oder aromatischen 8- bis 10-gliedrigen bicyclischen Ringsystem, enthaltend 1 bis 3 Heteroatom(e), ausgewählt aus O, S, SO₂ und N; wobei dieses Ringsystem gegebenenfalls substituiert ist mit einem oder mehreren Halogenatom(en), Oxo, Hydroxy, Cyano, Amino, -NH-CO-R^{c}, Trifluormethyl oder einer C₁₋₄-Alkylgruppe; (4) einer C₅₋₈-Cycloalkylgruppe, gegebenenfalls substituiert mit einer -(CH₂)ₘ-NR^{a}R^{b}-Guppe, ausgewählt ist;
R^{a} und R^{b} (1) ein Wasserstoffatom; (2) eine geradkettige oder verzweigte C₁₋₆-Alkylgruppe sind; (3) R^{a}, R^{b} und das Stickstoffatom, an das sie beide gebunden sind, zusammen einen gesättigten, teilweise ungesättigten oder aromatischen 4- bis 7-gliedrigen Ring, enthaltend 0 bis 3 Heteroatom(e) (zusätzlich zu dem Stickstoffatom, an das R^{a} und R^{b} gebunden sind), ausgewählt aus O, S, SO₂ und N, bilden; wobei dieser Ring gegebenenfalls substituiert ist mit einem oder mehreren Halogenatom(en), Oxo, Cyano, Hydroxy oder einer C₁₋₄-Alkylgruppe;
R^{c} ein Wasserstoffatom oder eine C₁₋₄-Alkylgruppe ist;
und optische Antipoden oder Racemate und/oder Salze und/oder Hydrate und/oder Solvate davon.

2. Verbindung gemäss Anspruch 1, ausgewählt aus der Gruppe:
4-[2-(2,4-Dichlor-phenoxy)-phenylsulfamoyl]-N-{[(piperidin-4-ylmethyl)-carbamoyl]-methyl}-benzamid-Hydrochlorid;
4-[2-(2,4-Dichlor-phenoxy)-phenylsulfamoyl]-N-[(3-piperidin-1-yl-propylcarbamoyl)-methyl]-benzamid;
4-[2-(4-Brom-phenoxy)-phenylsulfamoyl]-N-{[(piperidin-4-ylmethyl)-carbamoyl]-methyl}-benzamid trifluoracetat;
4-[2-(4-Brom-phenoxy)-5-fluor-phenylsulfamoyl]-N-{[(piperidin-4-ylmethyl)-carbamoyl]-methyl}-benzamid monotrifluoracetat;
4-(2-Phenoxy-phenylsulfamoyl)-N-{[(piperidin-4-ylmethyl)-carbamoyl]-methyl}-benzamid-Hydrochlorid;
4-[2-(2,4-Dichlor-phenoxy)-phenylsulfamoyl]-N-{2-phenyl-1-[(piperidin-4-ylmethyl)-carbamoyl]-ethyl}-benzamid-Hydrochlorid;
4-(5-fluor-2-phenoxy-phenylsulfamoyl)-N-{[(piperidin-4-ylmethyl)-carbamoyl]-methyl}-benzamid-Hydrochlorid;
4-[2-(2,4-Dichlor-phenoxy)-phenylsulfamoyl]-N-{(S)-1-[(piperidin-4-ylmethyl)-carbamoyl]-ethyl}-benzamid-Hydrochlorid;
4-(2-Phenoxy-phenylsulfamoyl)-N-[(2-piperidin-4-yl-ethylcarbamoyl)-methyl]-benzamid-Hydrochlorid;
4-[2-(2,4-Dichlor-phenoxy)-4-methoxy-phenylsulfamoyl]-N-{[(piperidin-4-ylmethyl)-carbamoyl]-methyl}-benzamid-Hydrochlorid;
4-[2-(4-Fluor-phenoxy)-phenylsulfamoyl]-N-{[(piperidin-4-ylmethyl)-carbamoyl]-methyl}-benzamid-Hydrochlorid;
4-[5-Fluor-2-(4-fluor-phenoxy)-phenylsulfamoyl]-N-[(2-piperidin-4-yl-ethylcarbamoyl)-methyl]-benzamid-Hydrochlorid;
N-{[(1-Carbamimidoyl-piperidin-4-ylmethyl)-carbamoyl]-methyl}-4-[2-(2,4-dichlor-phenoxy)-phenylsulfamoyl]-benzamid-Hydrochlorid;
4-[5-Fluor-2-(4-fluor-phenoxy)-phenylsulfamoyl]-N-{[(2-piperidin-4-ylmethyl)-carbamoyl]-methyl}-benzamid-Hydrochlorid;
N-[(2-Oxo-2-piperazin-1-yl-ethylcarbamoyl)-methyl]-4-(2-phenoxy-phenylsulfamoyl)-benzamid-Hydrochlorid;
4-(2-Benzoyl-phenylsulfamoyl)-N-{[(piperidin-4-ylmethyl)-carbamoyl]-methyl}-benzamid-Hydrochlorid;
4-[2-(2,4-Dichlor-phenoxy)-phenylsulfamoyl]-N-[(2-piperidin-4-yl-ethylcarbamoyl)-methyl]-benzamidacetat;
4-[2-(2,4-Dichlor-phenoxy)-phenylsulfamoyl]-N-[(2-piperidin-1-yl-ethylcarbamoyl)-methyl]-benzamid;
4-[2-(2,4-Dichlor-phenoxy)-phenylsulfamoyl]-N-[(3-dimethylamino-propylcarbamoyl)-methyl]-benzamid;
4-[2-(2,4-Dichlor-phenoxy)-phenylsulfamoyl]-N-[(3-morpholin-4-yl-propylcarbamoyl)-methyl]-benzamid;
4-[2-(2,4-Dichlor-phenoxy)-phenylsulfamoyl]-N-{[3-(2-methyl-piperidin-1-yl)-propylcarbamoyl]-methyl}-benzamid;
4-[2-(2,4-Dichlor-phenoxy)-phenylsulfamoyl]-N-[(3-imidazol-1-yl-propylcarbamoyl)-methyl]-benzamid;
4-[2-(2,4-Dichlor-phenoxy)-phenylsulfamoyl]-N-[(2-morpholin-4-yl-ethylcarbamoyl)-methyl]-benzamid;
4-[2-(2,4-Dichlor-phenoxy)-phenylsulfamoyl]-N-[(2-pyrrolidin-1-yl-ethylcarbamoyl)-methyl]-benzamid;
4-[2-(2,4-Dichlor-phenoxy)-phenylsulfamoyl]-N-[(3-dimethylamino-2,2-dimethyl-propylcarbamoyl)-methyl]-benzamid;
4-[2-(4-Brom-2-chlor-phenoxy)-phenylsulfamoyl]-N-{[(piperidin-4-ylmethyl)-carbamoyl]-methyl}-benzamid-Hydrochlorid;
4-[2-(2,4-Dichlor-phenoxy)-phenylsulfamoyl]-N-[(4-guanidinomethyl-benzylcarbamoyl)-methyl]-benzamid-Hydrochlorid;
4-[2-(2,4-Dichlor-phenoxy)-phenylsulfamoyl]-N-{[methyl-(2-pyridin-4-yl-ethyl)-carbamoyl]-methyl}-benzamid;
N-[(3-[1,4']bipiperidinyl-1'-yl-propylcarbamoyl)-methyl]-4-(2-phenoxy-phenylsulfamoyl)-benzamid;
N-[(4-[1,4']bipiperidinyl-1'-yl-phenylcarbamoyl)-methyl]-4-(2-phenoxy-phenylsulfamoyl)-benzamid;
trans-4-(2-Phenoxy-phenylsulfamoyl)-N-{[4-(2-pyrrolidin-1-yl-ethyl)-cyclohexyl-carbamoyl]-methyl}-benzamid;
N-[(3-Dimethylamino-propylcarbamoyl)-methyl]-4-(2-phenoxy-phenylsulfamoyl)-benzamid;
N-{[3-(2-Methyl-piperidin-1-yl)-propylcarbamoyl]-methyl}-4-(2-phenoxy-phenylsulfamoyl)-benzamid;
4-[2-(2,4-Dichlor-phenoxy)-phenylsulfamoyl]-N-[(4-piperidin-4-yl-butylcarbamoyl)-methyl]-benzamid-Hydrochlorid;
4-[2-(2,4-Dichlor-phenoxy)-phenylsulfamoyl]-N-(piperidin-4-ylcarbamoylmethyl)-benzamid-Hydrochlorid;
4-[2-(2,4-Dichlor-phenoxy)-phenylsulfamoyl]-N-[(3-piperidin-4-yl-propylcarbamoyl)-methyl]-benzamid-Hydrochlorid;
4-[2-(2,4-Dichlor-phenoxy)-phenylsulfamoyl]-N-[(4-piperidin-1-yl-cyclohexyl-carbamoyl)-methyl]-benzamid;
4-[2-(2,4-Dichlor-phenoxy)-phenylsulfamoyl]-N-[(4-piperidin-1-yl-butylcarbamoyl)-methyl]-benzamid;
4-(2-Phenoxy-phenylsulfamoyl)-N-(piperidin-4-ylcarbamoylmethyl)-benzamid-Hydrochlorid;
4-(2-Phenylamino-phenylsulfamoyl)-N-{[(piperidin-4-ylmethyl)-carbamoyl]-methyl}-benzamid-Hydrochlorid oder
N-[(4-Cyanomethyl-phenylcarbamoyl)-methyl]-4-(2-phenoxy-phenylsulfamoyl)-benzamid.

3. Verfahren zur Herstellung von Verbindungen der Formel (I) gemäss Anspruch 1, umfassend:
Verfahren (a) :
Umsetzen eines Aminderivats der Formel (II): 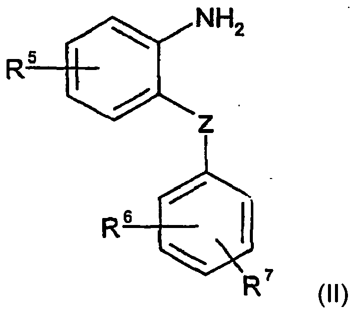 - worin die Bedeutungen von R⁵, R⁶ und R⁷ wie oben für die Formel (I) beschrieben sind - mit einem Sulfonylchlorid der Formel (III): 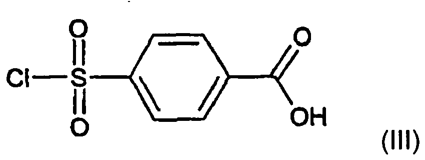 dann Umsetzen des so erhaltenen
Phenylsulfamoylbenzoesäurederivats der Formel (IV): 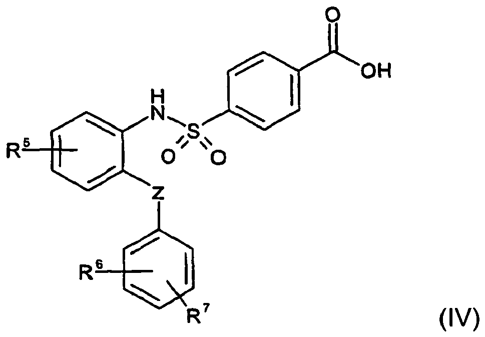 - worin die Bedeutungen von R⁵, R⁶ und R⁷ wie oben definiert sind - mit einem Aminderivat der Formel (V) : 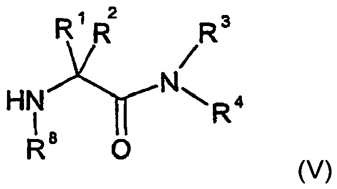 - worin die Bedeutungen von R¹, R², R³, R⁴ und R⁸ wie oben definiert sind - um ein Phenylsulfamoylbenzamidderivat der Formel (I) oder optische Antipoden oder Racemate und/oder pharmazeutisch annehmbare Salze und/oder Hydrate und/oder Solvate davon zu erhalten;
Verfahren (b):
Umsetzen eines Phenylsulfamoylbenzoesäurederivats der Formel (IV) - erhalten gemäss dem in Verfahren (a) beschriebenen Verfahren - mit einem Aminosäurederivat der Formel (VI) : 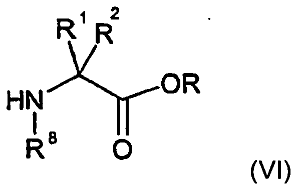 - worin die Bedeutungen von R¹, R² und R⁸ wie oben definiert sind und R eine C₁₋₄-Alkylgruppe ist - und Hydrolysieren der so erhaltenen Verbindung der Formel (VII) : 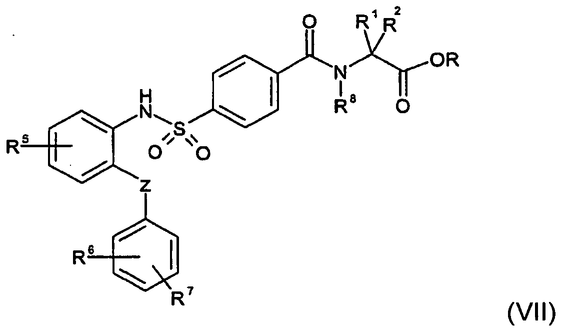 - worin die Bedeutungen von R¹, R², R⁵, R⁶, R⁷, R⁸ und R wie oben definiert sind - um ein Carbonsäurederivat der Formel (VIII): 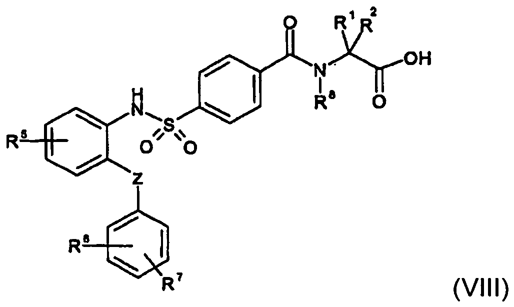 - worin die Bedeutungen von R¹, R², R⁵, R⁶, R⁷ und R⁸ wie oben definiert sind - zu liefern, und schliesslich Umsetzen des letzteren mit einem Aminderivat der Formel (IX): 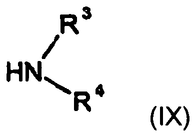 - worin die Bedeutungen von R³ und R⁴ wie oben definiert sind - um ein
Phenylsulfamoylbenzamidderivat der Formel (I) oder optische Antipoden oder Racemate und/oder pharmazeutisch annehmbare Salze und/oder Hydrate und/oder Solvate davon zu erhalten.

4. Verfahren zur Herstellung der Verbindungen der Formel (I) gemäss Anspruch 1, das die Umwandlung einer Verbindung der Formel (I) in eine andere Verbindung der Formel (I) durch Einführen neuer Substanzen und/oder Modifizieren oder Entfernen der existierenden Substanzen und/oder Salzbildung und/oder Freisetzen der Verbindung von dem Salz umfasst.

5. Verbindung der Formel (V), die 4-[2-(2-Amino-acetylamino)-ethyl]-piperidin-1-carbonsäure-tert-butylester ist.

6. Verbindung der Formel (IX), ausgewählt aus der Gruppe N-(4-Aminomethyl-benzyl)-guanidin-Dihydrochlorid oder 2-(4-Pyridin-4-yl-piperazin-1-yl)-propylamin.

7. Pharmazeutische Zusammensetzung, umfassend eine therapeutisch wirksame Menge einer Verbindung der Formel (I) gemäss Anspruch 1 oder optische Antipoden oder Racemate oder pharmazeutisch annehmbare Salze oder Hydrate oder Solvate davon und einen oder mehrere Hilfsstoff(e).

8. Verwendung einer Verbindung der Formel (I) gemäss Anspruch 1 oder optischen Antipoden oder Racematen oder pharmazeutisch annehmbaren Salzen oder Hydraten oder Solvaten davon zur Herstellung eines Medikaments zur Vorbeugung und/oder Behandlung eines Zustands, der die Inhibierung eines Bradykinin-Rezeptors erfordert.

9. Verwendung gemäss Anspruch 8, wobei der Bradykinin-Rezeptor ein Bradykinin B1-Rezeptor ist.

10. Verbindung der Formel (I) gemäss Anspruch 1 oder optische Antipoden oder Racemate oder pharmazeutisch annehmbare Salze oder Hydrate oder Solvate davon zur Verwendung in einem Verfahren zur Behandlung und/oder Vorbeugung eines Zustands, der die Inhibierung eines Bradykinin-Rezeptors erfordert.

11. Verbindung zur Verwendung gemäss Anspruch 10, wobei der Bradykinin-Rezeptor ein Bradykinin B1-Rezeptor ist.

## Revendications

1. Dérivés de phénylsulfamoyl benzamide antagonistes de récepteur de bradykinine B1 de formule (I) 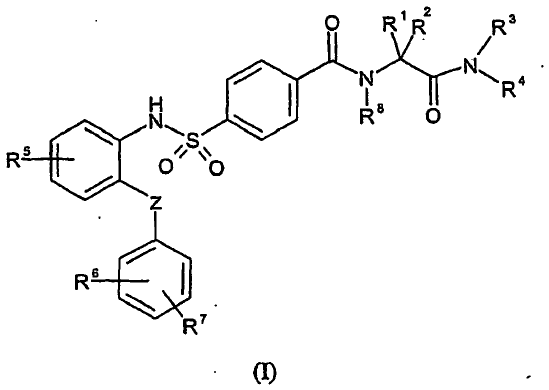 dans laquelle
R¹ est un atome d'hydrogène ou un groupe alkyle en C₁ à C₄ ;
R² est choisi parmi (1) un atome d'hydrogène ; (2) un groupe alkyle linéaire ou ramifié en C₁ à C₆ ; (3) -(CH₂)ₙ-NH₂; (4) -(CH₂)ₙ-OH ; (5) -(CH₂)ₙ-CO-NH₂ ; (6) -(CH₂)ₙ-COOR^{c}; (7) un groupe benzyle facultativement substitué par un ou plusieurs groupes hydroxy ou atomes d'halogène ; ou
R¹, R² et l'atome de carbone auquel ils sont tous deux attachés forment ensemble un cycle cycloalkyle à 3 à 7 chaînons ;
R³ est (1) un atome d'hydrogène, (2) un groupe alkyle linéaire ou ramifié en C₁ à C₈, facultativement substitué par un ou plusieurs substituants indépendamment choisis parmi les groupes amino, hydroxy, 1*H*-imidazol-4-yle, -NR^{a}R^{b}, -COOR^{c}, NH-C(=NH)-NH₂, -CO-NH₂ ;
R⁴ est (1) un atome d'hydrogène ; (2) un groupe -(CH₂)ₙ-NR^{a}R^{b}; (3) un groupe _{d}CH₂)ₘ-X-P;
R⁵, R⁶ et R⁷ sont indépendamment les uns des autres un atome d'hydrogène, un atome d'halogène, un groupe cyano, nitro, amino, ou amino substitué par un ou plusieurs groupes alkyle en C₁ à C₄ ; un groupe trifluorométhyle ; alkyle en C₁ à C₄, alcoxy en C₁ à C₄, -C(=O)-NH₂, alcoxycarbonyle en C₁ à C₄, trifluorométhoxy ou hydroxy ;
R⁸ est un atome d'hydrogène ou un groupe alkyle en C₁ à C₄ ;
Z est choisi parmi (1) une simple liaison ; (2) un atome d'oxygène ; (3) un groupe CH₂ ; (4) un groupe CO ; (5) un groupe NR^{c} ; (6) un atome S ; (7) un groupe SO₂;
n est un nombre entier de 1 à 6 ;
m est un nombre entier de 0 à 6 ;
X est choisi parmi (1) une simple liaison ; (2) un atome d'oxygène ; (3) un groupe -CO-NR^{c}; (4) un groupe CO ou SO₂;
P est choisi parmi (1) un groupe phényle, facultativement substitué par un ou plusieurs atomes d'halogène, groupes hydroxy, -(CH₂)ₘ-CN, -O-CO-NR^{c}R^{c}, -NH-CO-R^{c}, alkyle en C₁ à C₄, alcoxy en C₁ à C₄, cyano, amino, [1,4']bipipéridinyl-1'-yle ou - (CH₂)ₙ-NH-C(=NH)-NH₂ ; (2) un cycle à 4 à 7 chaînons saturé, partiellement insaturé ou aromatique contenant 1 à 3 hétéroatomes choisis parmi O, S, SO₂ et N ; où ledit cycle est facultativement substitué par un ou plusieurs atomes d'halogène, groupes oxo, hydroxy, cyano, amino, trifluorométhyle, -NH-CO-R^{c}, -C(=NH)-NH₂, alkyle en C₁ à C₄, pyridin-4-yle, pipéridin-1-yle ou pyridin-2-yle ; (3) un système cyclique bicyclique à 8 à 10 chaînons saturé, partiellement insaturé ou aromatique contenant 1 à 3 hétéroatomes choisis parmi O, S, SO₂ et N ; où ledit système cyclique est facultativement substitué par un ou plusieurs atomes d'halogène, groupes oxo, hydroxy, cyano, amino, -NH-CO-R^{c}, trifluorométhyle, ou alkyle en C₁ à C₄ ; (4) un groupe cycloalkyle en C₅ à C₈, facultativement substitué par un groupe -(CH₂)ₘ-NR^{a}R^{b} ;
R^{a} et R^{b} sont (1) un atome d'hydrogène ; (2) un groupe alkyle en C₁ à C₆ linéaire ou ramifié ; (3) R^{a}, R^{b} et l'atome d'azote auquel ils sont tous deux attachés forment ensemble un cycle à 4 à 7 chaînons saturé, partiellement saturé ou aromatique contenant 0 à 3 hétéroatomes (en plus de l'atome d'azote auquel R^{a} et R^{b} sont attachés) choisis parmi O, S, SO₂ et N ; où ledit cycle est facultativement substitué par un ou plusieurs atomes d'halogène, groupes oxo, cyano, hydroxy ou alkyle en C₁ à C₄;
R^{c} est un atome d'hydrogène ou un groupe alkyle en C₁ à C₄ ;
et leurs antipodes ou racémates optiques et/ou sels et/ou hydrates et/ou solvates.

2. Composé de la revendication 1 choisi dans le groupe constitué par le chlorhydrate de 4-[2-(2,4-dichloro-phénoxy)-phénylsulfamoyl]-*N*-{[(pipéridin-4-ylméthyl)-carbamoyl]-méthyl}-benzamide ; le 4-[2-(2,4-dichloro-phénoxy)-phénylsulfamoyl]-*N*-[(3-pipéridin-1-yl-propylcarbamoyl)-méthyl]-benzamide ; le trifluoroacétate de 4-[2-(4-bromo-phénoxy)-phénylsulfamoyl]-*N*-{[(pipéridin-4-ylméthyl)-carbamoyl]-méthyl}-benzamide; le mono trifluoroacétate de 4-[2-(4-bromo-phénoxy)-5-fluoro-phénylsulfamoyl]-*N*-{[(pipéridin-4-ylméthyl)-carbamoyl]-méthyl}-benzamide ; le chlorhydrate de 4-(2-phénoxy-phénylsulfamoyl)-*N*-{[(pipéridin-4-ylméthyl)-carbamoyl]-méthyl}-benzamide; le chlorhydrate de 4-[2-(2,4-dichloro-phénoxy)-phénylsulfamoyl]-*N*-{2-phényl-1-[(pipéridin-4-ylméthyl)-carbamoyl]-éthyl}-benzamide ; le chlorhydrate de 4-(5-fluoro-2-phénoxy-phénylsulfamoyl)-*N*-{[(pipéridin-4-ylméthyl)-carbamoyl]-méthyl}-benzamide ; le chlorhydrate de 4-[2-(2,4-dichloro-phénoxy)-phénylsulfamoyl]-*N-*{(S)-1-[(pipéridin-4-ylméthyl)-carbamoyl]-éthyl}-benzamide ; le chlorhydrate de 4-(2-phénoxy-phénylsulfamoyl)-*N*-[(2-pipéridin-4-yl-éthylcarbamoyl)-méthyl]-benzamide ; le chlorhydrate de 4-[2-(2,4-dichloro-phénoxy)-4-méthoxy-phénylsulfamoyl]-*N*-{[(pipéridin-4-ylméthyl)-carbamoyl]-méthyl}-benzamide; le chlorhydrate de 4-[2-(4-fluoro-phénoxy)-phénylsulfamoyl]-N-{[(pipéridin-4-ylméthyl)-carbamoyl]-méthyl}-benzamide ; le chlorhydrate de 4-[5-fluoro-2-(4-fluoro-phénoxy)-phénylsulfamoyl]-*N*-[(2-pipéridin-4-yl-éthylcarbamoyl)-méthyl]-benzamide ; le chlorhydrate de *N*-{[(1-carbamimidoyl-pipéridin-4-ylméthyl)-carbamoyl]-méthyl}-4-[2-(2,4-dichloro-phénoxy)-phénylsulfamoyl]-benzamide ; le chlorhydrate de 4-[5-fluoro-2-(4-fluoro-phénoxy)-phénylsulfamoyl]-*N*-{[(2-pipéridin-4-ylméthyl)-carbamoyl]-méthyl}-benzamide ; le chlorhydrate de *N*-[(2-oxo-2-pipérazin-1-yl-éthylcarbamoyl)-méthyl]-4-(2-phénoxy-phénylsulfamoyl)-benzamide ; le chlorhydrate de 4-(2-benzoyl-phénylsulfamoyl)-*N*-{[(pipéridin-4-ylméthyl)-carbamoyl]-méthyl}-benzamide; l'acétate de 4-[2-(2,4-dichloro-phénoxy)-phénylsulfamoyl)-*N*-[(2-pipéridin-4-yl-éthylcarbamoyl)-méthyl]-benzamide ; le 4-[2-(2,4-dichloro-phénoxy)-phénylsulfamoyl]-*N*-[(2-pipéridin-1-yl-éthylcarbamoyl)-méthyl]-benzamide; le 4-[2-(2,4-dichloro-phénoxy)-phénylsulfamoyl]-*N*-[(3-diméthylamino-propylcarbamoyl)-méthyl]-benzamide ; le 4-[2-(2,4-dichloro-phénoxy)-phénylsulfamoyl]-*N*-[(3-morpholin-4-yl-propylcarbamoyl}-méthyl]-benzamide ; le 4-[2-(2,4-dichloro-phénoxy)-phénylsulfamoyl]-*N*-{[3-(2-méthyl-pipéridin-1-yl)-propylcarbamoyl}-méthyl}-benzamide ; le 4-[2-(2,4-dichloro-phénoxy)-phénylsulfamoyl]-*N*-[(3-imidazol-1-yl-propylcarbamoyl)-méthyl]-benzamide ; le 4-[2-(2,4-dichloro-phénoxy)-phénylsulfamoyl)-*N*-[(2-morpholin-4-yl-éthylcarbamoyl)-méthyl]-benzamide ; le 4-[2-(2,4-dichloro-phénoxy)-phénylsulfamoyl)-*N*-[(2-pyrrolidin-1-yl-éthylcarbamoyl)-méthyl]-benzamide ; le 4-[2-(2,4-dichloro-phénoxy)-phénylsulfamoyl]-*N*-[(3-diméthylamino-2,2-diméthyl-propylcarbamoyl)-méthyl]-benzamide ; le chlorhydrate de 4-[2-(4-bromo-2-chloro-phénoxy)-phénylsulfamoyl]-*N-*{[(pipéridin-4-ylméthyl)-carbamoyl]-méthyl}-benzamide ; le chlorhydrate de 4-[2-(2,4-dichloro-phénoxy)-phénylsulfamoyl]-*N*-[(4-guanidinométhyl-benzylcarbamoyl)-méthyl)-benzamide ; le 4-[2-(2,4-dichloro-phénoxy)-phénylsulfamoyl]-*N*-{[méthyl-(2-pyridin-4-yl-éthyl)-carbamoyl]-méthyl}-benzamide; le *N*-[(3-[1,4']bipipéridinyl-1'-yl-propylcarbamoyl)-méthyl]-4-(2-phénoxy-phénylsulfamoyl)-benzamide ; le *N*-[(4-[1,4']bipipéridinyl-1'-yl-phénylcarbamoyl)-méthyl)-4-(2-phénoxy-phénylsulfamoyl)-benzamide ; le trans-4-(2-phénoxyphénylsulfarnoyl)-*N*-{[4-(2-pyrrolidin-1-yl-éthyl)-cyclohexyl-carbamoyl]-méthyl}-benzamide ; le *N*-[(3-diméthylamino-propylcarbamoyl)-méthyl]-4-(2-phénoxy-phénylsulfamoyl)-benzamide ; le *N*-{[3-(2-méthyl-pipéridin-1-yl)-propylcarbamoyl]-méthyl}-4-(2-phénoxy-phénylsulfamoyl)-benzamide ; le chlorhydrate de 4-[2-(2,4-dichloro-phénoxy)-phénylsulfamoyl]-*N*-[(4-pipéridin-4-yl-butylcarbamoyl)-méthyl]-benzamide ; le chlorhydrate de 4-[2-(2,4-dichloro-phénoxy)-phénylsulfamoyl]-*N*-(pipéridin-4-ylcarbamoylméthyl)-benzamide; le chlorhydrate de 4-[2-(2,4-dichloro-phénoxy)-phénylsulfamoyl)-*N*-[(3-pipéridin-4-yl-propylcarbamoyl)-méthyl]-benzamide; le 4-[2-(2,4-dichloro-phénoxy)-phénylsulfamoyl)-*N*-[(4-pipéridin-1-yl-cyclohexyl-carbamoyl)-méthyl)-benzamide ; le 4-[2-(2,4-dichloro-phénoxy)-phénylsulfamoyl]-*N*-[(4-pipéridin-1-yl-butylcarbamoyl)-méthyl]-benzamide ; le chlorhydrate de 4-(2-phénoxy-phénylsulfamoyl)-*N-*(pipéridin-4-ylcarbamoylméthyl)-benzamide ; le chlorhydrate de 4-(2-phénylamino-phénylsulfamoyl)-*N*-{[(pipéridin-4-ylméthyl)-carbamoyl]-méthyl}-benzamide ou le *N*-[(4-cyanométhyl-phénylcarbamoyl)-méthyl]-4-(2-phénoxy-phénylsulfamoyl)-benzamide.

3. Procédé de préparation des composés de formule (I) tels que revendiqués dans la revendication 1 qui comprend
Procédé a)
la réaction d'un dérivé amine de formule (II) 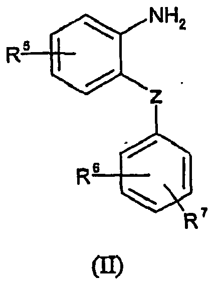 - dans laquelle la signification de R⁵, R⁶ et R⁷ est comme décrit ci-dessus pour la formule (I) - avec le chlorure de sulfonyle de formule (III) 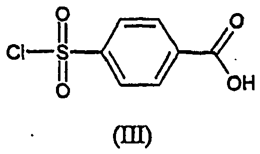 puis la réaction du dérivé d'acide phénylsulfamoyl benzoïque ainsi obtenu de formule (IV) 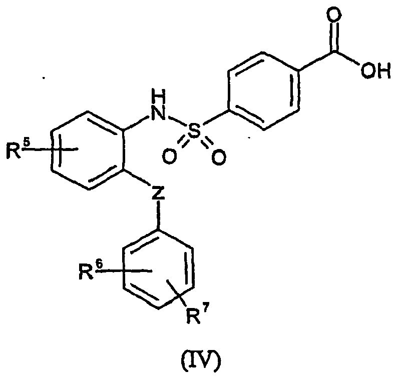 - dans laquelle la signification de R⁵, R⁶ et R⁷ est comme décrit ci-dessus - avec un dérivé amine de formule (V) 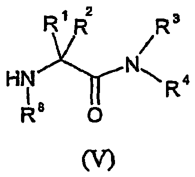 - dans laquelle la signification de R¹, R², R³, R⁴ et R⁸ est comme décrit ci-dessus - pour obtenir un dérivé de phénylsulfamoyl benzamide de formule (I) ou des antipodes ou racémates optiques et/ou sels et/ou hydrates et/ou solvates pharmaceutiquement acceptables de celui-ci.
Procédé b)
La réaction du dérivé d'acide phénylsulfamoyl benzoïque de formule (IV) - obtenu selon le procédé décrit dans le procédé a) - avec un dérivé d'acide aminé de formule (VI) 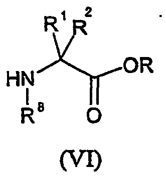
- dans laquelle la signification de R¹, R² et R⁸ est comme défini ci-dessus - et R représente un groupe alkyle en C₁ à C₄
- et l'hydrolyse du composé ainsi obtenu de formule (VII) 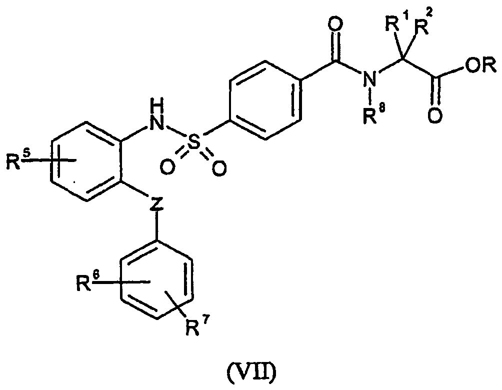 - dans laquelle la signification de R¹, R², R⁵, R⁶, R⁷, R⁸ et R est comme défini ci-dessus -pour former un dérivé acide carboxylique de formule (VIII) 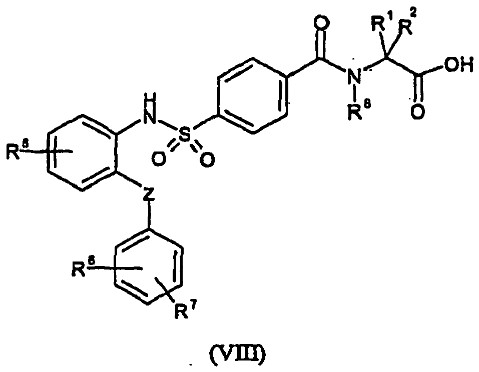 - dans laquelle la signification de R¹, R², R⁵, R⁶, R⁷ et R⁸ est comme défini ci-dessus - enfin la réaction de ce dernier avec un dérivé amine de formule (IX) 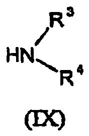 - dans laquelle la signification de R³ et R⁴ est comme défini ci-dessus - pour obtenir un dérivé de phénylsulfamoyl benzamide de formule (I) ou ses antipodes ou racémates optiques et/ou l'un de ses sels et/ou hydrates et/ou solvates pharmaceutiquement acceptables.

4. Procédé de préparation des composés de formule (I) selon la revendication 1 qui comprend la transformation d'un composé de formule (I) en un autre composé de formule (I) en introduisant de nouveaux substituants et/ou en modifiant ou éliminant ceux existants, et/ou par formation de sel et/ou par libération du composé des sels.

5. Composé de formule (V) qui est de l'ester *tert*-butylique de l'acide 4-[2-(2-amino-acétylamino)-éthyl]-pipéridine-1-carboxylique.

6. Composé de formule (IX) choisi dans le groupe constitué du dichlorhydrate de N-(4-aminométhyl-benzyl)-guanidine et de la 2-(4-pyridin-4-yl-pipérazin-1-yl)-propylamine.

7. Composition pharmaceutique comprenant une quantité thérapeutiquement efficace d'un composé de formule (I) selon la revendication 1 ou de ses antipodes ou racémates optiques ou l'un de ses sels ou hydrates ou solvates pharmaceutiquement acceptables et un ou plusieurs excipients pharmaceutiquement acceptables.

8. Utilisation d'un composé de formule (I) selon la revendication 1 ou de ses antipodes ou racémates optiques ou l'un de ses sels ou hydrates ou solvates pharmaceutiquement acceptables pour la fabrication d'un médicament destiné à la prévention et/ou au traitement d'une affection qui requiert l'inhibition d'un récepteur de bradykinine.

9. Utilisation selon la revendication 8, dans laquelle le récepteur de bradykinine est le récepteur de bradykinine B1.

10. Composé de formule (I) selon la revendication 1 ou ses antipodes ou racémates optiques ou l'un de ses sels ou hydrates ou solvates pharmaceutiquement acceptables pour l'utilisation dans un procédé de traitement et/ou de prévention d'une affection qui requiert l'inhibition d'un récepteur de bradykinine.

11. Composé pour l'utilisation selon la revendication 10 dans lequel le récepteur de bradykinine est un récepteur de bradykinine B 1.
